# EUROPEAN PATENT APPLICATION

(11) **EP 2 955 182 A1**
(43) Date of publication of application: **16.12.2015**
(21) Application number: 14172452.6
(22) Date of filing: 13.06.2014
(51) Int. Cl.: C07D 487/04, A61K 31/5025, A61P 35/00

(54) **Pyridazinones for the treatment of cancer**

(71) Applicant: Max-Planck-Gesellschaft zur Förderung der Wissenschaften e.V., 80539 München (DE)
(72) Inventor: WALDMANN, Herbert, Prof. Dr., 44265 Dortmund (DE)
(74) Representative: Arth, Hans-Lothar

(57) **Abstract**

The present invention relates to novel substituted pyrrolo- and pyrazolopyridazinones, as well as pharmaceutical compositions containing at least one of these substituted pyrrolo- and pyrazolo- pyridazinones together with at least one pharmaceutically acceptable carrier, excipient and/or diluent. Said novel substituted pyrrolo- and pyrazolo- pyridazinones are binding to the prenyl binding pocket of PDEδ and therefore, are useful for the prophylaxis and treatment of cancer by inhibition of the binding of PDEδ to K-Ras and of Ras signaling in cells.

## Description

The present invention relates to novel substituted pyrrolo- and pyrazolo-pyridazinones, as well as pharmaceutical compositions containing at least one of these substituted pyrrolo- and pyrazolo- pyridazinones together with at least one pharmaceutically acceptable carrier, excipient and/or diluent. Said novel substituted pyrrolo- and pyrazolo- pyridazinones are binding to the prenyl binding pocket of PDEδ and therefore, are useful for the prophylaxis and treatment of cancer by inhibition of the binding of PDEδ to K-Ras and of Ras signaling in cells.

### Background of the invention

In cancer treatment, there is an ongoing need for the development of novel substances, which are effective to induce cell cycle/proliferation arrest or cell death of cancer cells. The fundamental characteristics of these cells are that the control of the cell cycle and proliferation is disturbed and they evade oncogene induced stress response and cell senescence.

Ras proteins are key regulators of diverse cellular processes including proliferation and differentiation and are mutated in ca. 20 - 30 % of human cancers, with the K-Ras isoform most frequently mutated. Despite substantial efforts to interfere with signaling by oncogenic Ras proteins, in particular by means of Ras-farnesyltransferase inhibitors, up to present no clinically useful drug has been found. Signaling by Ras proteins critically depends on their correct subcellular localization, which in turn is regulated by lipid modifications at their C-terminus. Thus, K-Ras, a major proto-oncogenic isoform of the Ras proteins, is S-farnesylated at its C-terminal CaaX box. PDEδ binds K-Ras *via* the farnesylated C-terminus and has an essential role in sustaining its spatial organization and proper localization in cells, by facilitating its intracellular diffusion to enhance the kinetics of trapping at the right membrane compartment, i.e. the plasma membrane for K-Ras. This regulation of Ras localization and signaling by PDEδ suggests that interfering with the PDEδ-Ras interaction by means of small molecules might provide a novel opportunity to suppress signaling from oncogenic and normal Ras. Suppressing oncogenic Ras signaling results in halting Ras-dependent tumor proliferation.

It is the objective of the present invention to provide novel pyrrolo- and pyrazolo-pyridazinones that can be used as pharmaceutically active agents, especially for the treatment or prophylaxis of cancers, tumors and proliferative diseases, as well as compositions comprising at least one of these compounds as pharmaceutically active agent.

The objective of the present invention is solved by the teaching of the independent claims. Further advantageous features, aspects and details of the invention are evident from the dependent claims, the description, the figures, and the examples of the present application.

### Description of the invention

In the present invention, it was surprisingly found that the binding of pyridazinones of the general formula (I) to the prenyl binding pocket of PDEδ induces strong inhibition of the binding of PDEδ to K-Ras and oncogenic Ras signaling in cells, thereby causing inhibition of tumor cell proliferation and tumor cell death. Hence, these inventive compounds are useful for the treatment or prophylaxis of cancers, tumors and other proliferative diseases.

Thus, the present invention is directed to a pyridazinone of the general formula (I) 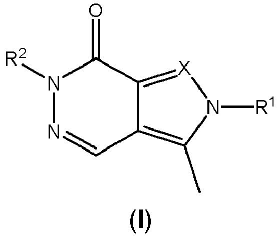 wherein
X represents N or C(CH₃);
R¹ represents R³ and R² represents -CH₂CH₂CH₂R⁴, or
R¹ represents -CH₂CH₂CH₂R⁴ and R² represents R³;
R³ represents 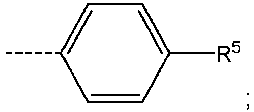 R⁵ is selected from -H, -CH₃, -C₂H₅, -CH₂CH₂CH₃, and -CH(CH₃)₂;
R⁴ represents: -C(O)-NH-R⁶, 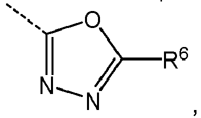 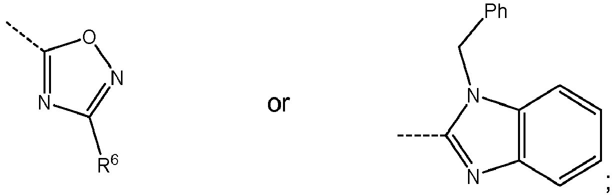 R⁶ represents -(CR⁸R⁹)ₙ₁-(CH₂)ₙ₂-R⁷;
R⁷ represents: 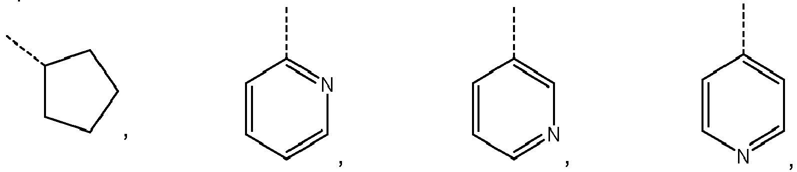 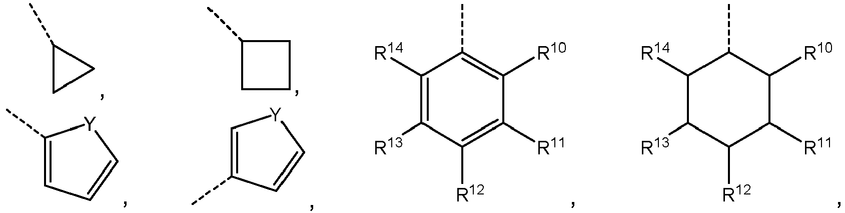 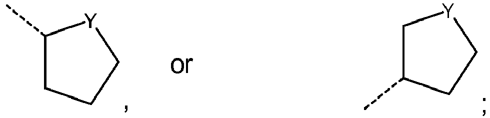 -Y- is selected from -O- and -S-; R⁸, R⁹ are independently of each other selected from -H, -CH₃ and -C₂H₅; R¹⁰, R¹¹, R¹², R¹³ and R¹⁴ are independently of each other selected from: -H, -Cl, -F, -CH₃, -CH₂CH₃, -CH₂CH₂CH₃, -CH(CH₃)₂, -OCH₃, -OCH₂CH₃, -OCH₂CH₂CH₃, -OCH(CH₃)₂ and -CF₃;

n1 and n2 are independently of each other selected from 0 and 1;
and enantiomers, mixtures of enantiomers, diastereomers, mixtures of diastereomers, hydrates, solvates, tautomers, racemates and pharmaceutically acceptable salts thereof.

An embodiment of the present invention relates to a pyrrolopyridazinone of general formula **(II)** 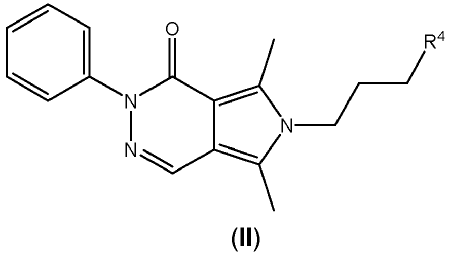 wherein
R⁴ represents: -C(O)-NH-R⁶, 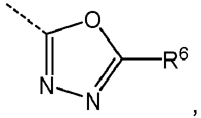 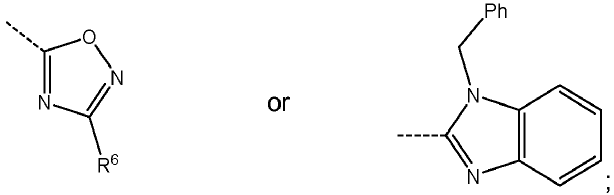 R⁶ represents -R⁷ or -CH₂-R⁷;
R⁷ represents: 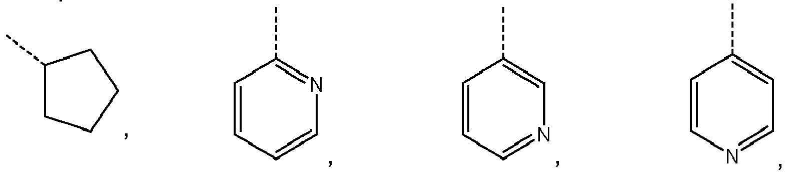 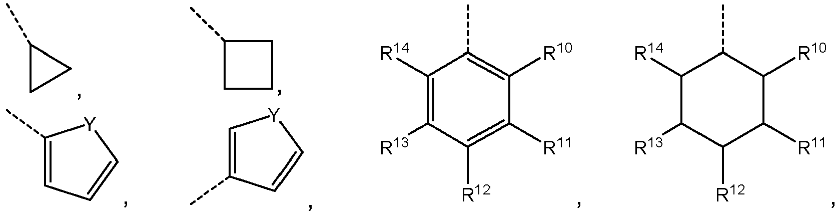 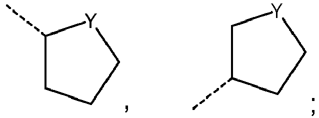 -Y- is selected from -O- and -S-, and
R¹⁰, R¹¹, R¹², R¹³ and R¹⁴ are independently of each other selected from: -H, -Cl, -F, -CH₃, -CH₂CH₃, -CH₂CH₂CH₃, -CH(CH₃)₂, -OCH₃, -OCH₂CH₃, -OCH₂CH₂CH₃, -OCH(CH₃)₂ and -CF₃.

A pyrrolopyridazinone of general formula **(III)** 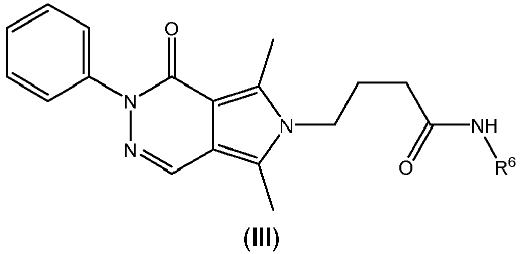 wherein residue R⁶ represents -R⁷ or -CH₂-R⁷; residue R⁷ is selected from: 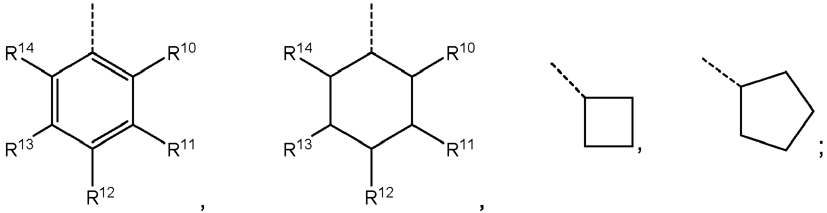 and residues R¹⁰, R¹¹, R¹², R¹³ and R¹⁴ have the meanings defined above is preferred.

Preferably, in general formula **(III)** the residue R⁶ represents -R⁷, the residue R⁷ is selected from: 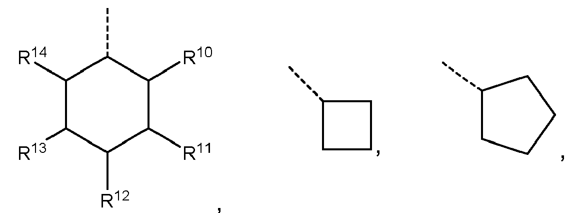 and the residues R¹⁰, R¹¹, R¹², R¹³ and R¹⁴ have the meanings defined above; or
preferably the residue R⁶ represents -CH₂-R⁷, the residue R⁷ represents 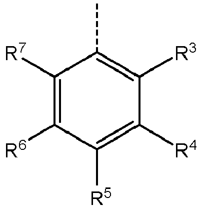 and the residues R¹⁰, R¹¹, R¹², R¹³ and R¹⁴ have the meanings defined above.

Hence, compounds of general formulae **(IIIa)**, **(IIIb), (IIIc)** and **(IIId),** wherein R¹⁰, R¹¹, R¹², R¹³ and R¹⁴ have the meanings defined above are especially preferred.

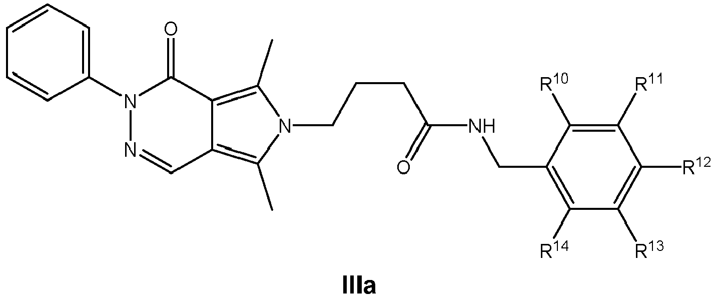

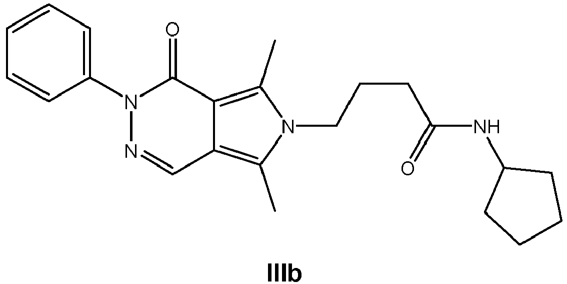

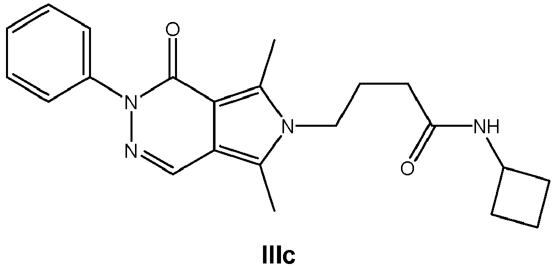

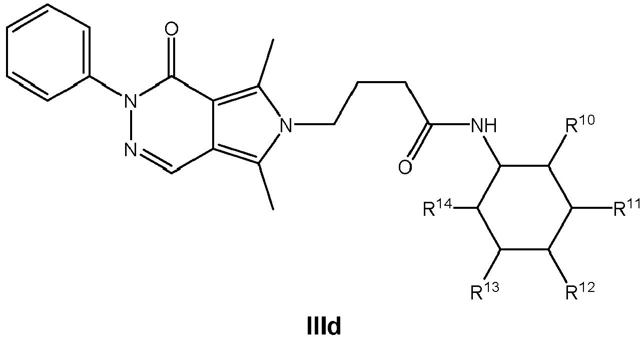

Especially preferred are compounds of general formula **(IIIa),** wherein at least one of the residues R¹⁰, R¹¹, R¹², R¹³ and R¹⁴ is -Cl and compounds of general formula **(IIId),** wherein at least one of the residues R¹⁰, R¹¹, R¹², R¹³ and R¹⁴, and preferably two of the residues R¹⁰, R¹¹, R¹², R¹³ and R¹⁴ are -CH₃.

Still preferred compounds are pyrrolopyridazinones of general formulae **(II)** and **(III)**, wherein the residue R⁶ is selected from: 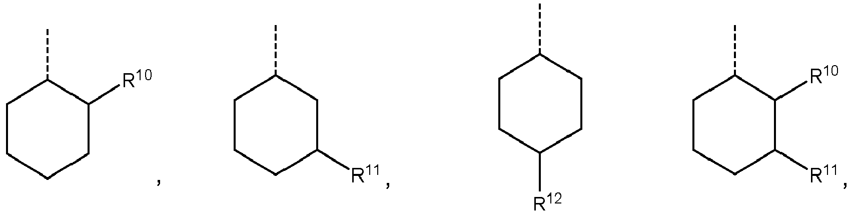 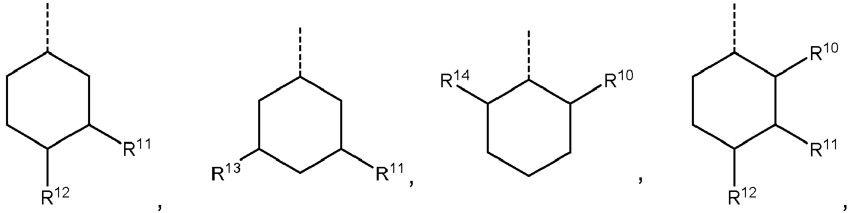 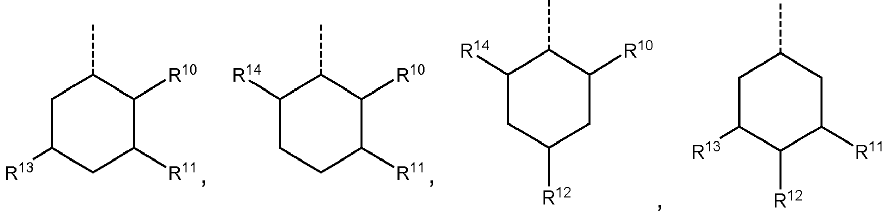 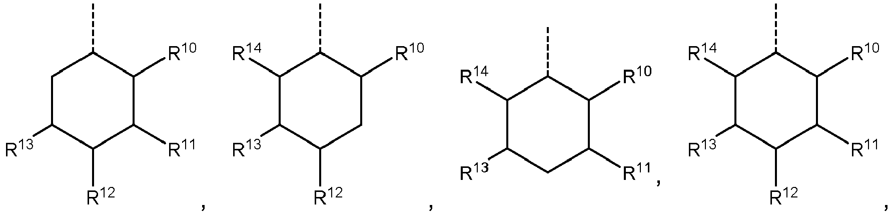 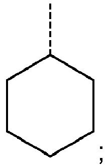 and the residues R¹⁰, R¹¹, R¹², R¹³ and R¹⁴ are independently of each other selected from: -CH₃, -CH₂CH₃, -CH₂CH₂CH₃, -CH(CH₃)₂ and -CF₃.

Other preferred compounds of the current invention are pyrrolopyridazinones of general formulae **(II)** and **(III)**, wherein R⁶ is selected from: 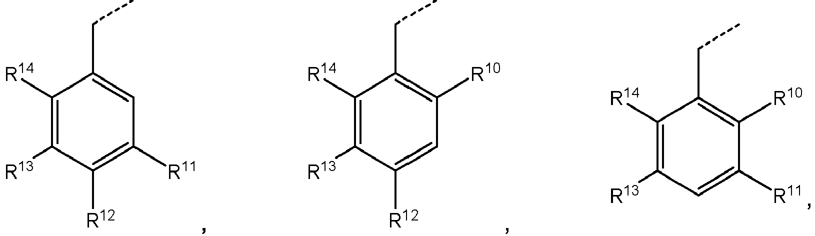 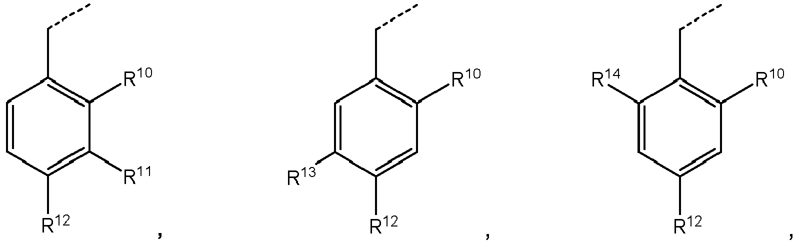 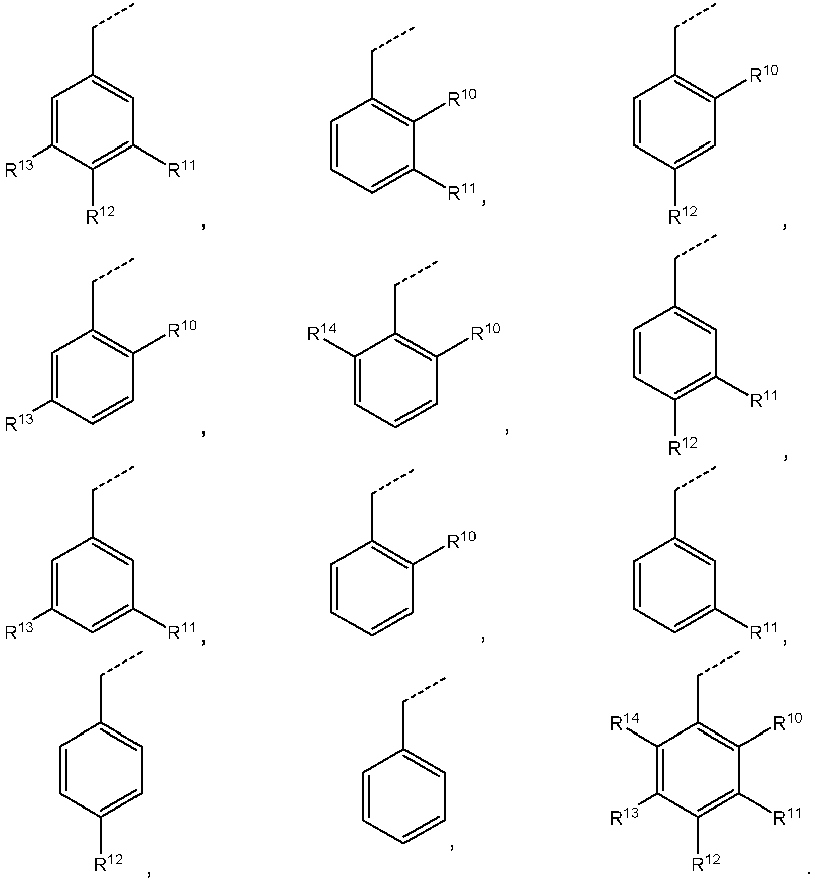 and R¹⁰, R¹¹, R¹², R¹³ and R¹⁴ are independently of each other selected from: -CH₃, -OCH₃, -CF₃, -Cl and -F.

Especially preferred compounds are pyrrolopyridazinones of general formula **(II)** and **(III)**, wherein R⁶ represents: 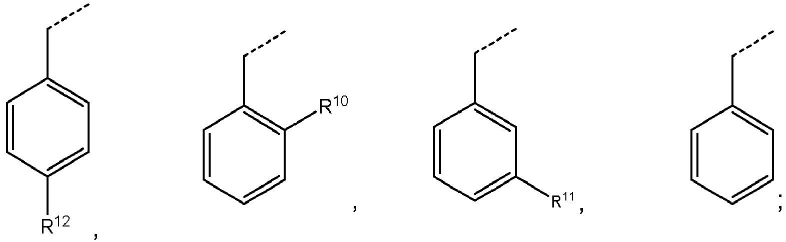 and R¹⁰, R¹¹ and R¹² represent independently of each other -Cl or -F.

Another embodiment of the present invention is directed to compounds of general formula **(II)** 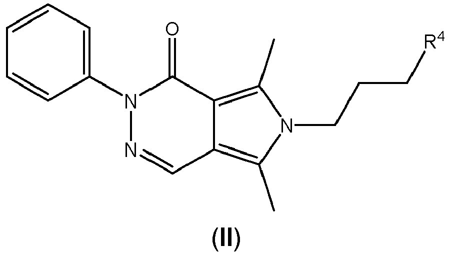 wherein

R⁴ represents: 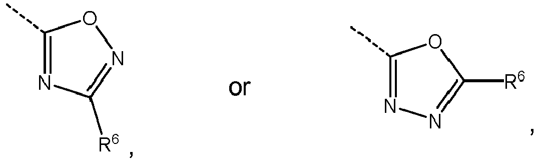 and R⁶ has the meaning defined above.

Thus, compounds of the following general formulae (V) and **(VI)** are also preferred.

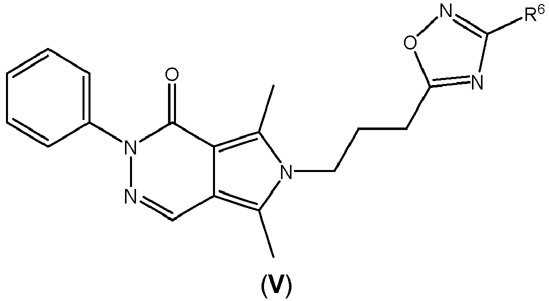

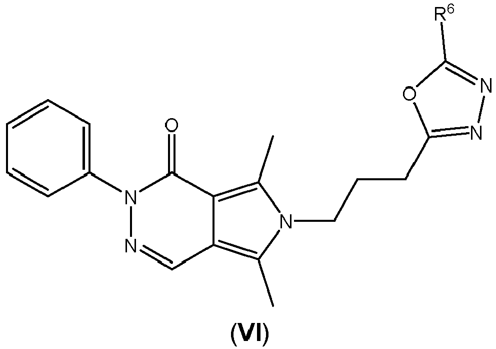

Even more preferred are compounds of general formula **(II)** 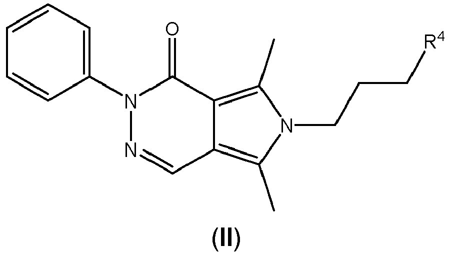 wherein
R⁴ represents: 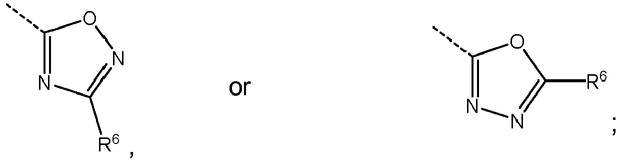 R⁶ is selected from: 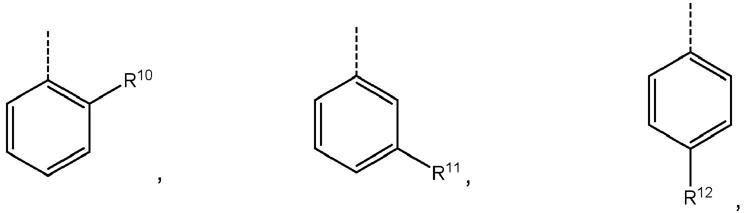 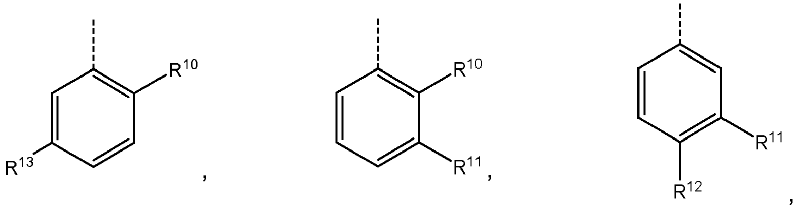 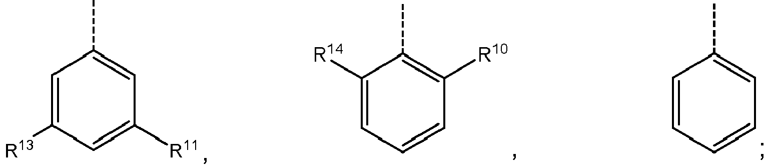 and R¹⁰, R¹¹, R¹², R¹³ and R¹⁴ are independently of each other selected from: -CH₃, -OCH₃, -CF₃, and -F.

Therefore, even more preferred are compounds of general formula (V) and (VI), wherein R⁶ is selected from: 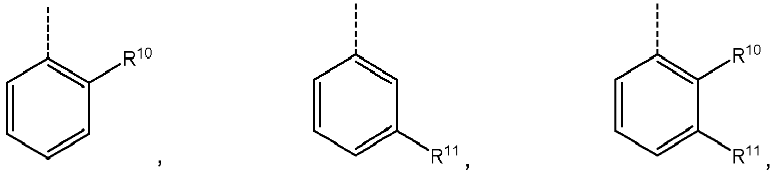 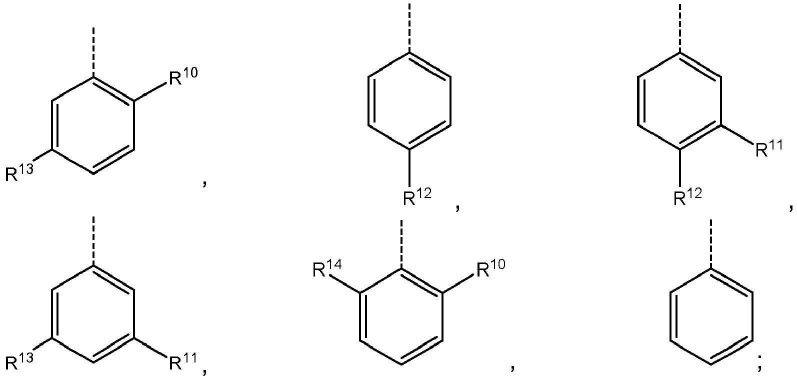 and R¹⁰, R¹¹, R¹², R¹³ and R¹⁴ are independently of each other selected from: -CH₃, -OCH₃, -CF₃, and -F.

Another preferred embodiment of the present invention relates to a pyrazolopyridazinone of general formula (IV) 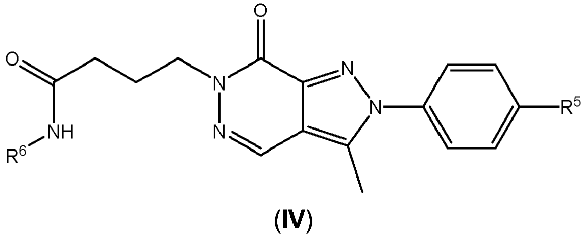 wherein
R⁵ is selected from -H and -CH₃;
R⁶ represents -(CR⁸R⁹)ₙ₁-(CH₂)ₙ₂-R⁷;
R⁷ represents: 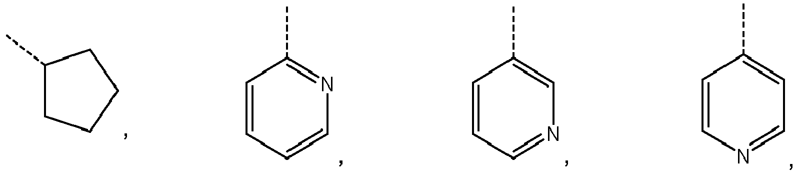 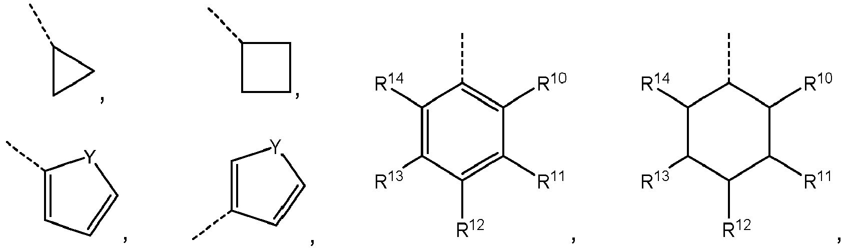 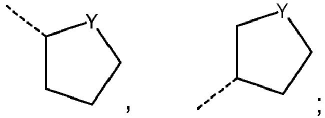 -Y- is selected from -O- and -S-;
R⁸, R⁹ are independently of each other selected from -H, -CH₃ and -C₂H₅; R¹⁰, R¹¹, R¹², R¹³ and R¹⁴ are independently of each other selected from: -H, -Cl, -F, -CH₃, -CH₂CH₃, -CH₂CH₂CH₃, -CH(CH₃)₂, -OCH₃, -OCH₂CH₃, -OCH₂CH₂CH₃, -OCH(CH₃)₂, and -CF₃; and n1 and n2 are independently of each other selected from 0 or 1.

Preferred compounds are pyrazolopyridazinones of general formula (IV) 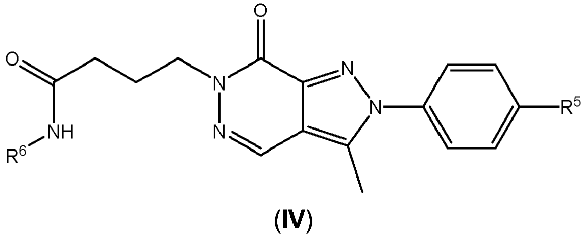 wherein,
the residue R⁵ represents -CH₃ and the residue R⁶ is selected from 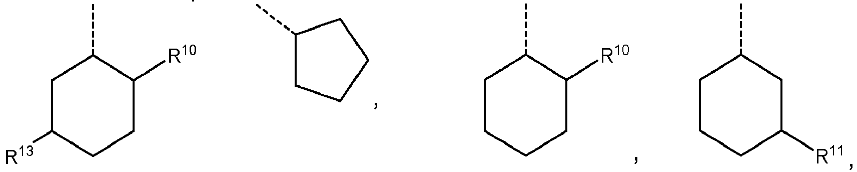 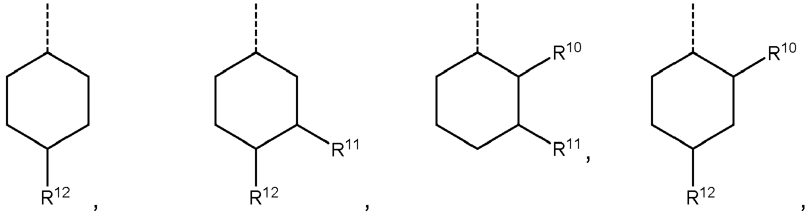 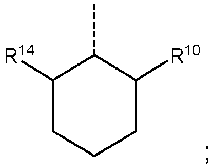 and the residues R¹⁰ R¹¹, R¹², R¹³ and R¹⁴ have the meanings defined above.

Even more preferred compounds are pyrazolopyridazinones of general formula **(IV)** 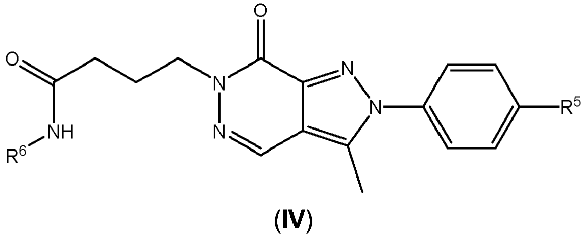 wherein the residue R⁶ is selected from: 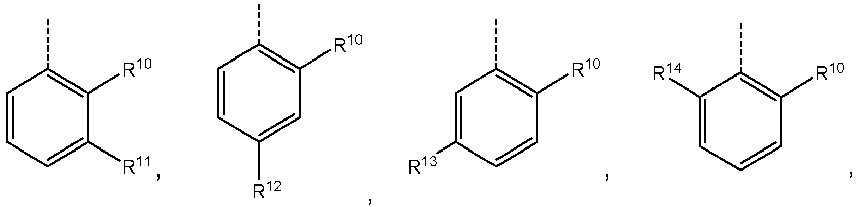 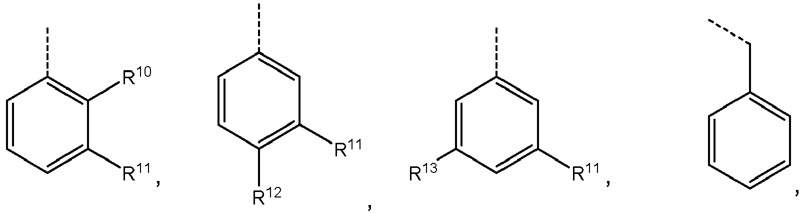 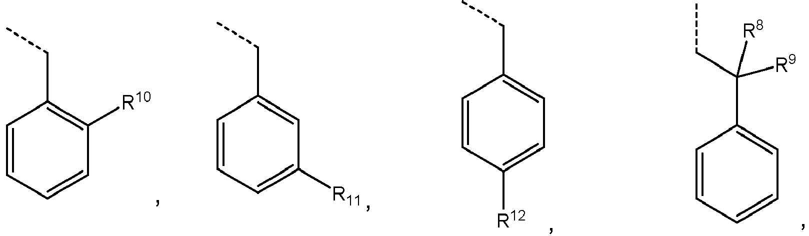 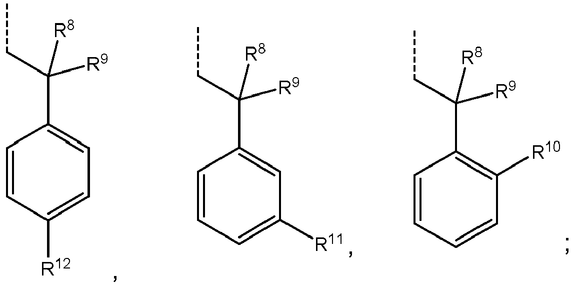 residues R⁸, R⁹ are independently of each other selected from -H, -CH₃ and -C₂H₅; and residues R¹⁰, R¹¹, R¹², R¹³ and R¹⁴ are independently of each other selected from: -Cl, -F, -CH₃, -OCH₃, -OCH(CH₃)₂ and -CF₃.

Especially preferred compounds are pyrazolopyridazinones of general formula (IV) 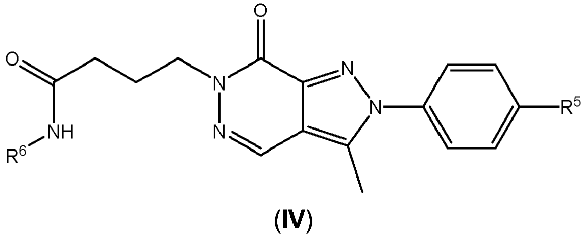 wherein the residue R⁶ is selected from: 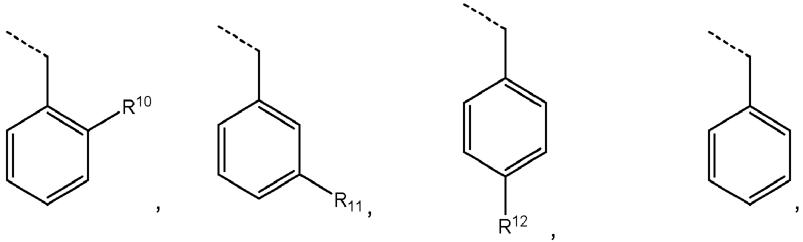 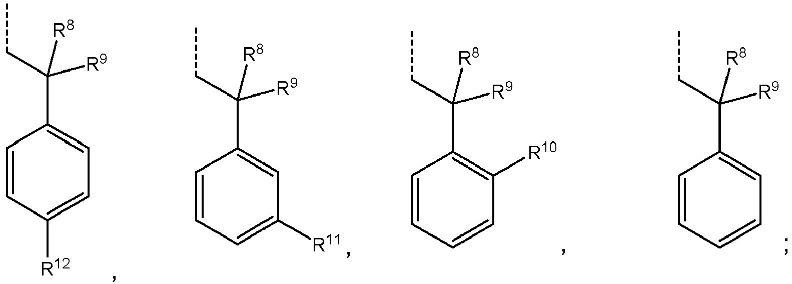 residues R⁸, R⁹ are independently of each other selected from -H, -CH₃ and -C₂H₅; and residues R¹⁰, R¹¹, R¹², R¹³ and R¹⁴ are independently of each other selected from: -Cl, -F, -CH₃, -OCH₃, -OCH(CH₃)₂ and -CF₃.

In general formula **(IV),** preferably the residue R⁶ represents: 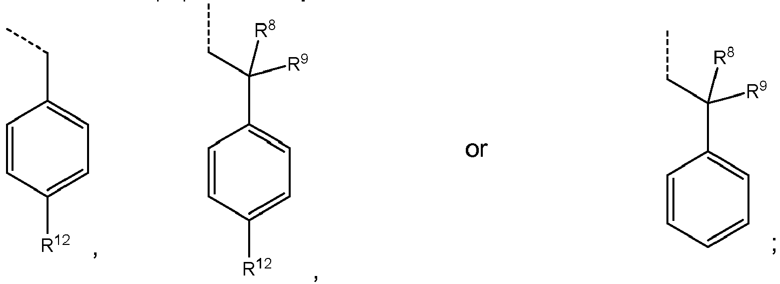 and the residue R¹² is defined as above and most preferably is selected from: -CH₃, -OCH₃, -OCH(CH₃)₂ and -CF₃.

Preferably, the compounds of the present invention are selected from:

| **Compound Number** | **Structure, IUPAC name** |
|---|---|
| 1 | 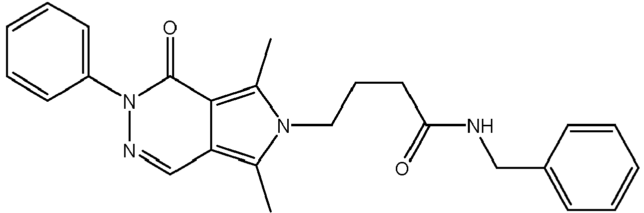 |
| | *N*-benzyl-4-(5,7-dimethyl-1-oxo-2-phenyl-1*H*-pyrrolo[3,4-d]pyridazin-6(2*H*)-yl)butanamide |
| 2 | 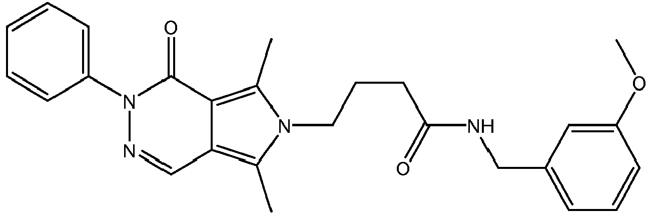 |
| | 4-(5,7-dimethyl-1-oxo-2-phenyl-1*H*-pyrrolo[3,4-*d*]pyridazin-6(2*H*)-yl)-*N*-(3-methoxybenzyl)butanamide |
| 3 | 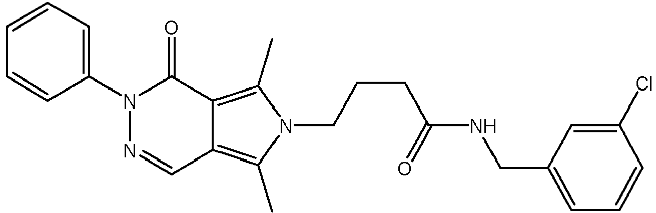 |
| | *N*-(3-chlorobenzyl)-4-(5,7-dimethyl-1-oxo-2-phenyl-1*H*-pyrrolo[3,4-*d*]pyridazin-6(2*H*)-yl)butanamide |
| 4 | 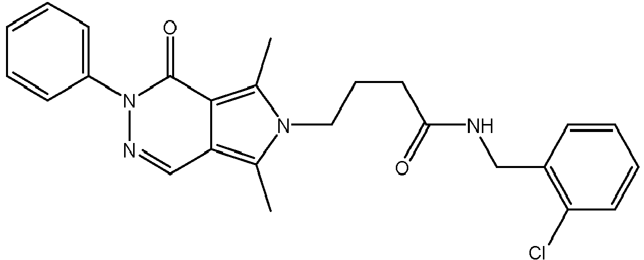 |
| | *N*-(2-chlorobenzyl)-4-(5,7-dimethyl-1-oxo-2-phenyl-1*H-*pyrrolo[3,4-*d*]pyridazin-6(2*H*)-yl)butanamide |
| 5 | 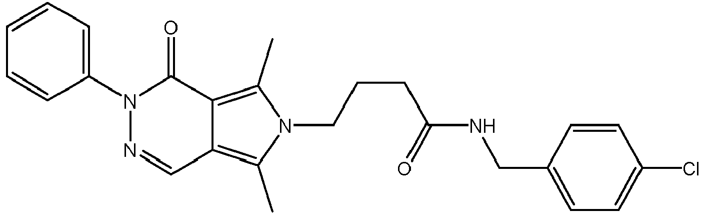 |
| | *N*-(4-chlorobenzyl)-4-(5,7-dimethyl-1-oxo-2-phenyl-1*H*-pyrrolo[3,4-*d*]pyridazin-6(2*H*)-yl)butanamide |
| 6 | 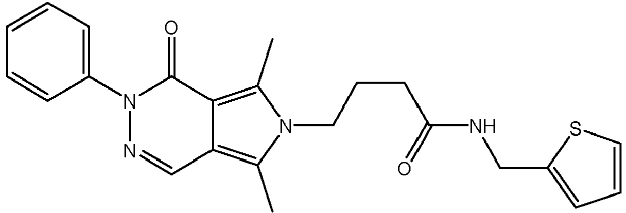 |
| | 4-(5,7-dimethyl-1-oxo-2-phenyl-1*H*-pyrrolo[3,4-*d*]pyridazin-6(2*H*)-yl)-N-(thiophen-2-ylmethyl)butanamide |
| 7 | 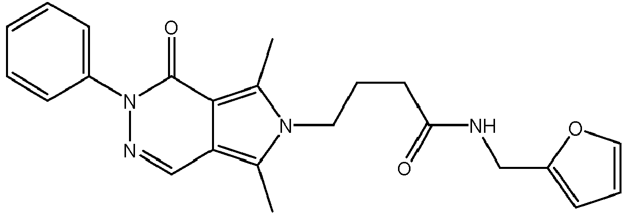 |
| | 4-(5,7-dimethyl-1-oxo-2-phenyl-1*H*-pyrrolo[3,4-*d*]pyridazin-6(2*H*)-yl)-*N*-(furan-2-ylmethyl)butanamide |
| 8 | 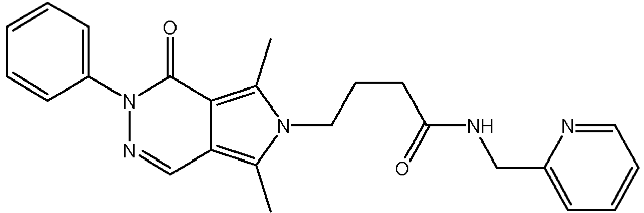 |
| | 4-(5,7-dimethyl-1-oxo-2-phenyl-1H-pyrrolo[3,4-*d*]pyridazin-6(2*H*)-yl)-*N*-(pyridin-2-ylmethyl)butanamide |
| 9 | 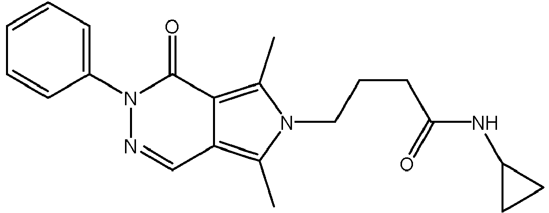 |
| | *N*-cyclopropyl-4-(5,7-dimethyl-1-oxo-2-phenyl-1*H*-pyrrolo[3,4-*d*]pyridazin-6(2*H*)-yl)butanamide |
| 10 | 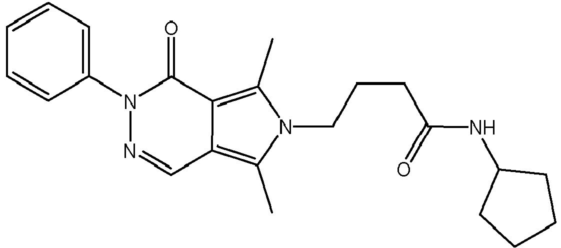 |
| | *N*-cyclopentyl-4-(5,7-dimethyl-1-oxo-2-phenyl-1*H*-pyrrolo[3,4-*d*]pyridazin-6(2*H*)-yl)butanamide |
| 11 | 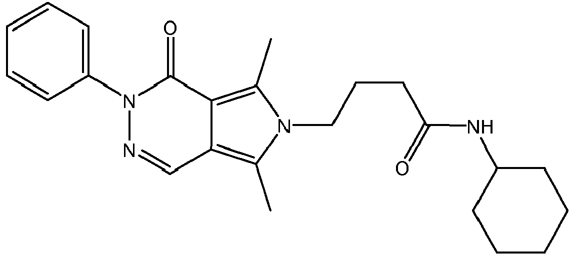 |
| | *N*-cyclohexyl-4-(5,7-dimethyl-1-oxo-2-phenyl-1*H*-pyrrolo[3,4-*d*]pyridazin-6(2*H*)-yl)butanamide |
| 12 | 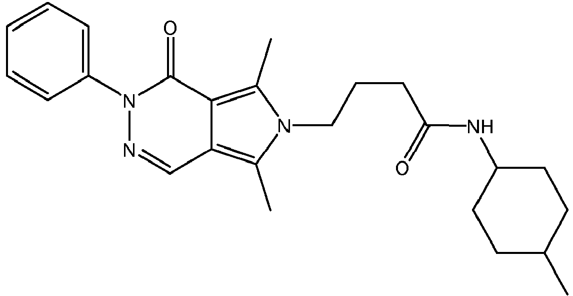 |
| | 4-(5,7-dimethyl-1-oxo-2-phenyl-1*H*-pyrrolo[3,4-*d*]pyridazin-6(2*H*)-yl)-*N*-(4-methylcyclohexyl)butanamide |
| 13 | 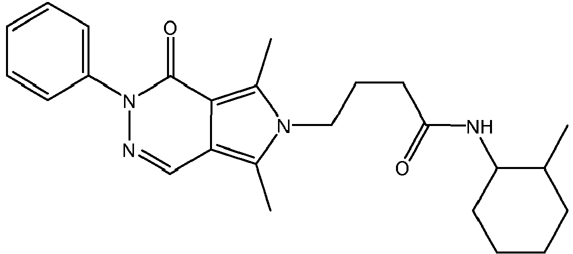 |
| | 4-(5,7-dimethyl-1-oxo-2-phenyl-1*H*-pyrrolo[3,4-*d*]pyridazin-6(2*H*)-yl)-N-(2-methylcyclohexyl)butanamide |
| 14 | 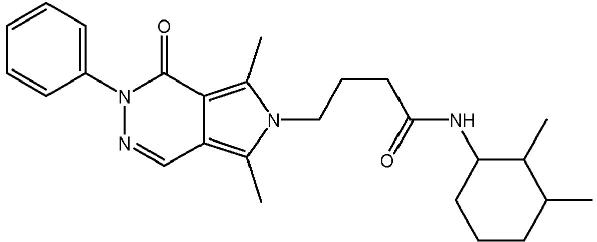 |
| | 4-(5,7-dimethyl-1-oxo-2-phenyl-1*H*-pyrrolo[3,4-*d*]pyridazin-6(2*H*)-yl)-*N*-(2,3-dimethylcyclohexyl)butanamide |
| 15 | 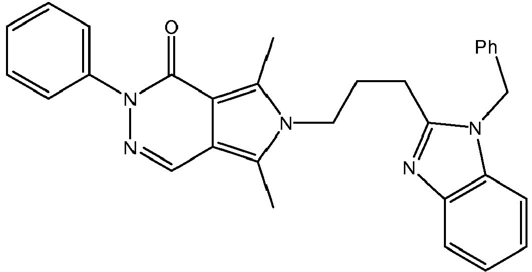 |
| | 6-(3-(1-benzyl-1*H-*benzo[d]imidazol-2-yl)propyl)-5,7-dimethyl-2-phenyl-2,6-dihydro-1*H*-pyrrolo[3,4-*d*]pyridazin-1 -one |
| 16 | 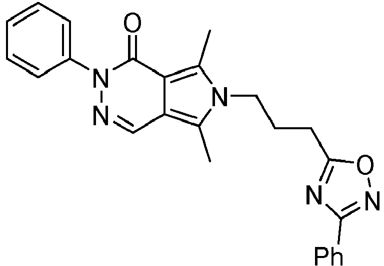 |
| | 5,7-dimethyl-2-phenyl-6-(3-(3-phenyl-1,2,4-oxadiazol-5-yl)propyl)-2,6-dihydro-1*H*-pyrrolo[3,4-*d*]pyridazin-1-one |
| 17 | 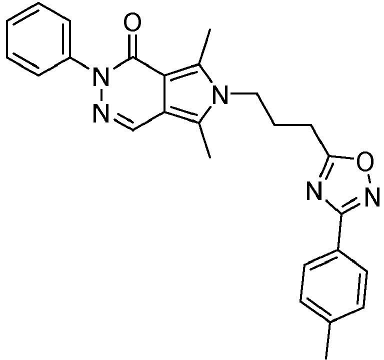 |
| | 5,7-dimethyl-2-phenyl-6-(3-(3-(p-tolyl)-1,2,4-oxadiazol-5-yl)propyl)-2,6-dihydro-1*H*-pyrrolo[3,4-*d*]pyridazin-1-one |
| 18 | 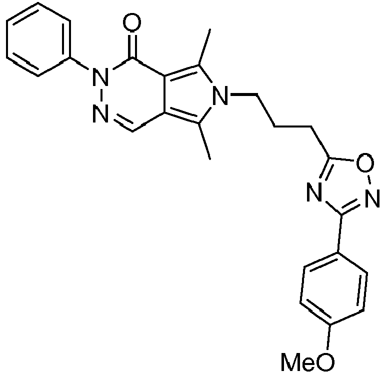 |
| | 6-(3-(3-(4-methoxyphenyl)-1,2,4-oxadiazol-5-yl)propyl)-5,7-dimethyl-2-phenyl-2,6-dihydro-1*H*-pyrrolo[3,4-*d*]pyridazin-1-one |
| 19 | 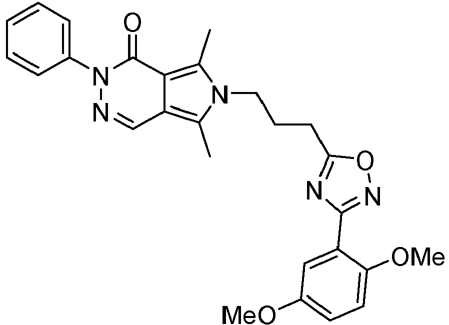 |
| | 6-(3-(3-(2,5-dimethoxyphenyl)-1,2,4-oxadiazol-5-yl)propyl)-5,7-dimethyl-2-phenyl-2,6-dihydro-1*H*-pyrrolo[3,4-*d*]pyridazin-1-one |
| 20 | 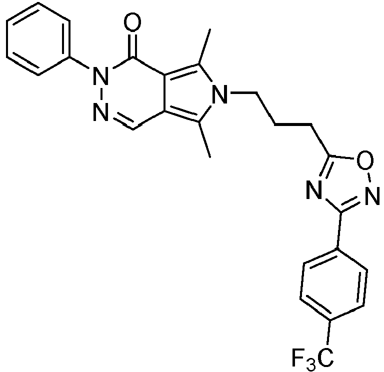 |
| | 5,7-dimethyl-2-phenyl-6-(3-(3-(4-(trifluoromethyl)phenyl)-1,2,4-oxadiazol-5-yl)propyl)-2,6-dihydro-1*H*-pyrrolo[3,4-*d*]pyridazin-1-one |
| 21 | 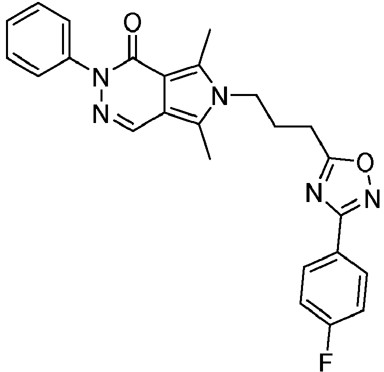 |
| | 6-(3-(3-(4-fluorophenyl)-1,2,4-oxadiazol-5-yl)propyl)-5,7-dimethyl-2-phenyl-2,6-dihydro-1*H*-pyrrolo[3,4-*d*]pyridazin-1-one |
| 22 | 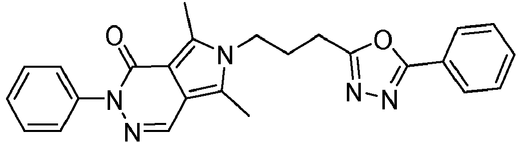 |
| | 5,7-dimethyl-2-phenyl-6-(3-(5-phenyl-1,3,4-oxadiazol-2-yl)propyl)-2,6-dihydro-1*H*-pyrrolo[3.4-*d*]pyridazin-1-one |
| 23 | 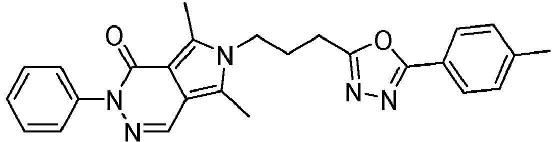 |
| | 5,7-dimethyl-2-phenyl-6-(3-(5-(p-tolyl)-1,3,4-oxadiazol-2-yl)propyl)-2,6-dihydro-1*H*-pyrrolo[3,4-*d*]pyridazin-1-one |
| 24 | 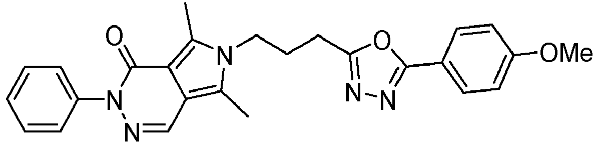 |
| | 6-(3-(5-(4-methoxyphenyl)-1,3,4-oxadiazol-2-yl)propyl)-5,7-dimethyl-2-phenyl-2,6-dihydro-1*H*-pyrrolo[3,4-*d*]pyridazin-1-one |
| 25 | 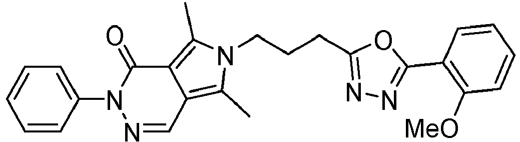 |
| | 6-(3-(5-(2-methoxyphenyl)-1,3,4-oxadiazol-2-yl)propyl)-5,7-dimethyl-2-phenyl-2,6-dihydro-1*H*-pyrrolo[3,4-*d*]pyridazin-1-one |
| 26 | 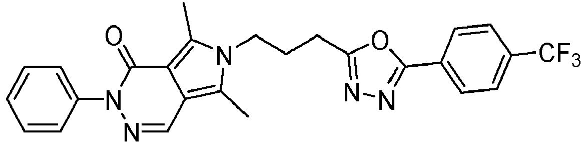 |
| | 5,7-dimethyl-2-phenyl-6-(3-(5-(4-(trifluoromethyl)phenyl)-1,3,4-oxadiazol-2-yl)propyl)-2,6-dihydro-1*H*-pyrrolo[3,4-*d*]pyridazin-1-one |
| 27 | 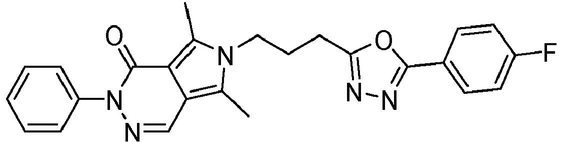 |
| | 6-(3-(5-(4-fluorophenyl)-1,3,4-oxadiazol-2-yl)propyl)-5,7-dimethyl-2-phenyl-2,6-dihydro-1*H*-pyrrolo[3,4-*d*]pyridazin-1-one |
| 28 | 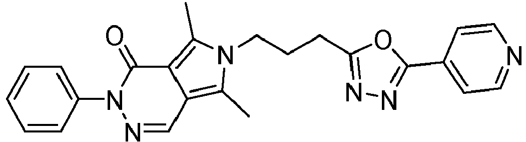 |
| | 5,7-dimethyl-2-phenyl-6-(3-(5-(pyridin-4-yl)-1,3,4-oxadiazol-2-yl)propyl)-2,6-dihydro-1*H*-pyrrolo[3,4-*d*]pyridazin-1-one |
| 29 | 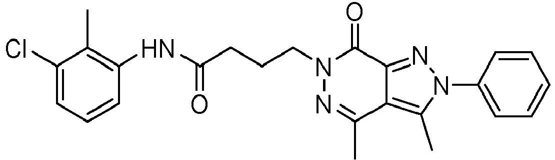 |
| | *N*-(3-chloro-2-methylphenyl)-4-(3,4-dimethyl-7-oxo-2-phenyl-2*H-*pyrazolo[3,4-*d*]pyridazin-6(7*H*)-yl)butanamide |
| 30 | 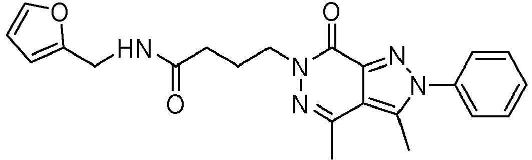 |
| | 4-(3,4-dimethyl-7-oxo-2-phenyl-2*H*-pyrazolo[3,4-*d*]pyridazin-6(7*H*)-yl)-N-(furan-2-ylmethyl)butanamide |
| 31 | 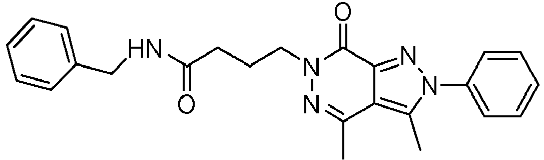 |
| | *N*-benzyl-4-(3,4-dimethyl-7-oxo-2-phenyl-2*H*-pyrazolo[3,4-*d*]pyridazin-6(7*H*)-yl)butanamide |
| 32 | 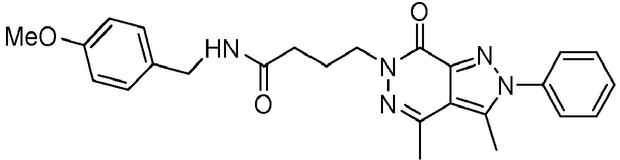 |
| | 4-(3,4-dimethyl-7-oxo-2-phenyl-2*H*-pyrazolo[3,4-*d*]pyridazin-6(7*H*)-yl)-*N*-(4-methoxybenzyl)butanamide |
| 33 | 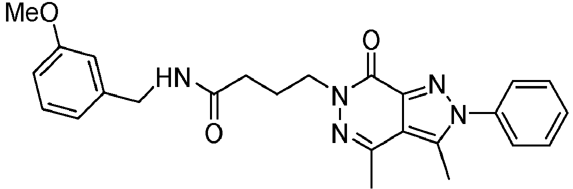 |
| | 4-(3,4-dimethyl-7-oxo-2-phenyl-2*H*-pyrazolo[3,4-*d*]pyridazin-6(7*H*)-yl)-*N*-(3-methoxybenzyl)butanamide |
| 34 | 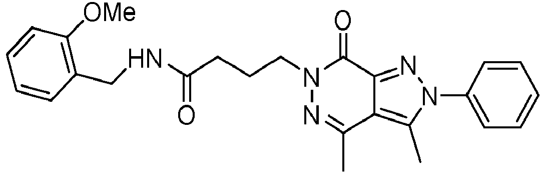 |
| | 4-(3,4-dimethyl-7-oxo-2-phenyl-2*H*-pyrazolo[3,4-*d*]pyridazin-6(7*H*)-yl)-N-(2-methoxybenzyl)butanamide |
| 35 | 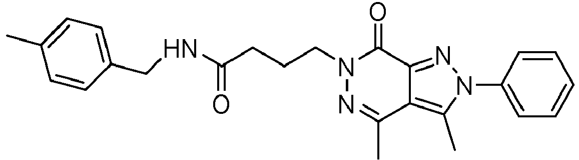 |
| | 4-(3,4-dimethyl-7-oxo-2-phenyl-2*H*-pyrazolo[3,4-*d*]pyridazin-6(7*H*)-yl)-*N*-(4-methylbenzyl)butanamide |
| 36 | 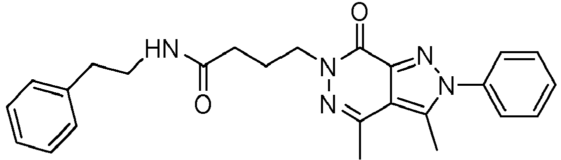 |
| | 4-(3,4-dimethyl-7-oxo-2-phenyl-2*H*-pyrazolo[3,4-*d*]pyridazin-6(7*H*)-yl)-*N*-phenethylbutanamide |
| 37 | 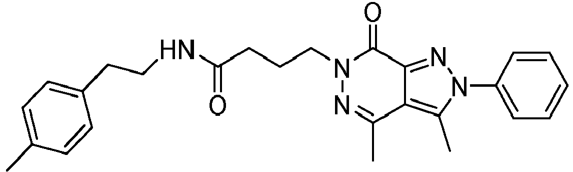 |
| | 4-(3,4-dimethyl-7-oxo-2-phenyl-2*H*-pyrazolo[3,4-*d*]pyridazin-6(7*H*)-yl)-*N*-(4-methylphenethyl)butanamide |
| 38 | 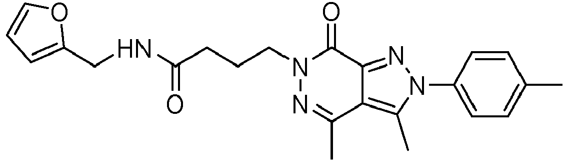 |
| | 4-(3,4-dimethyl-7-oxo-2-(p-tolyl)-2*H*-pyrazolo[3,4-*d*]pyridazin-6(7*H*)-yl)-*N*-(furan-2-ylmethyl)butanamide |
| 39 | 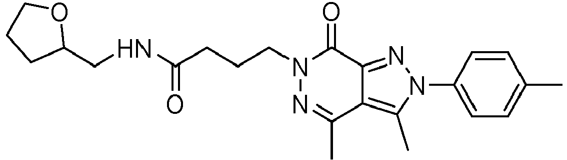 |
| | 4-(3,4-dimethyl-7-oxo-2-(*p*-tolyl)-2*H*-pyrazolo[3,4-*d*]pyridazin-6(7*H*)-yl)-*N*-((tetrahydrofuran-2-yl)methyl)butanamide |
| 40 | 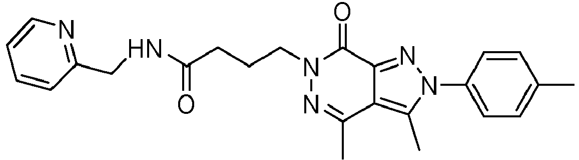 |
| | 4-(3,4-dimethyl-7-oxo-2-(*p*-tolyl)-2*H*-pyrazolo[3,4-*d*]pyridazin-6(7*H*)-yl)-*N*-(pyridin-2-ylmethyl)butanamide |
| 41 | 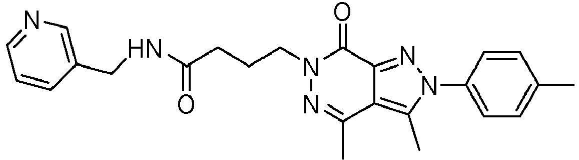 |
| | 4-(3,4-dimethyl-7-oxo-2-(*p*-tolyl)-2*H*-pyrazolo[3,4-*d*]pyridazin-6(7*H*)-yl)-*N*-(pyridin-3-ylmethyl)butanamide |
| 42 | 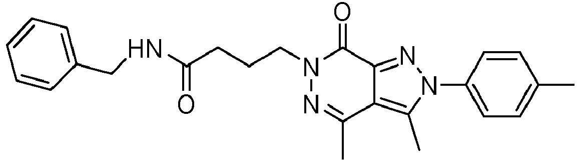 |
| | *N*-benzyl-4-(3,4-dimethyl-7-oxo-2-(*p*-tolyl)-2*H*-pyrazolo[3,4-*d*]pyridazin-6(7*H*-yl)butanamide |
| 43 | 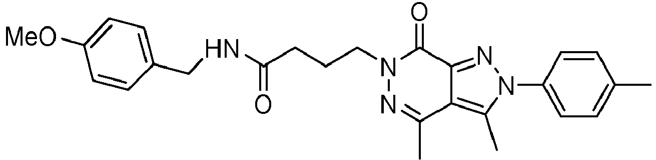 |
| | 4-(3,4-dimethyl-7-oxo-2-(*p*-tolyl)-2*H*-pyrazolo[3,4-*d*]pyridazin-6(7*H*)-yl)-*N*-(4-methoxybenzyl)butanamide |
| 44 | 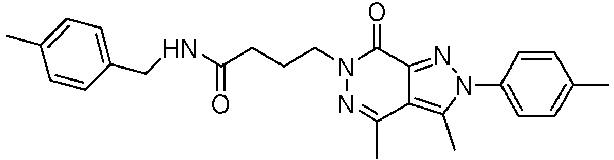 |
| | 4-(3,4-dimethyl-7-oxo-2-(*p*-tolyl)-2*H*-pyrazolo[3,4-*d*]pyridazin-6(7*H*)-yl)-*N*-(4-methylbenzyl)butanamide |
| 45 | 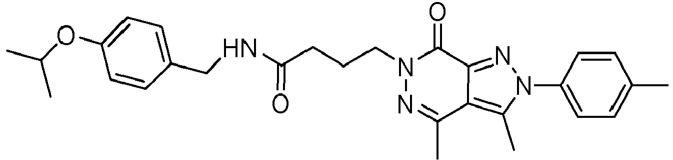 |
| | 4-(3,4-dimethyl-7-oxo-2-(*p*-tolyl)-2*H*-pyrazolo[3,4-dJpyridazin-6(7H)-yl)-*N*-(4-isopropoxybenzyl)butanamide |
| 46 | 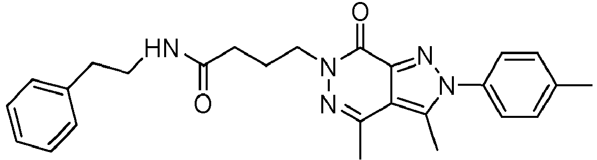 |
| | 4-(3,4-dimethyl-7-oxo-2-(*p*-tolyl)-2*H*-pyrazolo[3,4-*d*]pyridazin-6(7*H*)-yl)-*N*-phenethylbutanamide |
| 47 | 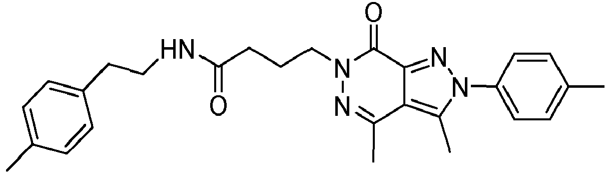 |
| | 4-(3,4-dimethyl-7-oxo-2-(*p*-tolyl)-2*H*-pyrazolo[3,4-*d*]pyridazin-6(7*H*)-yl)-*N*-(4-methylphenethyl)butanamide |
| 48 | 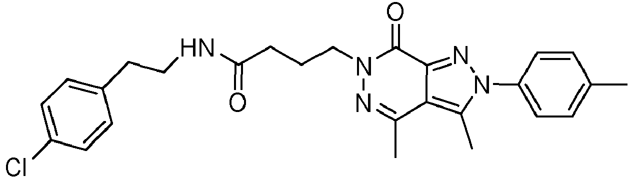 |
| | *N*-(4-chlorophenethyl)-4-(3,4-dimethyl-7-oxo-2-(p-tolyl)-2*H-*pyrazolo[3,4-*d*]pyridazin-6(7*H*)-yl)butanamide |
| 49 | 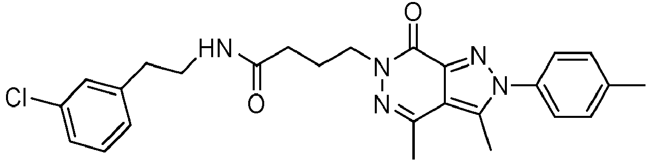 |
| | *N*-(3-chlorophenethyl)-4-(3,4-dimethyl-7-oxo-2-(*p*-tolyl)-2*H-*pyrazolo[3,4-*d*]pyridazin-6(7*H*)-yl)butanamide |
| 50 | 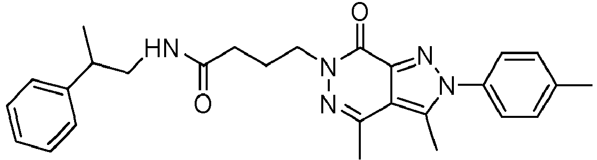 |
| | 4-(3,4-dimethyl-7-oxo-2-(*p*-tolyl)-2*H*-pyrazolo[3,4-*d*]pyridazin-6(7*H*)-yl)-*N*-(2-phenylpropyl)butanamide |
| 51 | 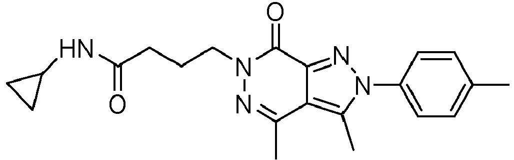 |
| | *N*-cyclopropyl-4-(3,4-dimethyl-7-oxo-2-(*p*-tolyl)-2*H*-pyrazolo[3,4-*d*]pyridazin-6(7*H*)-yl)butanamide |
| 52 | 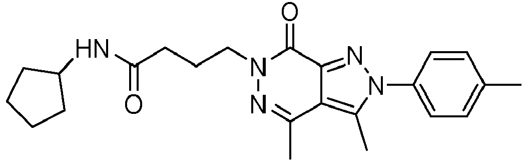 |
| | *N*-cyclopentyl-4-(3,4-dimethyl-7-oxo-2-(*p*-tolyl)-2*H*-pyrazolo[3,4-*d*]pyridazin-6(7*H*)-yl)butanamide |
| 53 | 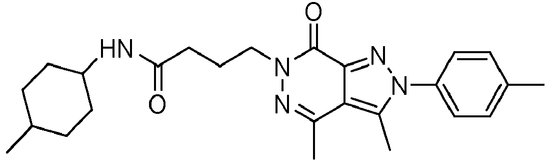 |
| | 4-(3,4-dimethyl-7-oxo-2-(*p*-tolyl)-2*H*-pyrazolo[3,4-*d*]pyridazin-6(7*H*)-yl)-*N*-(4-methylcyclohexyl)butanamide |

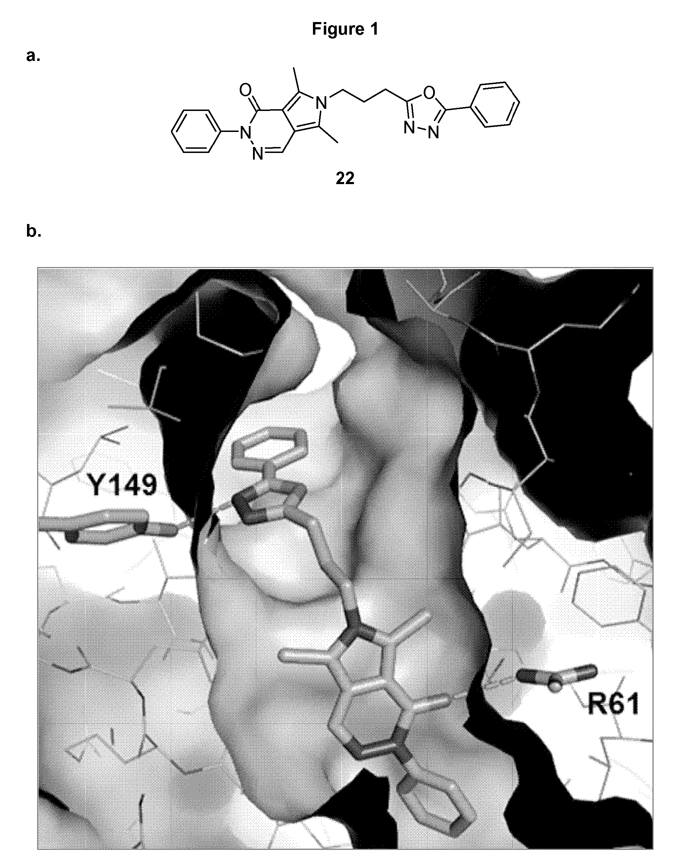

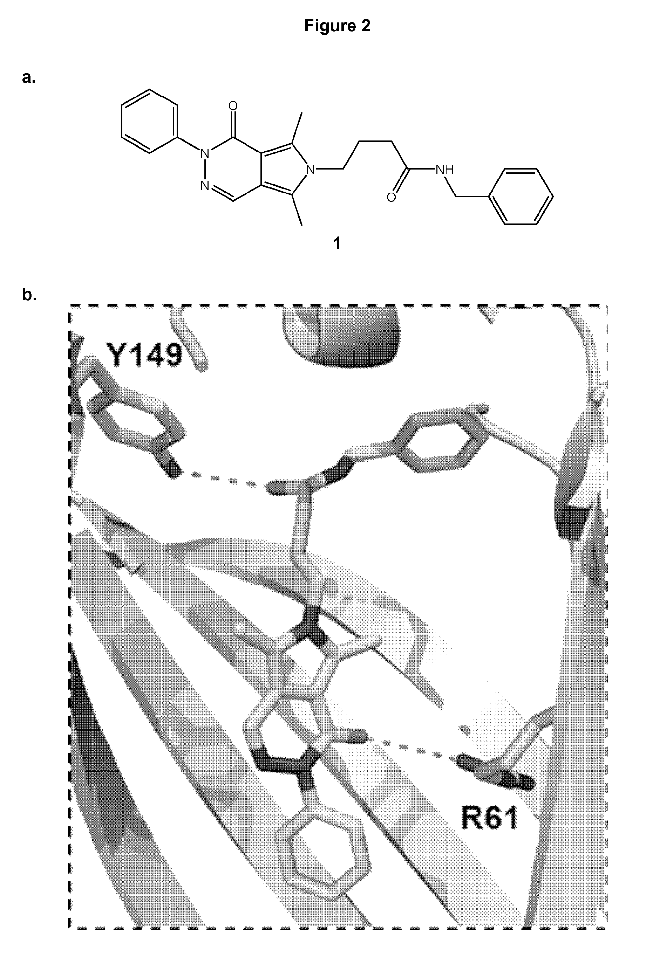

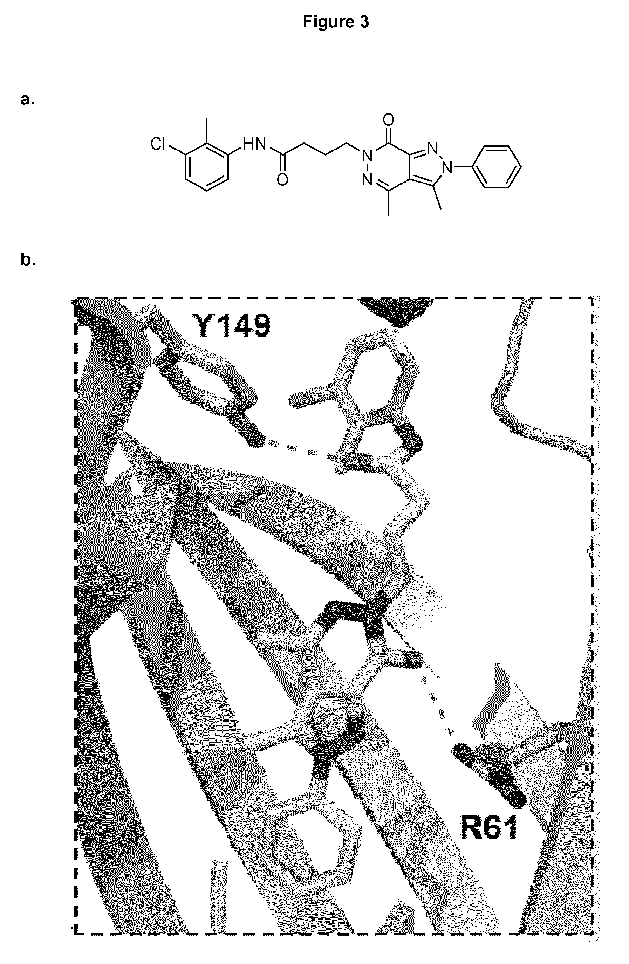

As suggested by the docking experiment (see **Figure 2****)** and confirmed by the crystal structure of the complex compound **22** / PDEδ (see **Figure 1****)** and **29** / PDEδ (see **Figure 3****),** the inventive compounds of general formula (I) are able to bind deeply in the hydrophobic tunnel of PDEδ by establishing hydrophobic interactions and two H-bondings to Tyr-149 and Arg-61.

### Chemical synthesis

The inventive pyrrolopyridazinones of general formula **(II)** according to the present invention can be prepared by methods known to one skilled in the art. Hence, pyrrolopyridazinones of general formula **(II),** wherein R⁴ represents -C(O)-NH-R⁶ i.e. pyrrolopyridazinones of general formula **(III)** can be disconnected to amine **1*** and carboxylic acid **2*** that can be further disconnected to phenylhydrazine **3*** and ester **4*,** which can be easily accessed from commercially available ethylacetoacetate, chloroacetone and 4-aminobutanoic acid (see **Scheme 1).** 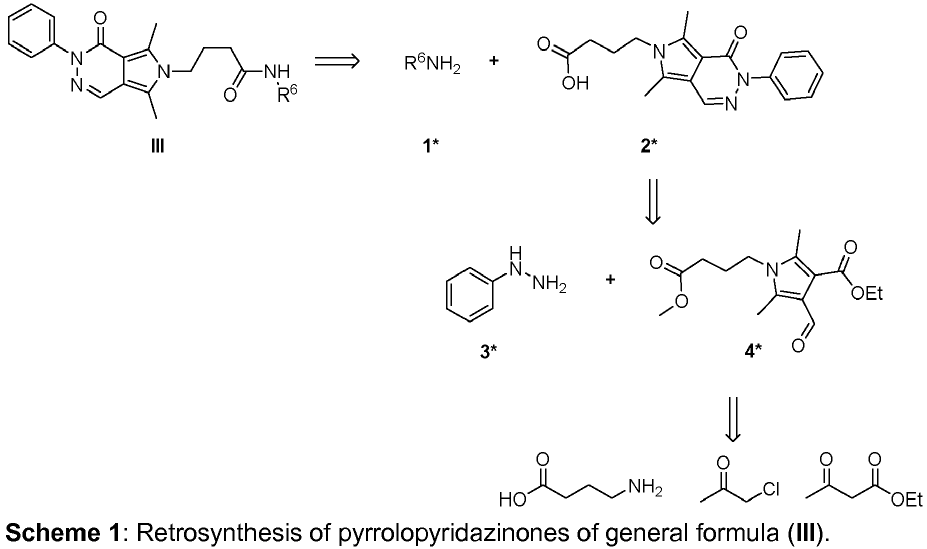 The assembly of carboxylic acid **2*** commences with the treatment of commercially available ethyl acetoacetate with chloroacetone in the presence of sodium hydride to provide intermediate **5*** that is subsequently reacted with commercially available 4-aminobutanoic acid in the presence of acetic acid to furnish *N*-substituted pyrrole **6*** (see **Scheme 2).**

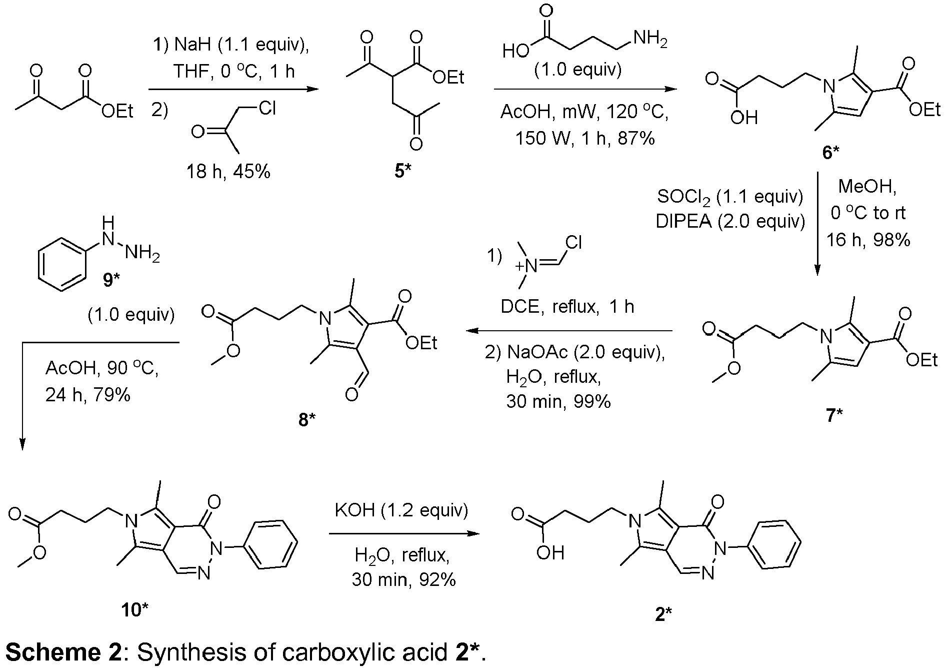

After masking the carboxylic acid as a methyl ester, intermediate **7*** is reacted with Vilsmeier reagent to introduce the aldehyde func-tion at the fourth position of the pyrrole moiety. Condensation of compound **8*** with phenylhydrazine **9*** afforded pyrrolopyridazinone **10***. Cleavage of the methyl ester on compound **10*** with potassium hydroxide provided target carboxylic acid **2***.

With carboxylic acid **2*** in hands, pyrrolopyridazinones of general formula **(III)** can be easily accessed by simple amide coupling (see **Scheme 3).** Thus, treatment of carboxylic acid **2*** with the suitable amine **1***in the presence of 1-ethyl-3-(3-dimethylaminopropyl)carbodiimide hydrochloride (EDC·HCl) and 4-dimethylaminopyridine (DMAP) provides target pyrrolopyridazinone of general formula **(III).** Conveniently, amines of general formula R⁶NH₂ are commercially available or can be easily synthesized using methods known to the person skilled in the art.

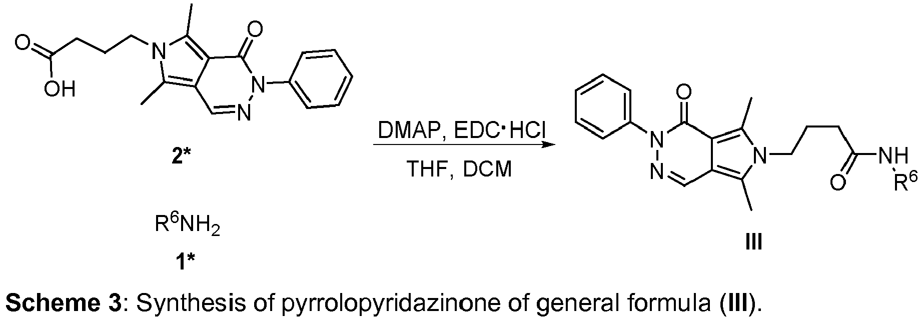

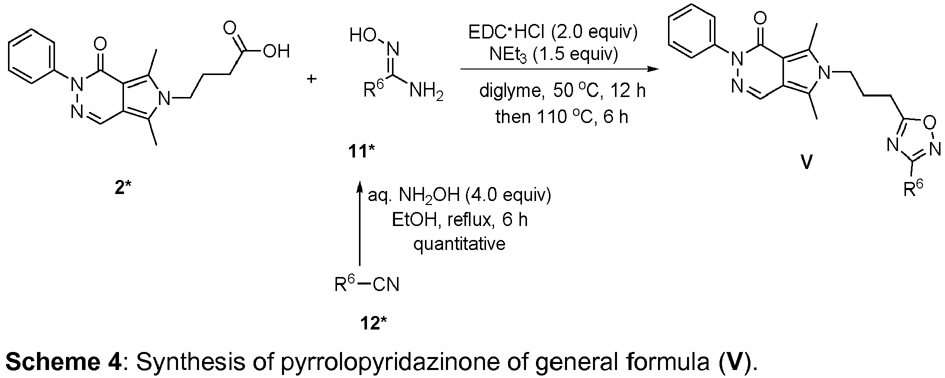

Pyrrolopyridazinones of general formula **(II),** wherein R⁴ represents a 1,2,4-oxadiazole ring substituted at the third position with the substituent R⁶ having the meaning defined above, i.e. compounds of general formula (V) can be synthesized by treatment of the common carboxylic acid intermediate **2*** with the suitable amidoxime **11*** in the presence of 1-ethyl-3-(3-dimethylaminopropyl) carbodiimide hydrochloride (EDC·HCl) and triethylamine (see **Scheme 4).** Amidoxime **11*** can be easily prepared by treating the corresponding commercially available nitrile **12*** with hydroxylamine and ethanol at reflux.

Pyrrolopyridazinones of general formula **(II),** wherein R⁴ represents a 1,3,4-oxadiazole ring substituted at the second position with the substituent R⁶ having the meaning defined above, i.e compounds of general formula **(VI)** can be accessed *via* a two-step synthetic pathway starting from carboxylic acid **2*** (see **Scheme 5).** Hence, treatment of carboxylic acid **2*** with acylhydrazine derivatives **13*** in the presence of *N,N,N',N'*-tetramethyl-O-(1*H*-benzotriazol-1-yl)uronium hexafluorophosphate (HBTU) and diisopropylethylamine (DIPEA) furnishes an intermediate that *via* cyclocondensation in the presence of tosyl chloride and DIPEA furnishes target pyrrolopyridazinones of general formula **VI.**

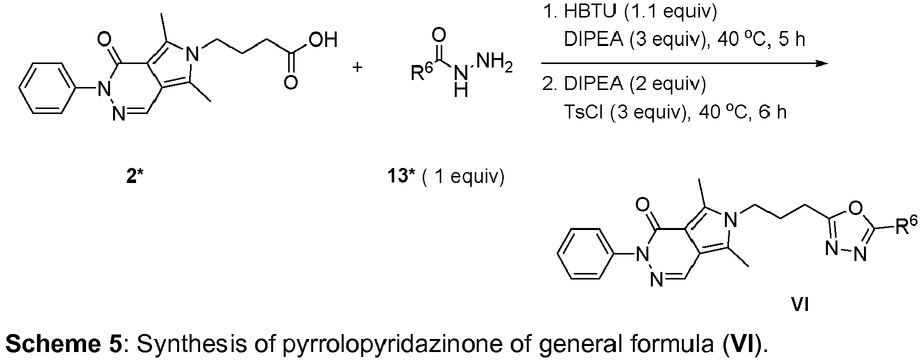

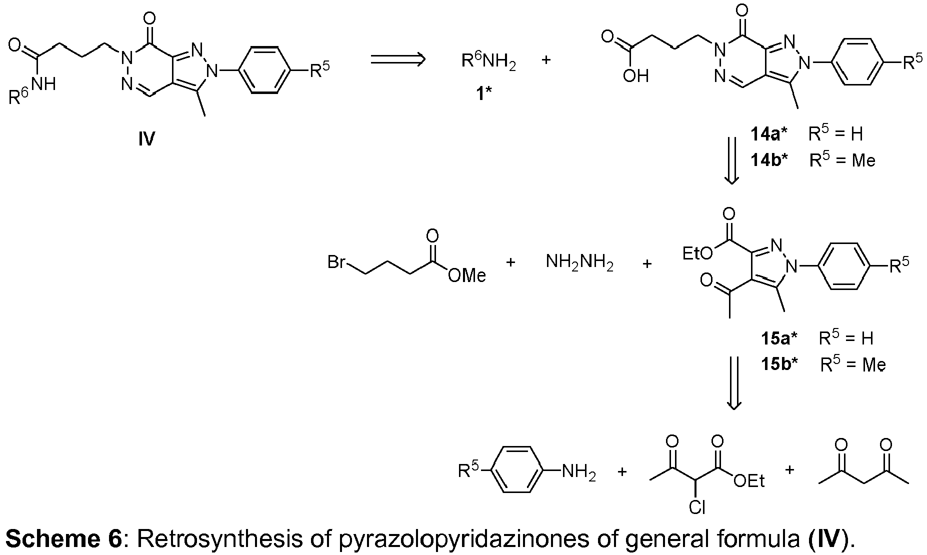

The pyrazolopyridazinone compounds of general formula **(IV)** can be disconnected to amine **1*** and carboxylic acid **14*** that can be further disconnected to methyl-4-bromobutanoate, hydrazine and ester **15*,** which can be easily accessed from commercially available aniline derivatives, 2-chloroethylacetoacetate and acetylacetone (see Scheme **6).**

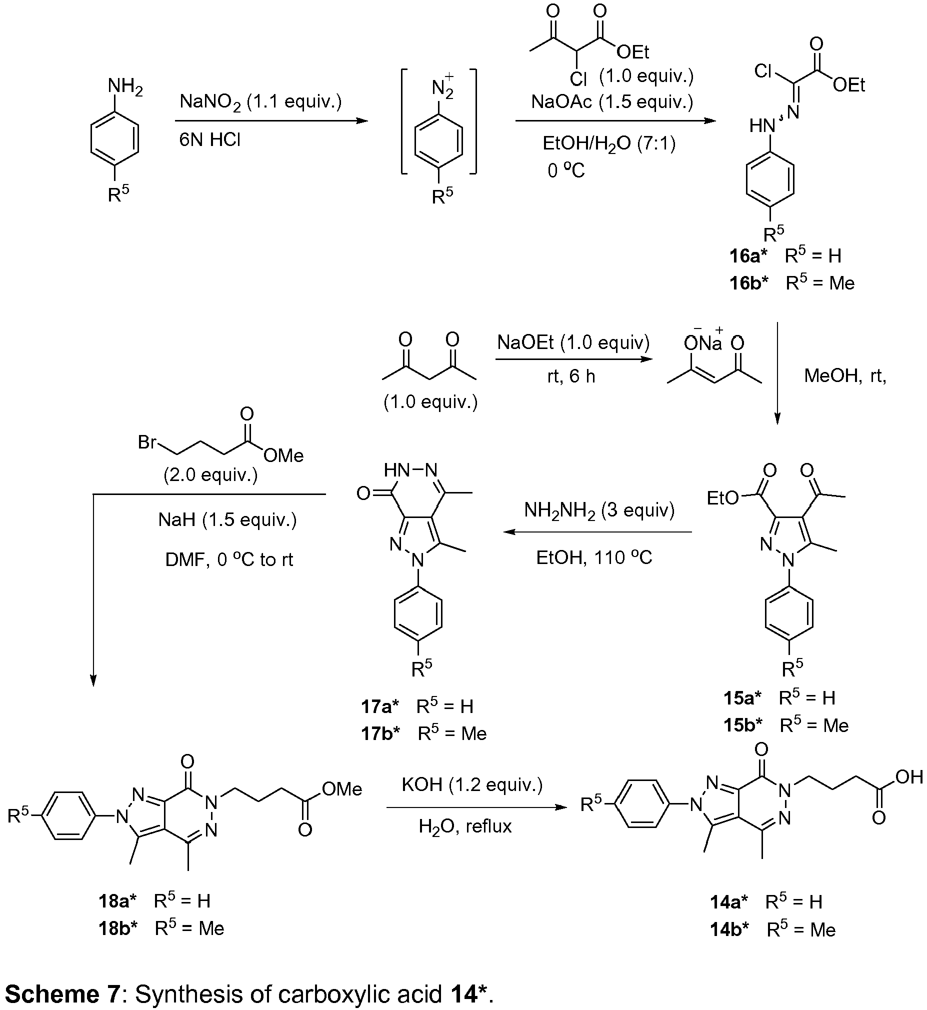

The assembly of carboxylic acid **14*** commences with the treatment of commercially available p-toluidine or aniline with sodium nitrite under acidic conditions to afford intermediate diazonium salt that is subsequently treated with 2-chloroethylacetoacetate to provide benzenediazonium chloride **16*** (see **Scheme 7).** The sodium enolate of acetylacetone was then reacted with **16*** to furnish pyrazole **15*** that is without further purification subjected to condensation reaction with hydrazine to give pyrazolopyridazinone **17*.** Finally, treatment of **17*** with methyl-4-bromobutanoate in the presence of sodium hydride delivered ester **18*.** Cleavage of the methyl ester with potassium hydroxide provided target carboxylic acid **14*.**

With carboxylic acid **14*** in hands, pyrazolopyridazinones of general formula **(IV)** can be easily accessed by simple amide coupling (see **Scheme 8).** Thus, treatment of carboxylic acid **14*** with the suitable amine **1*** in the presence of 1-ethyl-3-(3-dimethylaminopropyl)carbodiimide hydrochloride (EDC·HCl) and 4-dimethylaminopyridine (DMAP) provides target pyrazolopyridazinone of general formula **(IV).** Conveniently, amines of general formula R⁶NH₂ are commercially available or can be easily synthesized using methods known to the person skilled in the art.

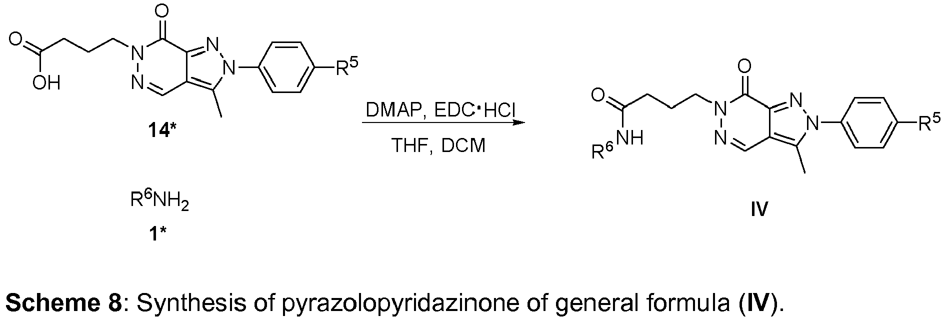

Some of the compounds of the present invention may be crystallized or recrystallized from solvents such as aqueous and organic solvents. In such cases solvates may be formed. This invention includes within its scope stoichiometric solvates including hydrates as well as compounds containing variable amounts of water that may be produced by processes such as lyophilization.

The compounds of the general formulas (I) may exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses.

The compounds of the general formula (I) may form salts with organic or inorganic acids. Examples of suitable acids for such acid addition salt formation are trifluoroacetic acid, hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

### Indications and Pharmaceutical compositions

Another aspect of the present invention relates to the use of the inventive substituted pyrrolo- and pyrazolo-pyridazinones as drugs, i.e. as pharmaceutically active agents applicable in medicine.

Surprisingly, it was found that the above-mentioned pyridazinones, as well as the pharmaceutical compositions comprising said pyridazinones are useful for treatment or prophylaxis of cancer, tumors and proliferative diseases, preferably cancer, tumors and proliferative diseases caused by or associated with K-Ras.

Thus, the pyridazinone compounds of the present invention can be used for prophylaxis and/or treatment of cancers, tumors and proliferative diseases or for the preparation of a pharmaceutical formulation for prophylaxis and/or treatment of cancers, tumors and proliferative diseases, preferably cancer, tumors and proliferative diseases caused by or associated with K-Ras.

More specifically, the cancers, tumors and proliferative diseases that can be treated and/or prevented by the inventive compounds are selected from the group comprising or consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioma, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

Cancer cells often depend on the survival signaling emanating from oncogene products, particularly from oncogenic K-Ras, for their survival. The induction of programmed cell death by the loss of such survival signaling, as disclosed for the compounds of the present invention, is especially useful in the treatment of cancer by inducing the death of oncogenic Ras dependent malignant cells. Since all kinds of cancer cells are destroyable through the induction of programmed cell death, all different kinds of cancer and abnormal proliferating cells can be treated with the compounds of the present invention.

Therefore, another aspect of the present invention is directed to pharmaceutical compositions comprising at least one compound of the present invention as active ingredient, together with at least one pharmaceutically acceptable carrier, excipient and/or diluent. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention.

The pharmaceutical compositions according to the present invention containing at least one compound according to the present invention as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the inventive pyridazone of general formula (I) as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimize the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood, which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilized in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances, which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

### Description of the Figures:

**Figure 1** shows **a)** the structure of compound **22; b)** the crystal structure of inhibitor **22** in complex with PDEδ at 2.0 A resolution showing H-bonding interactions with Tyr149 and Arg61.
**Figure 2** shows **a)** the structure of compound **1; b)** the modeling (Schrödinger, Maestro suite) of compound **1** into the prenyl binding site of PDEδ, showing H-bonding interactions with Tyr149 and Arg61.
**Figure 3** shows **a)** the structure of compound **29; b)** crystal structure of compound **29** in complex with PDEδ at 2.6 A resolution H-bonding interactions with Tyr149 and Arg61.

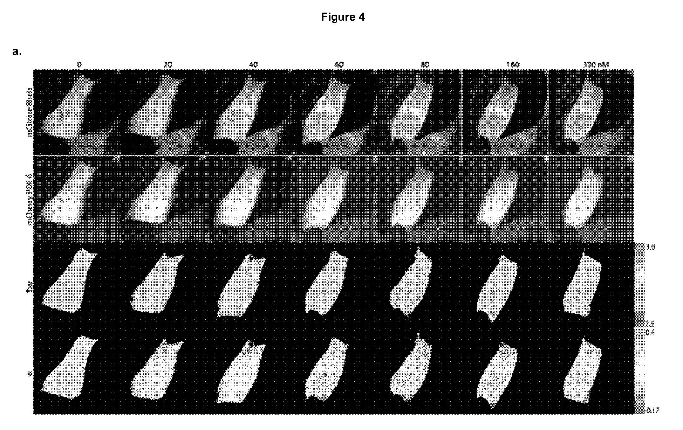

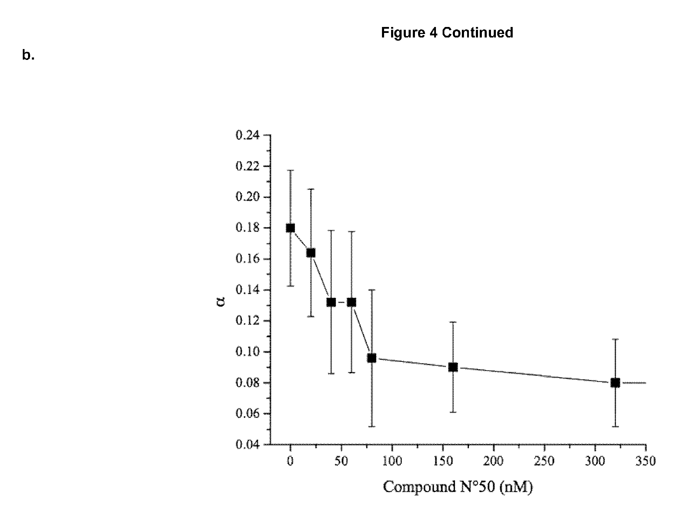

**Figure 4** shows **a)** compound **50** dose dependence of molar fraction (α) of interacting mCitrine-Rheb with mCherry-PDEδ. First row shows fluorescence intensity distribution of mCitrine-Rheb, second row shows fluorescence intensity distribution of mCherry-PDEδ, third row represents average mCitrine fluorescence lifetime (tₐᵥ) in ns and forth row represents molar fraction (α) of interacting mCitrine-Rheb with mCherry-PDEδ. The concentration of compound **50** is indicated at the top of the panel in nM; **b)** Fit of the dose-response relationship of molar interacting fraction (α) in dependence to increasing concentration of compound **50** to a binding model (n=5).

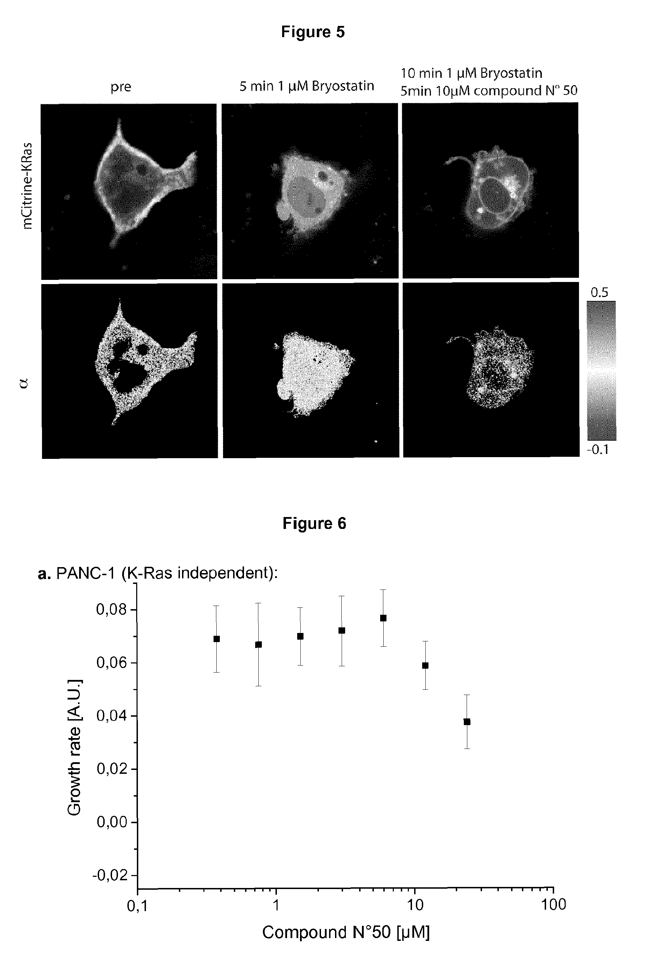

**Figure 5** shows K-Ras-PDEδ interaction and inhibition. Upper row: fluorescence intensity of mCitrine-K-Ras distribution, lower row: molar fraction (α) of interacting mCitrine-Rheb with mCherry-PDEδ. To monitor if K-Ras is indeed a client of the Arl-PDEδ system, the non-plasma membrane bound fraction of K-Ras was increased by a PKC induced (Bryostatin) phosphorylation of a Serine (S181) located in its polybasic stretch. The administration of Bryostatin relocated K-Ras and increased the interacting fraction (middle column) with PDEδ. Subsequent addition of compound 50 (right column) abolished the interaction between K-Ras and PDEδ.

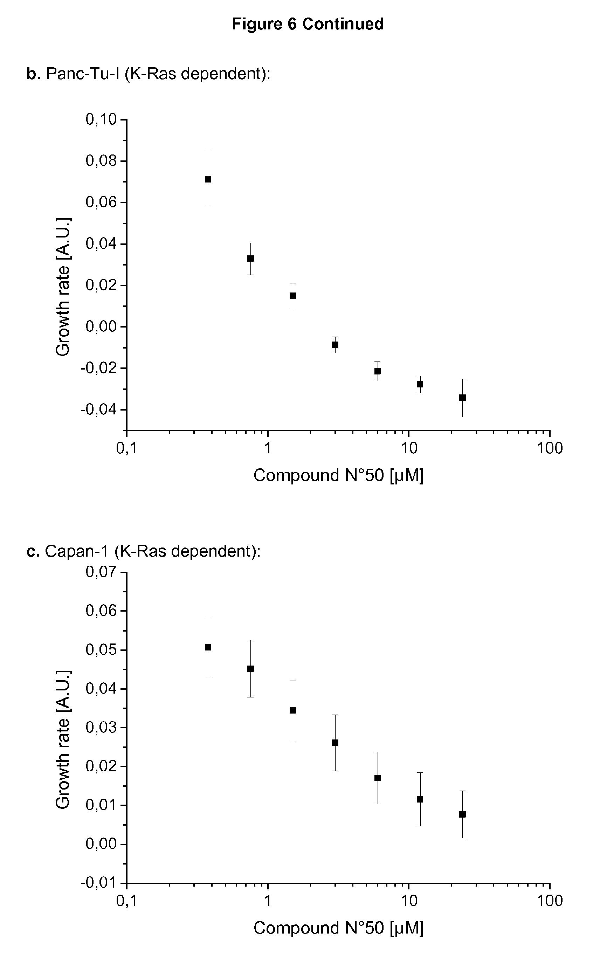

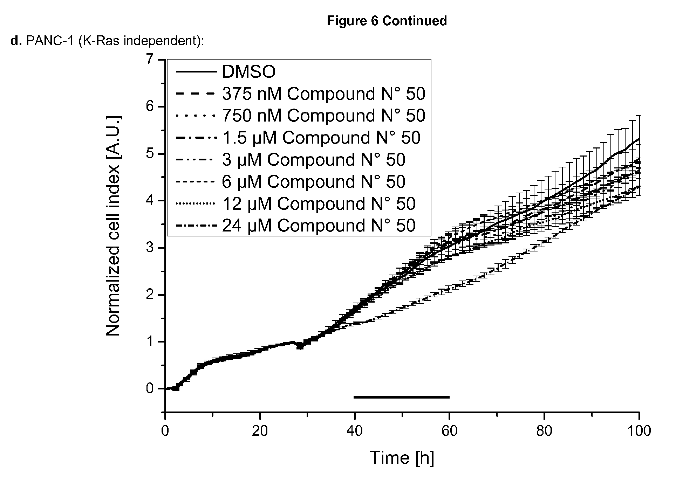

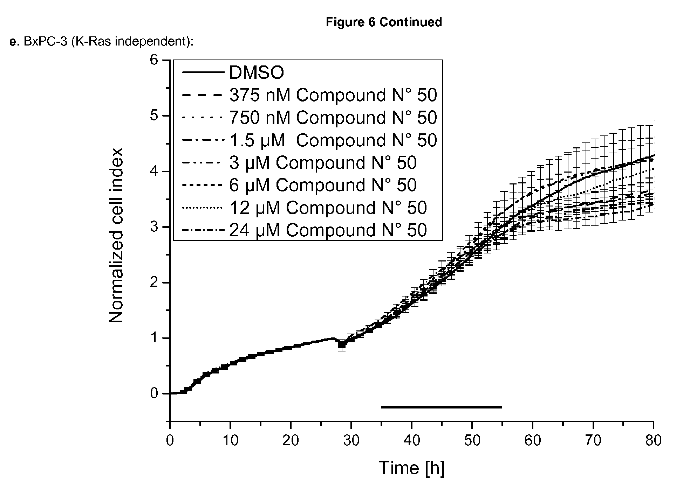

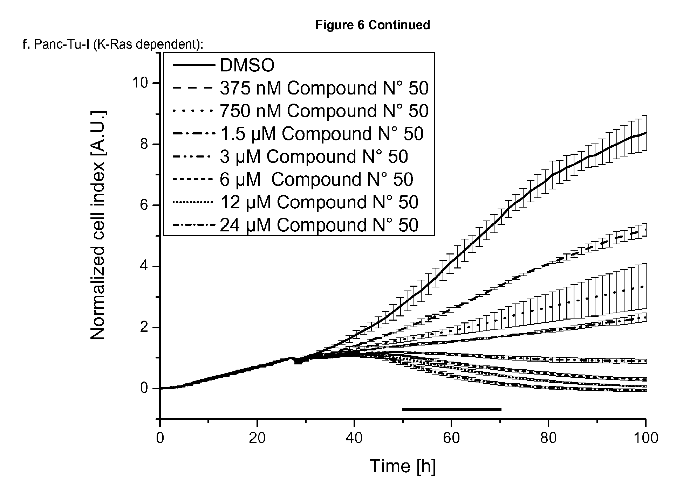

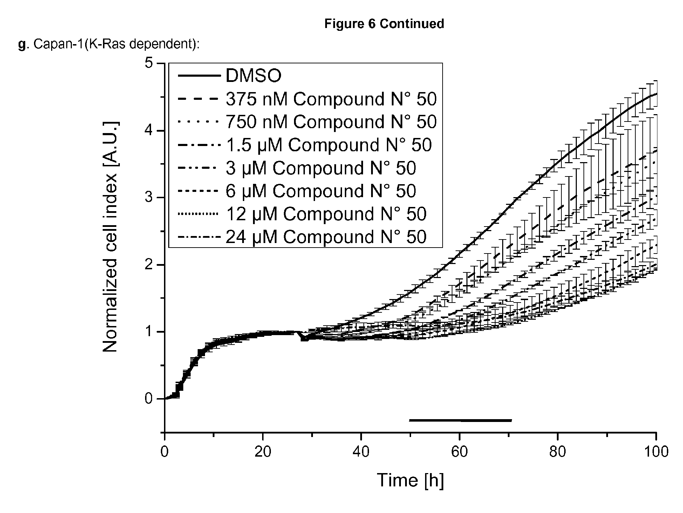

**Figure 6** shows continuous impedance measurements (RTCA) (monitored every 15 min up to 100 hours) of compound **50** dose-dependent cell proliferation response (for PANC-1 (see Figure 6d), BxPC-3 (see Figure 6e), Panc-Tu-I (see Figure 6f), Capan-1 (see Figure 6g)). Compound 50 was administered 24 hours after seeding at indicated concentrations. Figures 6a, 6b, 6c show the growth rates calculated from the RTCA curves shown in **Figures 6d-6g** over the indicated time span **(****Figure 6a****:** 40 to 60 min; **Figure 6b****:** 50 to 70 min; **Figure 6c****:** 50 to 70 min; time span indicated by a black bar in **Figures 6d****,** **6f** **and** **6g****)** at given concentrations of compound **50.**

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### A. Chemical Synthesis

### General Information:

All reactions involving air- or moisture-sensitive reagents or intermediates were carried out in flame dried glassware under an argon atmosphere. Dry solvents (THF, toluene, MeOH, DMF) were used as commercially available; CH₂Cl₂ was purified by the Solvent Purification System M-BRAUN Glovebox Technology SPS-800. Analytical thin-layer chromatography (TLC) was performed on Merck silica gel aluminium plates with F-254 indicator. Compounds were visualized by irradiation with UV light or potassium permanganate staining. Column chromatography was performed using silica gel Merck 60 (particle size 0.040-0.063 mm). ¹H-NMR and ¹³C-NMR were recorded on a Bruker DRX400 (400 MHz), Bruker DRX500 (500 MHz) and INOVA500 (500 MHz) at 300 K using CDCl₃ or (CD₃)₂SO as solvent. All resonances are reported relative to TMS. Spectra were calibrated relative to solvent's residual proton and carbon chemical shift: CDCl₃ (δ = 7.26 ppm for ¹H NMR and δ = 77.16 ppm for ¹³C NMR); (CD₃)₂SO: δ= 2.50 ppm for ¹H NMR and δ= 39.52 ppm for ¹³C NMR). Multiplicities are indicated as: br s (broadened singlet), s (singlet), d (doublet), t (triplet), q (quartet), quin (quintet), m (multiplet); and coupling constants (J) are given in Hertz (Hz). High resolution mass spectra were recorded on a LTQ Orbitrap mass spectrometer coupled to an Acceka HPLC-System (HPLC column: Hypersyl GOLD, 50 mm x 1 mm, particle size 1.9 µm, ionization method: electron spray ionization). Fourier transform infrared spectroscopy (FT-IR) spectra were obtained with a Bruker Tensor 27 spectrometer (ATR, neat) and are reported in terms of frequency of absorption (cm⁻¹). Atorvastatin was purchased from Sequoia Reseach Products. All chemicals and solvents were purchased from Sigma-Aldrich, Fluka, TCI, Across Organics, ABCR and Alfa Aesar. Unless otherwise noted, all commercially available compounds were used as received without further purifications.

### Example A.1: Synthesis of ethyl 2-acetyl-4-oxopentanoate (5*)

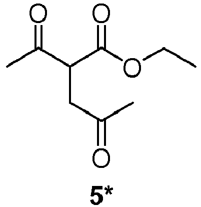

A solution of ethyl acetoacetate (13.01 g, 100.0 mmol) in THF (150 mL) was cooled to 0 °C and NaH (60 % in mineral oil, 4.40 g, 110.0 mmol, 1.1 equiv) was added to it slowly over 15 min. After complete addition, the mixture was stirred at room temperature for another 1 h. Then chloroacetone (7.96 mL, 100.0 mmoL, 1.0 equiv.) was added dropwise and the mixture was stirred for an additional 16 h at rt. The volatile components were removed and the residue was re-dissolved in diethyl ether (100 mL). The solution was washed with water (20 ml x 2) before being dried over Na₂SO₄ and concentrated *in vacuo.* Crude product was purified by flash column chromatography (FCC) on silica gel using 10% ethyl acetate/Petroleum ether (EA/PE) as an eluent to provide analytically pure product **5*** (6.0 g, 33% yield) as a colorless liquid. FT-IR (neat): *ṽ* = 2984, 1739, 1711, 1359, 1259, 1157, 1019, 866, 744 cm⁻¹. ¹H NMR (500 MHz, CDCl₃) δ 4.17 (q, *J* = 7.1 Hz, 2H), 3.99 (dd, *J* = 8.2, 5.7 Hz, 1H), 3.12 (dd, *J* = 18.5, 6.9 Hz, 1H), 2.93 (dd, *J* = 18.5, 6.9 Hz, 1 H), 2.33 (s, 3H), 2.17 (s, 3H), 1.26 (t, *J =* 7.1 Hz, 3H). ¹³C NMR (126 MHz, CDCl₃) δ 205.7, 202.3, 168.9, 61.8, 53.9, 41.6, 30.2, 29.8, 14.1. HRMS (ESI): calc. for [M+Na]⁺ C₉H₁₄O₄Na: 209.07843, found: 209.07843.

### Example A.2: Synthesis of 4-(3-(ethoxycarbonyl)-2,5-dimethyl-1 H-pyrrol-1-yl)butanoic acid (6*)

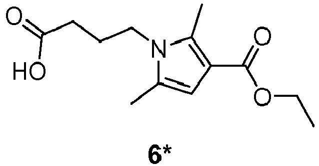

Ethyl 2-acetyl-4-oxopentanoate (**5***) (0.56 g, 3.0 mmol, 1 equiv) and 4-aminobutanoic acid (0.31 g, 1.0 equiv.) were dissolved in acetic acid (2.62 mL) and stirred at ambient temperature for 30 min. After all solid had dissolved, the reaction mixture was heated in a CEM microwave reactor (close vessel, 150 W, 120 °C) for 30 min. The reaction was completed after 30 min as monitored by TLC (40% EA/PE). After completion, the reaction mixture was diluted with ethyl acetate (10 mL) and acetic acid was neutralized using saturated NaHCO₃ (If excess NaHCO₃ was used, then the desired compound was transferred to the H₂O layer, which could be recovered by neutralization with acid to pH 4). The aqueous layer was extracted with ethyl acetate (2 x 20 mL); the combined organic layers were sequentially washed with H₂O (10 ml x 2), brine (10 mL), dried over Na₂SO₄, filtered and concentrated *in vacuo* to give the desired product **6*** (0.66 g, 87%) as a brown solid in good purity. The compound was used in the next step without further purification. FT-IR (neat): *ṽ* = 3545, 2942, 1942, 1743, 1708, 1559, 1475, 1371, 1248, 1222, 1095, 815, 775 cm⁻¹. ¹H NMR (400 MHz, CDCl₃) δ 10.75 (s, 1 H), 6.25 (s, 1 H), 4.23 (q, *J* = 7.1 Hz, 2H), 3.82 (t, *J* = 7.6 Hz, 2H), 2.51 (s, 3H), 2.40 (t, *J* = 7.0 Hz, 2H), 2.19 (s, 3H), 2.02 - 1.81 (m, 2H), 1.31 (t, *J* = 7.1 Hz, 3H). ¹³C NMR (101 MHz, CDCl₃) δ 178.1, 166.0, 135.1, 127.4, 110.9, 107.8, 59.4, 42.6, 30.7, 25.2, 14.6, 12.20, 11.40. HRMS (ESI): calc. for [M+H]⁺ C₁₃H₂₀O₄N: 254.13868, found: 254.13817.

### Example A.3: Synthesis of ethyl 1-(4-methoxy-4-oxobutyl)-2,5-dimethyl-1H-pyrrole-3-carboxylate (7*)

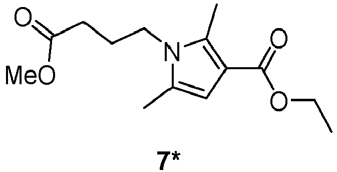

To a solution of **6*** (3,36 g, 13.26 mmol, 1 equiv) and DIPEA (4.62 mL, 26. 53 mmol, 2.0 equiv) in anhydrous methanol (40 mL) at 0 °C was added drop wise thionyl chloride (1.15 mL, 15.92 mmol, 1.2 equiv) and the resulting reaction mixture was left to stir for 16 h. After completion, volatile components were removed and the reaction mixture was diluted with dichloromethane (25 mL) and water (20 mL). The aqueous layer was extracted with dichloromethane (2 x 10 mL); the combined organic layers were sequentially washed with H₂O (10 ml x 2), brine (20 mL), dried over Na₂SO₄,filtered and concentrated under vacuum to give the desired product **7*** (3.47 g, 98%) in good purity. FT-IR (neat): *ṽ* = 2951, 1735, 1689, 1578, 1532, 1420, 1371, 1218, 1184, 1171, 1093, 1062, 867, 772 cm⁻¹. ¹H NMR (400 MHz, CDCl₃) δ 6.24 (s, 1 H), 4.22 (q, *J* = 7.1 Hz, 2H), 3.80 (t, *J* = 7.6 Hz, 2H), 3.68 (s, 3H), 2.51 (s, 3H), 2.34 (t, *J* = 7.0 Hz, 2H), 2.19 (s, 3H), 1.92 (quin, *J =* 7.4 Hz, 2H), 1.31 (t, *J =* 7.1 Hz, 3H). ¹³C NMR (101 MHz, CDCl₃) δ 173.1, 165.8, 135.0, 127.4, 111.0, 107.8, 59.2, 51.9, 42.7, 30.7, 25.5, 14.6, 12.2, 11.4. HRMS (ESI): calc. for [M+H]⁺ C₁₄H₂₂O₄N: 268.15433, found: 268.15384.

### Example A.4: Synthesis of ethyl 4-formyl-1-(4-methoxy-4-oxobutyl)-2,5-dimethyl-1 H-pyrrole-3-carboxylate (8*)

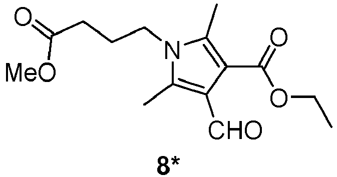

Phosphorus oxychloride (1.91 mL, 20.5 mmol, 1.6 equiv) was added to anhydrous DMF (1.99 mL, 25.7 mmol, 2.0 equiv) at 0 °C during 8 min, and the resulting mixture was stirred at the same temperature for 30 min. Then, the reaction mixture was diluted by 1,2-dichloroethane (12 mL), followed by addition of a solution of pyrrole derivative **7*** (3.43 g, 12.83 mmol, 1.0 equiv) in 1,2-dichloroethane (20 mL). The mixture was then heated at reflux for 45 min. After slight cooling, a solution of sodium acetate (4.21 g, 51.32 mmol, 4.0 equiv) in H₂O (20.15 mL) was added and the mixture was reheated at reflux for an additional 30 min. After completion, the reaction mixture was allowed to come to room temperature and was diluted with dichloromethane (30 mL) and water (20 mL). The aqueous layer was extracted with dichloromethane (2 x 10 mL); the combined organic layers were sequentially washed with H₂O (10 ml x 2), brine (25 mL), dried over Na₂SO₄,filtered and concentrated under vacuum to give the desired product **8*** (3.79 g, 99%) in good purity. FT-IR (neat): *ṽ* = 2986, 2925, 1732, 1702, 1648, 1555, 1525, 1438, 1361, 1267, 1197, 1178, 1141, 1096, 971, 890, 869, 777 cm⁻¹. ¹H NMR (400 MHz, CDCl₃) δ 10.37 (s, 1 H), 4.29 (q, *J* = 7.1 Hz, 2H), 3.86 (t, *J* = 8.0 Hz, 2H), 3.67 (s, 3H), 2.53 (s, 3H), 2.50 (s, 3H), 2.37 (t, *J* = 6.8 Hz, 2H), 1.90 (quin, *J* = 7.4 Hz, 2H), 1.33 (t, *J =* 7.1 Hz, 3H). ¹³C NMR (101 MHz, CDCl₃) δ 190.1, 172.8, 165.1, 136.2, 136.1, 120.0, 112.3, 60.1, 52.0, 42.5, 30.5, 25.0, 14.5, 11.6, 11.4. HRMS (ESI): calc. for [M+H]⁺ C₁₅H₂₂O₅N: 296.14925, found: 296.14893.

### Example A.5: Synthesis of methyl 4-(5,7-dimethyl-1-oxo-2-phenyl-1 H-pyrrolo[3,4-d]pyridazin-6(2H)-yl)butanoate (10*)

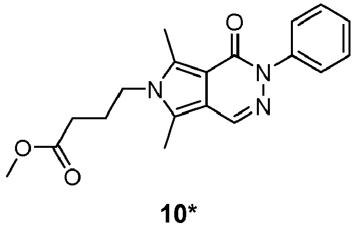

Pyrrolo precursor **8*** (3.74 g, 12.7 mmol, 1 equiv) and phenyl hydrazine **9*** (1.31 mL, 13.31 mmol, 1.05 equiv) were dissolved in acetic acid (52 mL) and the mixture was heated to 90 °C for 24 h. After completion, the reaction was diluted with DCM and aqueous layer was extracted with dichloromethane (3 x 20 mL). The combined organic layers were sequentially washed with saturated NaHCO₃ solution (20 mL x 2), H₂O (20 ml x 2) and brine (25 mL), dried over Na₂SO₄, filtered and concentrated under vacuum. The crude residue was further purified by FCC on silica-gel using 2% MeOH/CH₂Cl₂ as an eluent to afford desired **10*** (3.42 g, 79%). FT-IR (neat): *ṽ* = 2941, 1726, 1658, 1561, 1313, 1255, 1089, 978, 956, 764, 697 cm⁻¹. ¹H NMR (400 MHz, CDCl₃) δ 8.03 (s, 1 H), 7.58 (d, *J* = 7.7 Hz, 2H), 7.44 (t, *J* = 7.7 Hz, 2H), 7.30 (t, *J* = 7.7 Hz, 2H), 4.04 (t, *J* = 7.8 Hz, 2H), 3.72 (s, 3H), 2.75 (s, 3H), 2.50 (s, 3H), 2.42 (t, *J* = 6.8 Hz, 2H), 2.09 - 1.97 (m, 2H). ¹³C NMR (101 MHz, CDCl₃) δ 172.8, 159.3, 142.5, 134.0, 129.8, 128.5, 126.9, 126.4, 122.6, 115.6, 111.3, 52.0, 43.4, 30.5, 25.2, 11.1, 10.3. HRMS (ESI): calc. for [M+H]⁺ C₁₉H₂₂O₃N₃: 340.16557, found: 340.16539.

### Example A.6: Synthesis of 4-(5,7-Dimethyl-1-oxo-2-phenyl-1H-pyrrolo[3,4-d]pyridazin-6(2H)-yl)butanoic acid (2*)

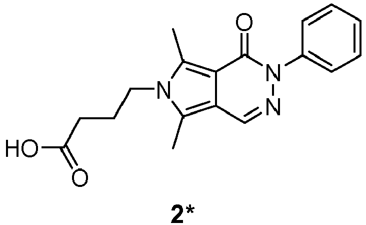

A heterogeneous mixture of pyrrolopyridazone derivative **10*** (3.37 g, 9.93 mmol, 1.0 equiv) and KOH (0.67 g, 11.92 mmol, 1.2 equiv) in H₂O (43 mL) was heated to reflux until all the solid had dissolved (it takes around 30 min). Then the reaction mixture was allowed to come to room temperature, cooled to 0 °C and was acidified with 10% HCl solution to a pH of 5-6. The resultant precipitation was then filtered, dried under vacuum to yield analytically pure **2*** as a light brown solid (3.0 g, 92%). FT-IR (neat): *ṽ* = 3304, 1687, 1645, 1542, 1505, 1455, 1391, 1316, 1207, 1024, 1091, 962, 832, 767, 698 cm⁻¹. ¹H NMR (400 MHz, DMSO) δ 12.28 (s, 1H), 8.23 (s, 1 H), 7.48 (d, *J* = 7.5 Hz, 2H), 7.42 (t, *J* = 7.7 Hz, 2H), 7.29 (t, *J* = 7.2 Hz, 1 H), 4.05 (t, *J* = 7.8 Hz, 2H), 2.64 (s, 3H), 2.49 (s, 3H), 2.34 (t, *J* = 6.9 Hz, 2H), 1.85 (quin, J = 7.6 Hz, 2H). ¹³C NMR (101 MHz, DMSO) δ 173.7, 158.2, 142.5, 134.4, 129.3, 128.1, 126.3, 126.3, 123.7, 114.6, 109.9, 43.1, 30.4, 24.9, 10.7, 9.7. HRMS (ESI): calc. for [M+H]⁺ C₁₈H₂₀O₃N₃: 326.14992, found: 326.14948.

### Example A.7: General procedure I for amide coupling

To a solution of acid derivative **2*** (0.049 g, 0.15 mmol, 1.0 equiv) and DMAP (0.024 g, 0.195 mmol, 1.3 equiv) in THF/DCM (1:1, 0.6 mL and 0.6 mL) was added EDC·HCl (0.037 g, 0.195 mmol, 1.3 equiv), followed by the corresponding amine derivative **1*** (0.158 mmol, 1.05 equiv) and the resulting solution was stirred for 16 h at ambient temperature. Once completed, as observed by TLC, the reaction mixture was diluted with CH₂Cl₂ (5 mL) and quenched by adding saturated NaHCO₃ solution (5 mL). The aqueous layer was extracted with dichloromethane (2 x 5 mL) and combined organic layers were sequentially washed with saturated NH₄Cl solution (5 mL x 2), H₂O (5 ml x 2) and brine (5 mL); dried over Na₂SO₄, filtered and concentrated under vacuum. The crude residue was further purified by FCC on silica-gel using 2% MeOH/CH₂Cl₂ as an eluent to afford desired compounds.

### Example A.7.1: Synthesis of N-cyclohexyl-4-(5,7-dimethyl-1-oxo-2-phenyl-1H-pyrrolo[3,4-d]pyridazin-6(2H)-yl)butanamide (11)

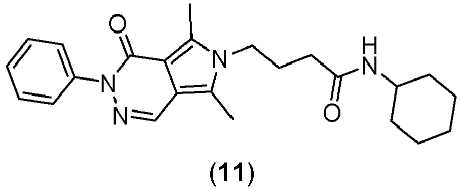

The title compound (0.05 g, 89%) was prepared according to general procedure I. FT-IR (neat): *ṽ* = 3287, 2929, 2359, 2341, 1634, 1543, 1446, 1321, 1124, 963, 757, 725, 693 cm⁻¹. ¹H NMR (400 MHz, DMSO) δ 8.23 (s, 1 H), 7.74 (d, *J* = 7.7 Hz, 1 H), 7.47 (d, *J* = 7.4 Hz, 2H), 7.42 (t, *J* = 7.6 Hz, 2H), 7.34 - 7.26 (m, 1 H), 4.02 (t, *J* = 7.5 Hz, 2H), 3.58 - 3.46 (m, 1 H), 3.35 (s, 3H), 2.63 (s, 3H), 2.13 (t, *J* = 6.9 Hz, 2H), 1.94 - 1.79 (m, 2H), 1.77 - 1.61 (m, 4H), 1.60 - 1.49 (m, 1 H), 1.32 - 1.18 (m, 2H), 1.17 - 1.03 (m, 3H). ¹³C NMR (101 MHz, DMSO) δ 169.9, 158.2, 142.5, 134.4, 129.3, 128.1, 126.3, 126.2, 123.8, 114.6, 109.9, 47.4, 43.3, 32.5, 31.8, 25.4, 25.3, 24.6, 10.7, 9.8. HRMS (ESI): calc. for [M+H]⁺ C₂₄H₃₁O₂N₄: 407.24415, found: 407.24318.

### Example A.8

### Synthesis of pyrrolopyridazinones of general formula V

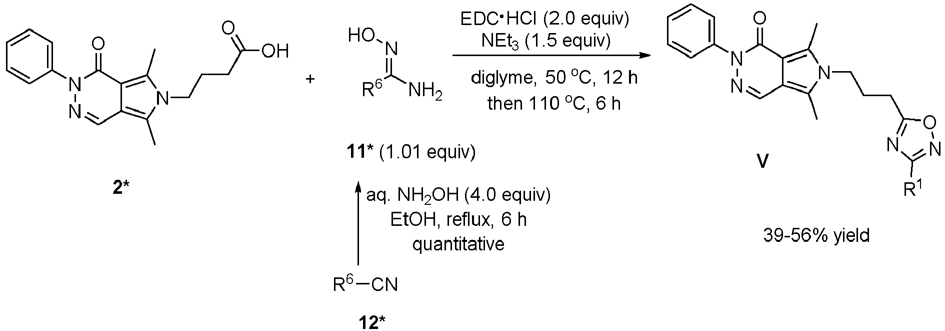

### General procedure II for the synthesis pyrrolopyridazinones of general formula V presenting a 1,2,4-oxadiazole ring:

Hydroxylamine (50% by weight in H₂O, 1.32 mL, 20 mmol, 4 equiv) and the corresponding nitrile derivatives **12*** (5 mmol, 1 equiv) were combined in EtOH (20 mL) and heated to reflux for 2 h. Then the reaction mixture was cooled to 23 °C and was concentrated *in vacuo* to give the corresponding amidoxime derivatives **11**^{*} as white solids in quantitative yields.(A. R. Gangloff, J. Litvak, E. J. Shelton, D. Sperandio, V. R. Wang, K. D. Rice, Tetrahedron Lett. 2001, 42, 1441.) The analytical data of synthesized amidoxime derivatives **11*** were in good correlation with literature reported data. (X. Yang, G. Liu, H. Li, Y. Zhang, D. Song, C. Li, R. Wang, B. Liu, W. Liang, Y. Jing, G. Zhao, J. Med. Chem. 2010, 53, 1015; C. Quan, M. Kurth, J. Org. Chem. 2004, 69, 1470.)

Synthesized amidoxime derivatives **11*** (0.101 mmol, 1.01 equiv) were treated with acid precursor **2*** (0.033 g, 0.1 mmol, 1 equiv) in the presence of EDC·HCl (0.038 g, 0.2 mmol, 2 equiv) and triethylamine (0.028 mL, 0.2 mmol, 2 equiv) in diglyme (2-3 mL) and heated to 50 °C for 12 h. Then the reaction mixture was further heated to 110 °C for 5 h for the cyclodehydration step to take place. After completion, the reaction mixture was diluted with dichloromethane (5 mL) and water (5 mL). The aqueous layer was extracted with dichloromethane (2 x 5 mL); the combined organic layers were sequentially washed with H₂O (10 ml x 2), saturated brine (5 mL), dried over Na₂SO₄,filtered and concentrated *in vacuo* to give crude **V.** The resulting crude material was purified by FCC on silica gel using 3% methanol/dichloromethane as an eluent to give the desired 1,2,4-oxadiazole derivatives **V** in 39-56% yields.

### Example A.8.1: Synthesis of 5,7-dimethyl-2-phenyl-6-(3-(3-phenyl-1,2,4-oxadiazol-5-yl)propyl)-2,6-dihydro-1H-pyrrolo[3,4-d]pyridazin-1-one (16)

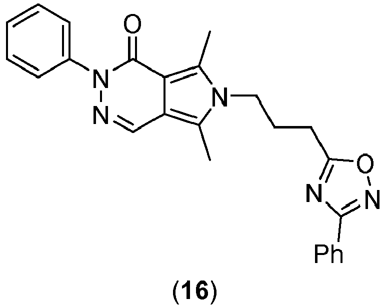

The title compound was prepared according to general procedure II (0.023 g, 53%). FT-IR (neat): *ṽ* = 2922, 1652, 1594, 1444, 1351, 1306, 1116, 1090, 961, 903, 720, 692 cm⁻¹. ¹H NMR (500 MHz, CDCl₃) δ 8.07 (dd, *J* = 8.1, 1.6 Hz, 2H), 8.01 (s, 1 H), 7.55 (dd, *J* = 8.4, 1.1 Hz, 2H), 7.53 - 7.48 (m, 3H), 7.45 - 7.40 (m, 2H), 7.30 (t, *J =* 7.4 Hz, 1 H), 4.19 (t, *J* = 7.8 Hz, 2H), 3.05 (t, *J* = 7.0 Hz, 2H), 2.78 (s, 3H), 2.52 (s, 3H), 2.33 (quin, *J* = 7.5 Hz, 2H). ¹³C NMR (126 MHz, CDCl₃) δ 178.1, 168.5, 142.5, 133.9, 131.5, 129.8, 129.1, 128.8, 128.6, 127.5, 127.0, 126.6, 126.5, 122.5, 115.9, 111.6, 43.3, 26.7, 23.8, 11.3, 10.5. HRMS (ESI): calc. for [M+H]⁺ C₂₅H₂₄O₂N₅: 426.19245, found: 426.19208.

### Example A.8.2: Synthesis of 5,7-dimethyl-2-phenyl-6-(3-(3-(p-tolyl)-1,2,4-oxadiazol-5-yl)propyl)-2,6-dihydro-1H-pyrrolo[3,4-d]pyridazin-1-one (17)

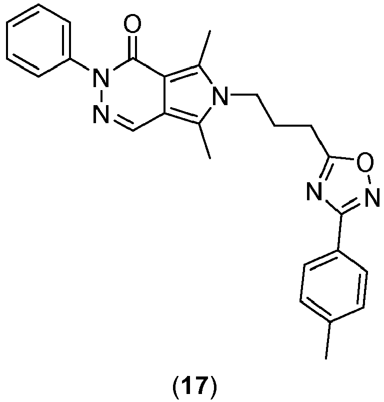

The title compound was prepared according to general procedure II (0.018 g, 41%). FT-IR (neat): *Ṽ* = 2918, 1653, 1590, 1560, 1305, 1180, 1115, 1090, 960, 797, 693 cm⁻¹. ¹H NMR (500 MHz, CDCl₃) δ 8.01 (s, 1 H), 7.95 (d, *J* = 8.2 Hz, 2H), 7.55 (d, *J* = 7.4 Hz, 2H), 7.42 (t, *J* = 7.9 Hz, 2H), 7.30 (dd, J = 7.6, 3.4 Hz, 3H), 4.18 (t, *J* = 7.8 Hz, 2H), 3.04 (t, *J* = 7.0 Hz, 2H), 2.78 (s, 3H), 2.51 (s, 3H), 2.42 (s, 3H), 2.32 (quin, J = 7.5 Hz, 2H). ¹³C NMR (126 MHz, CDCl₃) δ 177.9, 168.6, 159.3, 142.6, 141.9, 133.8, 129.9, 129.8, 128.6, 127.4, 126.9, 126.5, 123.8, 122.5, 116.0, 111.7, 43.3, 26.8, 23.8, 21.7, 11.3, 10.5. HRMS (ESI): calc. for [M+H]⁺ C₂₆H₂₆O₂N₅: 440.20810, found: 440.20793.

### Example A.8.3: Synthesis of 6-(3-(3-(4-methoxyphenyl)-1,2,4-oxadiazol-5-yl)propyl)-5,7-dimethyl-2-phenyl-2,6-dihydro-1H-pyrrolo[3,4-d]pyridazin-1-one (18)

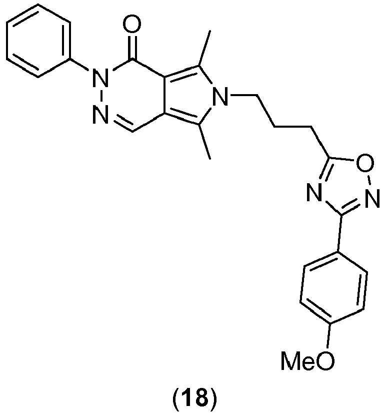

The title compound was prepared according to general procedure II (0.020 g, 44%). FT-IR (neat): *Ṽ* = 2937, 1652, 1612, 1590, 1303, 1252, 1172, 1116, 1090, 1025, 960, 901, 839, 754, 693 cm-¹. ¹H NMR (500 MHz, CDCl₃) δ 8.05 - 7.97 (m, 3H), 7.55 (d, *J* = 7.7 Hz, 2H), 7.42 (t, *J* = 7.8 Hz, 2H), 7.29 (t, *J* = 7.4 Hz, 1 H), 6.99 (d, *J* = 8.8 Hz, 2H), 4.17 (t, *J* = 7.8 Hz, 2H), 3.86 (s, 3H), 3.03 (t, *J* = 6.9 Hz, 2H), 2.78 (s, 3H), 2.51 (s, 3H), 2.31 (quin, J = 7.5 Hz, 2H). ¹³C NMR (126 MHz, CDCl₃) δ 178.1, 168.5, 162.5, 159.6, 142.8, 134.1, 130.1, 129.4, 128.8, 127.2, 126.7, 122.8, 119.3, 116.3, 114.8, 111.9, 55.8, 43.6, 27.0, 24.1, 11.5, 10.7. HRMS (ESI): calc. for [M+H]⁺ C₂₆H₂₆O₃N₅: 456.20302, found: 456.20280.

### Example A.8.4: Synthesis of 6-(3-(3-(2,5-dimethoxyphenyl)-1,2,4-oxadiazol-5-yl)propyl)-5,7-dimethyl-2-phenyl-2,6-dihydro-1H-pyrrolo[3,4-d]pyridazin-1-one (19)

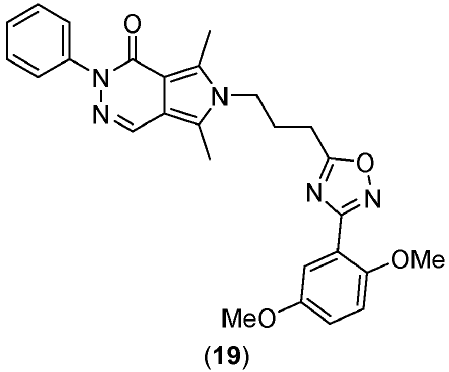

The title compound was prepared according to general procedure II (0.027 g, 56%). FT-IR (neat): *Ṽ* = 2925, 1653, 1575, 1500, 1460, 1272, 1217, 1179, 1042, 959, 810, 767, 694 cm⁻¹. ¹H NMR (400 MHz, CDCl₃) δ 8.03 (s, 1 H), 7.60 - 7.53 (m, 3H), 7.45 (t, *J* = 7.7 Hz, 2H), 7.31 (t, *J* = 7.4 Hz, 1 H), 7.10 - 6.99 (m, 2H), 4.19 (t, *J* = 7.8 Hz, 2H), 3.95 (s, 3H), 3.85 (s, 3H), 3.07 (t, *J* = 6.9 Hz, 2H), 2.79 (s, 3H), 2.52 (s, 3H), 2.32 (quin, J = 7.6 Hz, 2H). ¹³C NMR (101 MHz, CDCl₃) δ 176.9, 166.9, 159.3, 153.6, 152.5, 142.4, 133.9, 129.8, 128.6, 127.0, 126.4, 122.5, 118.4, 116.0, 115.8, 115.7, 113.3, 111.5, 56.7, 56.0, 43.3, 26.7, 23.6, 11.2, 10.5. HRMS (ESI): calc. for [M+H]⁺ C₂₇H₂₈O₄N₅: 486.21358, found: 486.21360.

### Example A.8.5: Synthesis of 5,7-dimethyl-2-phenyl-6-(3-(3-(4-(trifluoromethyl)phenyl)-1,2,4-oxadiazol-5-yl)propyl)-2,6-dihydro-1H-pyrrolo[3,4-d]pyridazin-1-one (20)

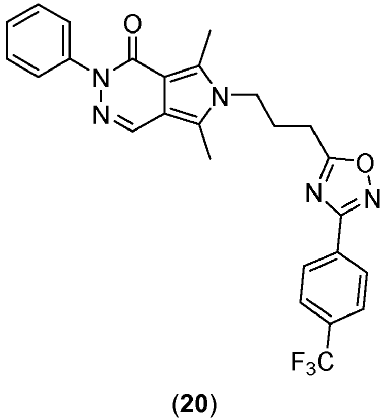

The title compound was prepared according to general procedure **II** (0.024 g, 47%). FT-IR: *Ṽ* = 1654, 1620, 1416, 1345, 1321, 1171, 1112, 1065, 853, 770, 717, 702 cm⁻¹. ¹H NMR (600 MHz, CDCl₃) δ 8.19 (d, *J* = 8.2 Hz, 2H), 8.04 (s, 1 H), 7.76 (d, *J* = 8.2 Hz, 2H), 7.51 (dd, *J* = 8.2, 1.0 Hz, 2H), 7.43 (t, *J* = 7.9 Hz, 2H), 7.31 (t, *J* = 7.4 Hz, 1 H), 4.20 (t, *J* = 7.5 Hz, 2H), 3.08 (t, *J* = 7.0 Hz, 2H), 2.78 (s, 3H), 2.53 (s, 3H), 2.35 (quin, *J* = 7.3 Hz, 2H). ¹³C NMR (151 MHz, CDCl₃) δ 178.6, 167.6, 159.4, 142.2, 134.1, 133.27 *(q, J =* 32.8 Hz), 130.0, 129.98 *(q, J =* 1.1 Hz), 128.7, 127.9, 127.3, 126.5, 126.1 (q, *J =* 3.7 Hz), 125.6 (q, *J =* 272.5 Hz), 122.7, 115.9, 111.6, 43.4, 26.7, 23.9, 11.3, 10.5.HRMS (ESI): calc. for [M+H]⁺ C₂₆H₂₃O₂N₅F₃: 494.17984, found: 494.17988.

### Example A.8.6: Synthesis of 6-(3-(3-(4-fluorophenyl)-1,2,4-oxadiazol-5-yl)propyl)-5,7-dimethyl-2-phenyl-2,6-dihydro-1H-pyrrolo[3,4-d]pyridazin-1-one (21)

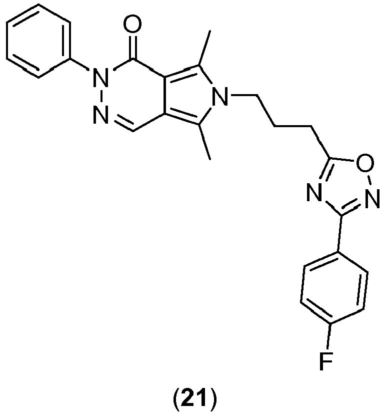

The title compound was prepared according to general procedure II (0.017 g, 39%). FT-IR (neat): *ṽ* = 2918, 1654, 1606, 1483, 1417, 1308, 1224, 1155, 1116, 1091, 846, 752, 694 cm⁻¹. ¹H NMR (500 MHz, CDCl₃) δ 8.09 - 8.04 (m, 2H), 8.01 (s, 1 H), 7.55 (d, *J* = 7.7 Hz, 2H), 7.43 (t, *J* = 7.8 Hz, 2H), 7.30 (t, *J* = 7.4 Hz, 1 H), 7.18 (t, *J* = 8.7 Hz, 2H), 4.18 (t, *J* = 7.8 Hz, 2H), 3.04 (t, *J* = 7.0 Hz, 2H), 2.78 (s, 3H), 2.51 (s, 3H), 2.33 (quin, J = 7.3 Hz, 2H). ¹³C NMR (126 MHz, CDCl₃) δ 178.3, 166.8 (d, *J =* 243.3 Hz), 163.8, 159.3, 142.5, 133.8, 129.8, 129.6 (d, *J =* 8.8 Hz), 128.6, 127.0, 126.4, 122.9 (d, *J* = 3.3 Hz), 122.5, 116.3 (d, *J* = 22.1 Hz), 111.7, 110.1, 43.3, 26.7, 23.8, 11.3, 10.5. HRMS (ESI): calc. for [M+H]⁺ C₂₅H₂₃O₂N₅F: 444.18303, found: 444.18276.

### Example A.9: Synthesis of pyrrolopyridazinone of general formula (VI) presenting a 1,3,4-oxadiazole ring.

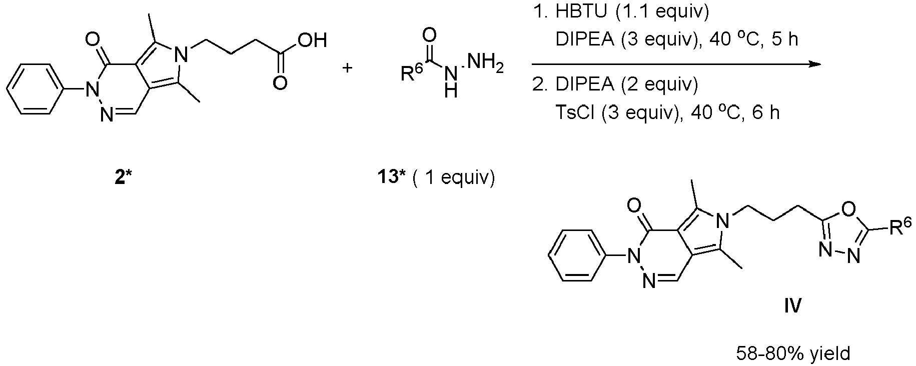

### General procedure III for the synthesis of 1,3,4-oxadiazole derivatives:

To a mixture of carboxylic acid **2*** (0.033 g, 0.1 mmol, 1.0 equiv), respective hydrazide **13*** (0.1 mmol, 1 equiv) and diisopropylethylamine (0.052 mL, 0.3 mmol, 3 equiv) in acetonitrile (1.2 mL) at room temperature was added HBTU (0.042 g, 0.11 mmol, 1.1 equiv) and the resulting mixture was heated to 40 °C for 6 h. Then the reaction mixture was allowed to come to room temperature and diisopropylethylamine (0.035 mL, 2 equiv), followed by 4-methylbenzenesulfonyl chloride (0.057 g, 3 equiv) were added. The resulting reaction mixture was stirred at 40 °C for another 6 h. After completion, the reaction mixture was diluted with dichloromethane (5 mL) and water (5 mL). The aqueous layer was extracted with dichloromethane (2 x 5 mL); the combined organic layers were sequentially washed with H₂O (5 ml x 2), saturated brine (5 mL), dried over Na₂SO₄, filtered and concentrated in *vacuo* to give crude **VI.** The resulting crude material was purified by FCC on silica gel using 3% methanol/dichloromethane as an eluent to give the desired 1,3,4-oxadiazole derivatives **VI** in 58-80% yields.

### Example A.9.1: Synthesis of 5,7-dimethyl-2-phenyl-6-(3-(5-phenyl-1,3,4-oxadiazol-2-yl)propyl)-2,6-dihydro-1H-pyrrolo[3,4-d]pyridazin-1-one (22)

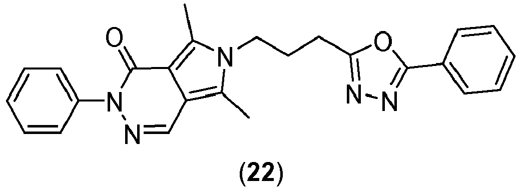

The title compound was prepared according to general procedure III (0.033 g, 78%). FT-IR (neat): *Ṽ* = 2360, 2341, 1660, 1549, 1489, 1313, 1121, 1086, 1014, 991, 842, 768, 689 cm⁻¹. ¹H NMR (600 MHz, CDCl₃) δ 8.03 - 7.98 (m, 3H), 7.56-7.52 (m, 3H), 7.52 - 7.48 (m, 2H), 7.42 (t, *J* = 7.9 Hz, 2H), 7.31 - 7.26 (m, 1 H), 4.19 (t, *J* = 7.8 Hz, 2H), 3.00 (t, *J* = 7.0 Hz, 2H), 2.75 (s, 3H), 2.49 (s, 3H), 2.31 (quin, *J* = 7.4 Hz, 2H). ¹³C NMR (151 MHz, CDCl₃) δ 165.3, 159.3, 142.4, 133.9, 132.0, 129.8, 129.2, 129.0, 127.0, 126.9 (x 2), 126.4, 123.7, 122.8, 115.8, 111.5, 43.3, 26.51, 22.7, 11.3, 10.5. HRMS (ESI): calc. for [M+H]⁺ C₂₅H₂₄O₂N₅: 426.19245, found: 426.19209.

### Example A.9.2: Synthesis of 5,7-dimethyl-2-phenyl-6-(3-(5-(p-tolyl)-1,3,4-oxadiazol-2-yl)propyl)-2,6-dihydro-1H-pyrrolo[3,4-d]pyridazin-1-one (23)

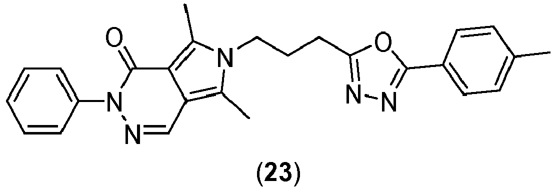

The title compound was prepared according to general procedure III (0.035, 80%). FT-IR (neat): *Ṽ* = 2260, 2341, 1657, 1560, 1496, 1450, 1389, 1313, 1120, 1085, 988, 958, 823, 758, 726, 692 cm⁻¹. ¹H NMR (500 MHz, CDCl₃) δ 7.99 (s, 1H), 7.89 (d, *J* = 8.2 Hz, 2H), 7.54 (dd, J = 8.4, 1.0 Hz, 2H), 7.41 (t, *J* = 7.9 Hz, 2H), 7.32-7.27 (m, 3H), 4.18 (t, *J* = 7.8 Hz, 2H), 2.99 (t, *J* = 7.0 Hz, 2H), 2.74 (s, 3H), 2.48 (s, 3H), 2.41 (s, 3H), 2.29 (quin, *J* = 7.5 Hz, 2H). ¹³C NMR (126 MHz, CDCl₃) δ 165.3, 164.9, 159.2, 142.5, 142.5, 133.9, 129.9, 129.8, 128.5, 126.9, 126.8, 126.4, 122.7, 120.9, 115.8, 111.5, 43.3, 26.5, 22.7, 21.7, 11.2, 10.5. HRMS (ESI): calc. for [M+H]⁺ C₂₆H₂₆O₂N₅: 440.20810, found: 440.20794.

### Example A.9.3: Synthesis of 6-(3-(5-(4-Methoxyphenyl)-1,3,4-oxadiazol-2-yl)propyl)-5,7-dimethyl-2-phenyl-2,6-dihydro-1H-pyrrolo[3,4-d]pyridazin-1-one (24)

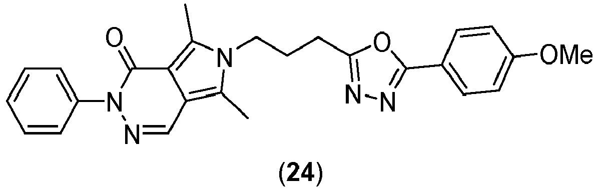

The title compound was prepared according to general procedure III (0.032, 70%). FT-IR (neat): *ṽ* = 2360, 2341, 1655, 1612, 1498, 1306, 1256, 1173, 1017, 958, 839, 761, 693 cm⁻¹. ¹H NMR (500 MHz, CDCl₃) δ 7.99 (s, 1 H), 7.94 (d, *J =* 8.8 Hz, 2H), 7.58 - 7.51 (m, 2H), 7.41 (t, *J* = 7.9 Hz, 2H), 7.28 (t, *J* = 7.4 Hz, 1 H), 6.99 (d, *J* = 8.8 Hz, 2H), 4.20 (t, *J* = 7.8 Hz, 2H), 3.86 (s, 3H), 2.98 (t, *J* = 7.0 Hz, 2H), 2.75 (s, 3H), 2.49 (s, 3H), 2.30 (quin, J = 7.5 Hz, 2H). ¹³C NMR (126 MHz, CDCl₃) δ 165.1, 164.7, 162.5, 159.2, 142.5, 133.9, 129.8, 128.6, 128.6, 126.9, 126.4, 122.7, 116.2, 115.8, 114.7, 111.6, 55.6, 43.3, 26.6, 22.7, 11.3, 10.5. HRMS (ESI): calc. for [M+H]⁺ C₂₆H₂₆O₃N₅: 456.20302, found: 456.20281.

### Example A.9.4: Synthesis of 6-(3-(5-(2-methoxyphenyl)-1,3,4-oxadiazol-2-yl)propyl)-5,7-dimethyl-2-phenyl-2,6-dihydro-1H-pyrrolo[3,4-d]pyridazin-1-one (25)

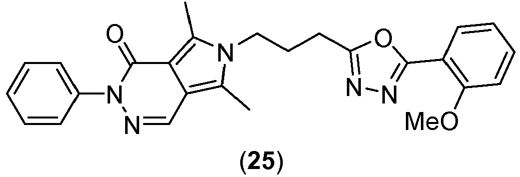

The title compound was prepared according to general procedure **III** (0.032, 70%). FT-IR (neat): *Ṽ* = 2360, 2341, 1655, 1494, 1451, 1308, 1257, 1165, 1120, 1016, 958, 758, 692 cm⁻¹. ¹H NMR (400 MHz, CDCl₃) δ 8.01 (s, 1 H), 7.89 (d, *J* = 7.5 Hz, 1 H), 7.59 - 7.47 (m, 3H), 7.42 (t, *J* = 7.8 Hz, 2H), 7.29 (t, *J* = 7.4 Hz, 1 H), 7.07 (t, *J* = 8.0 Hz, 2H), 4.20 (t, *J* = 7.6 Hz, 2H), 3.96 (s, 3H), 3.02 (t, *J* = 6.9 Hz, 2H), 2.75 (s, 3H), 2.50 (s, 3H), 2.30 (quin, J = 7.2 Hz, 2H). ¹³C NMR (101 MHz, CDCl₃) δ 164.9, 164.0, 159.3, 157.9, 142.4, 133.9, 133.3, 130.4, 129.8, 128.6, 127.0, 126.4, 122.8, 120.9, 115.7, 112.7, 112.0, 111.5, 56.1, 43.3, 26.6, 22.7, 11.2, 10.5. HRMS (ESI): calc. for [M+H]⁺ C₂₆H₂₆O₃N₅: 456.20302, found: 456.20278.

### Example A.9.5: Synthesis of 5,7-dimethyl-2-phenyl-6-(3-(5-(4-(trifluoromethyl)phenyl)-1,3,4-oxadiazol-2-yl)propyl)-2,6-dihydro-1H-pyrrolo[3,4-d]pyridazin-1-one (26)

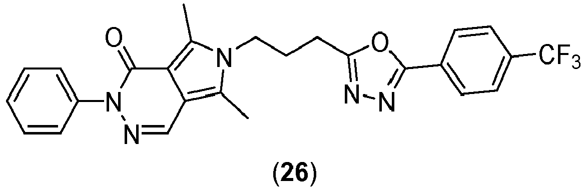

The title compound was prepared according to general procedure III (0.039, 79%). FT-IR (neat): *ṽ* = 1654, 1558, 1321, 1167, 1119, 1089, 1063, 961, 849, 759, 694 cm⁻¹. ¹H NMR (500 MHz, CDCl₃) δ 8.13 (d, *J* = 8.2 Hz, 2H), 7.99 (s, 1 H), 7.77 (d, *J* = 8.3 Hz, 2H), 7.53 (d, *J* = 7.7 Hz, 2H), 7.41 (t, *J* = 7.8 Hz, 2H), 7.29 (t, *J* = 7.4 Hz, 1 H), 4.20 (t, *J* = 7.8 Hz, 2H), 3.01 (t, *J* = 7.1 Hz, 2H), 2.75 (s, 3H), 2.49 (s, 3H), 2.32 (quin, J = 7.5 Hz, 2H). ¹³C NMR (126 MHz, CDCl₃) δ 165.9, 164.0, 159.2, 142.5, 133.8, 133.6 (q, *J* = 32.9 Hz), 129.7, 128.5, 127.2, 126.8, 126.4, 126.3 (q, *J* = 3.8 Hz), 125.8 (q, *J* = 272.6 Hz), 122.6, 115.9, 111.6, 43.2, 26.4, 22.8, 11.3, 10.5. HRMS (ESI): calc. for [M+H]⁺ C₂₆H₂₃O₂N₅F₃: 494.17984, found: 494.17985.

### Example A.9.6: Synthesis of 6-(3-(5-(4-fluorophenyl)-1,3,4-oxadiazol-2-yl)propyl)-5,7-dimethyl-2-phenyl-2,6-dihydro-1H-pyrrolo[3,4-d]pyridazin-1-one (27)

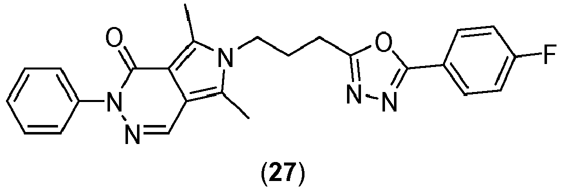

The title compound was prepared according to general procedure III (0.036, 81%). FT-IR (neat): *ṽ* = 2360, 2341, 1660, 1496, 1312, 1242, 1158, 1120, 957, 839, 759, 735, 692 cm⁻¹. ¹H NMR (400 MHz, CDCl₃) δ 8.08 - 7.98 (m, 3H), 7.56 (d, *J* = 7.6 Hz, 2H), 7.44 (t, *J* = 7.7 Hz, 2H), 7.35 - 7.27 (m, 1 H), 7.21 (t, *J* = 8.6 Hz, 2H), 4.22 (t, *J* = 7.0 Hz, 2H), 3.01 (t, *J* = 7.0 Hz, 2H), 2.77 (s, 3H), 2.52 (s, 3H), 2.33 (t, *J =* 7.0 Hz, 2H). ¹³C NMR (101 MHz, CDCl₃) δ 165.2, 164.9 (d, *J* = 253.4 Hz), 164.4, 159.2, 142.4, 133.9, 129.8, 129.2 (d, J = 9.0 Hz), 128.6, 126.9, 126.4, 122.7, 120.0 (d, *J* = 3.4 Hz), 116.6 (d, *J* = 22.4 Hz), 115.8, 111.5, 43.2, 26.5, 22.7, 11.3, 10.5. HRMS (ESI): calc. for [M+H]⁺C₂₅H₂₃O₂N₅F: 444.18303, found: 444.18283.

### Example A.9.7: Synthesis of 5,7-dimethyl-2-phenyl-6-(3-(5-(pyridin-4-yl)-1,3,4-oxadiazol-2-yl)propyl)-2,6-dihydro-1H-pyrrolo[3,4-d]pyridazin-1-one (28)

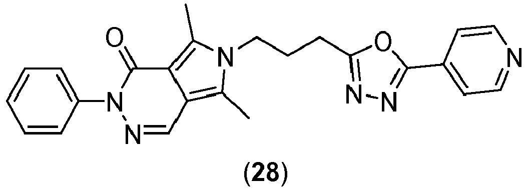

The title compound was prepared according to general procedure III (0.025, 59%). FT-IR (neat): *ṽ* = 1651, 1558, 1492, 1305, 1118, 1090, 962, 830, 768, 723, 693 cm⁻¹. ¹H NMR (400 MHz, CDCl₃) δ 8.81 (d, *J* = 5.5 Hz, 2H), 7.99 (s, 1 H), 7.87 (d, *J* = 5.8 Hz, 2H), 7.53 (d, *J* = 7.7 Hz, 2H), 7.41 (t, *J* = 7.7 Hz, 2H), 7.29 (t, *J* = 7.2 Hz, 1 H), 4.20 (t, *J* = 7.4 Hz, 2H), 3.03 (t, *J* = 7.1 Hz, 2H), 2.75 (s, 3H), 2.50 (s, 3H), 2.33 (quin, J = 7.2 Hz, 2H). ¹³C NMR (101 MHz, CDCl₃) δ 166.3, 163.4, 159.2, 150.9, 142.4, 133.9, 130.8, 129.7, 128.6, 127.0, 126.4, 122.6, 120.3, 115.8, 111.5, 43.2, 26.4, 22.8, 11.3, 10.5. HRMS (ESI): calc. for [M+H]⁺ C₂₄H₂₃O₂N₆: 427.18770, found: 427.18713.

### Example A.10: Synthesis of 6-(3-(1H-benzo[d]imidazol-2-yl)propyl)-5,7-dimethyl-2-phenyl-2,6-dihydro-1H-pyrrolo[3,4-d]pyridazin-1-one (S1)

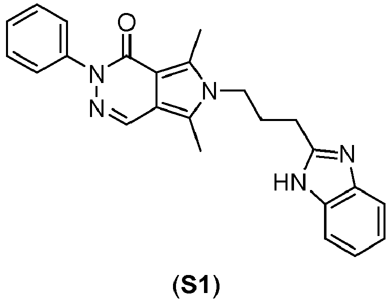

The acid derivative **2*** (0.195 g, 0.6 mmol, 1 equiv) and *o*-phenylenediamine (0.065 g, 0.6 mmol, 1 equiv) in polyphosphoric acid (1.2 g) were heated at 175 °C for 24 h. The reaction mixture was then allowed to come to room temperature and poured on ice. The resulting suspension was brought to pH ≈ 8 using ammonium hydroxide. The resulting light brown solid was filtered, washed with water, dried under vaccum to yield the desired benzimidazole derivative S1 (0.20 g, 84%) as a brown solid, which was used in the next step without further purification. FT-IR (neat): *ṽ* = 2915, 1640, 1557, 1453, 1307, 1270, 1089, 960, 725, 693 cm⁻¹. ¹H NMR (500 MHz, DMSO) δ 8.23 (s, 1 H), 7.52 - 7.46 (m, 4H), 7.42 (t, *J* = 7.9 Hz, 2H), 7.29 (t, *J* = 7.3 Hz, 1 H), 7.15 - 7.10 (m, 2H), 4.20 (t, *J* = 7.8 Hz, 2H), 2.91 (t, *J* = 7.3 Hz, 2H), 2.65 (s, 3H), 2.49 (s, 3H), 2.20 (quin, J = 7.5 Hz, 2H). ¹³C NMR (126 MHz, DMSO) δ 158.2, 153.8, 142.5, 141.7, 134.3, 129.3, 128.3, 128.0, 126.2, 126.2, 126.1, 123.71, 121.2, 114.6, 109.9, 43.2, 27.4, 25.4, 10.7, 9.7. HRMS (ESI): calc. for [M+H]⁺ C₂₄H₂₄ON₅: 398.19754, found: 398.19571.

### Example A.11: Synthesis of 6-(3-(1-Benzyl-1 H-benzo[d]imidazol-2-yl)propyl)-5,7-dimethyl-2-phenyl-2,6-dihydro-1H-pyrrolo[3,4-d]pyridazin-1-one (15)

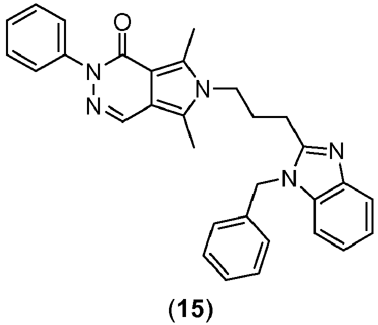

To a suspension of benzimidazole derivative S1 (0.016 g, 0.04 mmol, 1 equiv) in acetonitrile (0.5 mL) was added Cs₂CO₃ (0.020 g, 0.06 mmol, 1.5 equiv) and benzyl bromide (5.0 µL, 0.044 mmol, 1.1 equiv). The reaction mixture was stirred at room temperature for 12 h. After completion, the reaction mixture was concentrated *in vacuo* and the residue was re-dissolved in CH₂Cl₂ (5 mL) and sat. NaHCO₃ (5 mL) was added. The aqueous layer was extracted with CH₂Cl₂ (2 x 5 mL). The combined organic layers were dried over Na₂SO₄, filtered and the solvent was removed under reduced pressure. The crude product was purified by flash chromatography using 4% MeOH/CH₂Cl₂ as an eluent to give the desired product 15 (0.014 g, 72%) as a brown foam like solid. FT-IR (neat): *ṽ* = 2923, 1650, 1494, 1453, 1306, 1117, 1089, 960, 725, 693 cm⁻¹. ¹H NMR (500 MHz, CDCl₃) δ 7.98 (s, 1 H), 7.82 - 7.77 (m, 1 H), 7.57 (d, *J* = 7.5 Hz, 2H), 7.43 (t, *J* = 7.9 Hz, 2H), 7.34 - 7.27 (m, 7H), 7.04 - 6.97 (m, 2H), 5.29 (s, 2H), 4.14 (t, *J* = 7.5 Hz, 2H), 2.82 (t, *J* = 7.2 Hz, 2H), 2.64 (s, 3H), 2.38 (s, 3H), 2.30 (t, *J* = 7.3 Hz, 2H). ¹³C NMR (126 MHz, CDCl₃) δ 159.4, 153.1, 142.6, 135.7, 135.6, 134.0, 129.9, 129.3, 128.6, 128.4, 126.9, 126.5, 126.3, 123.2, 122.8, 122.7, 119.4, 115.7, 111.5, 109.7, 109.6, 47.1, 43.6, 27.3, 24.2, 11.3, 10.4. HRMS (ESI): calc. for [M+H]⁺ C₃₁H₃₀ON₅: 488.24449, found: 488.24406.

### Example A.12: Ethyl 2-chloro-2-(2-(p-tolyl)hydrazono)acetate (16b*)

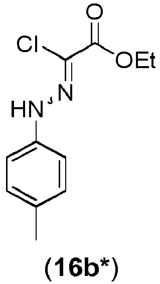

A solution of aniline derivative (20 mmol) in dilute HCl (1:1, 14 mL) was cooled to 0 °C, and a cold solution of NaNO₂ (1.1 equiv.) in H₂O (19 mL) was added drop wise over 15 min while maintaining internal solution temperature below 5 °C. After addition, the reaction mixture was stirred for another 10 min keeping the internal temperature below 0 °C. The resulting ice-cold solution of diazonium derivative was then added drop wise *via* cannula to a precooled (0 °C) solution of ethyl-2-chloroacetoacetate (1.0 equiv.) and NaOAc (1.5 equiv.) in H₂O/EtOH (1:7, 80 mL). After complete addition, the reaction mixture was stirred for 4 h at the same temperature and then was quenched by addition of 200 mL of cold water. The resultant precipitate was filtered and dried under vacuum to furnish the desired product **16b*** (3.50 g, 72%) as an off-white solid. ¹H NMR (400 MHz, CDCl₃) δ 8.34 (s, 1 H), 7.13 (s, 4H), 4.38 (q, *J =* 7.1 Hz, 2H), 2.31 (s, 3H), 1.40 (t, *J =* 7.1 Hz, 3H). ¹³C NMR (101 MHz, CDCl₃) δ 159.9, 139.4, 132.8, 130.0, 115.4, 114.2, 62.8, 20.8, 14.4. HRMS: calc. for [M+H]⁺C₁₁H₁₄O₂N₂Cl: 241.07383, found: 241.07342.

### Example A.13: 3,4-Dimethyl-2-(p-tolyl)-2H-pyrazolo[3,4-d]pyridazin-7(6H)-one (17b*)

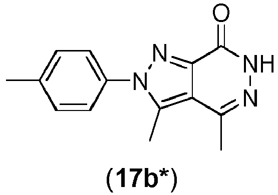

2,4-Pentanedione (1.34 mL, 13.0 mmol, 1 equiv) was added drop wise to a solution of NaOEt (21% wt in EtOH, 4.85 mL, 13 mmol, 1.0 equiv.) in anhydrous MeOH (10 mL) at ambient temperature and the reaction mixture was stirred for 4 h. Then the corresponding solid hydrazonyl chloride **16b*** (3.13 g, 13 mmol, 1.0 equiv) was added in portions and the reaction was left to stir for 16 h. After completion, the volatile components were removed and the crude material was re-dissolved in dichloromethane (30 mL). The organic layer was then sequentially washed with H₂O (10 ml x 2), saturated brine (15 mL), dried over Na₂SO₄, filtered and concentrated in *vacuo* to give crude **15b* (15b*** was isolated as a mixture of ethyl and methyl ester, which was confirmed by GC-MS. Transesterification was observed as MeOH was used as solvent. Reaction works equally well in EtOH. So trans-esterification can be avoided by using NaOEt as a base in combination with anhydrous EtOH as solvent). Crude **15b*** was subjected to the next step without further purification.

**15b*** (3.75 g, 13.0 mmol) and hydrazine monohydrate (1.91 mL, 39.3 mmol, 3.0 equiv.) were dissolved in EtOH (20 mL) and the mixture was heated in a sealed tube at 110°C for 6 h. After completion, the reaction mixture was allowed to come to room temperature and then was cooled in an ice-bath. The resulting precipitation was filtered, washed with water and dried under vacuum to yield the desired product **17b*** (2.4 g, 9.44 mmol, 72% over two step) as grey solid. ¹H NMR (500 MHz, DMSO) δ 11.98 (s, 1 H), 7.49 (d, *J* = 8.2 Hz, 2H), 7.43 (d, *J* = 8.2 Hz, 2H), 2.59 (s, 3H), 2.49 (s, 3H), 2.43 (s, 3H). ¹³C NMR (126 MHz, DMSO) δ 156.4, 141.2, 141.0, 139.2, 137.1, 135.9, 129.8, 125.7, 117.4, 20.7, 19.2, 11.8. HRMS: calc. for [M+H]⁺ C₁₄H₁₅ON₄: 255.12404, found: 255.12345.

### Example A.14: Methyl 4-(3,4-dimethyl-7-oxo-2-(p-tolyl)-2H-pyrazolo[3,4-d]pyridazin-6(7H)-yl)butanoate (18b*)

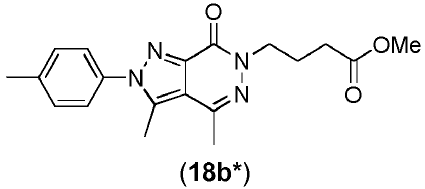

NaH (60 % in mineral oil, 0.236 g, 5.89 mmol, 1.50 equiv.) was added in small portions to a solution of **17b*** (1.0 g, 3.93 mmol, 1.0 equiv) in DMF (3 mL) at 0 °C and the reaction mixture was stirred for an hour at this temperature. Then a solution of methyl 4-bromobutanoate (0.99 mL, 7.86 mmol, 2.0 equiv) in DMF (0.5 mL) was added drop wise and the resulting reaction mixture was stirred for an additional 4 h at room temperature. After completion, the reaction was quenched by adding H₂O (20 mL) and diluted with EtOAc (20 mL). The aqueous layer was extracted with EtOAc (2 x 10 mL); combined organic layers were sequentially washed with H₂O (2 x 10 mL), brine (2 x 10 mL), dried over Na₂SO₄, filtered, and concentrated under vacuum. The crude residue was purified by flash column chromatography using 4% MeOH/CH₂Cl₂ as an eluent to give the desired product **18b*** (1.2 g, 3.39 mmol, 86%) as a white solid. ¹H NMR (400 MHz, CDCl₃) δ 7.34 (d, *J* = 8.4 Hz, 2H), 7.30 (d, *J* = 8.4 Hz, 2H), 4.22 (t, *J* = 6.9 Hz, 2H), 3.65 (s, 3H), 2.61 (s, 3H), 2.53 (s, 3H), 2.43 (s, 3H), 2.40 (t, *J* = 8.0 Hz , 2H), 2.15 (quin, J = 7.1 Hz, 2H). ¹³C NMR (101 MHz, CDCl₃) δ 173.6, 156.2, 142.0, 140.9, 139.7, 136.3, 136.0, 129.9, 125.9, 125.8, 117.7, 51.6, 48.8, 31.3, 24.1, 21.3, 19.9, 12.3. HRMS: calc. for [M+H]⁺ C₁₉H₂₃O₃N₄: 355.17647, found: 355.17677.

### Example A.15: 4-(3,4-dimethyl-7-oxo-2-(p-tolyl)-2H-pyrazolo[3,4-d]pyridazin-6(7H)-yl)butanoic acid (14b*)

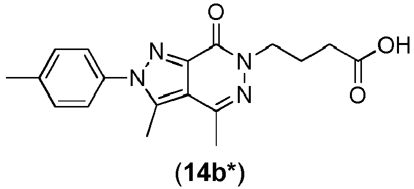

A heterogeneous mixture of pyrazolopyridazone derivative **18b*** (1.1 g, 3.10 mmol, 1.0 equiv) and KOH (0.21 g, 3.72 mmol, 1.2 equiv) in H₂O (15 mL) was heated to reflux until the entire solid had dissolved (30 min). Then the reaction mixture was allowed to come to room temperature, cooled to 0°C and was acidified with 10% aqueous HCl solution to a pH of 5-6. The resultant precipitation was then filtered, dried under vacuum to yield analytically pure **14b*** as a white solid (1.03 g, 3.03 mmol, 98%). ¹H NMR (400 MHz, DMSO) δ 12.09 (s, 1 H), 7.47 (d, *J =* 8.2 Hz, 2H), 7.41 (d, *J* = 8.2 Hz, 2H), 4.06 (t, *J* = 6.9 Hz, 2H), 2.58 (s, 3H), 2.50 (s, 3H), 2.41 (s, 3H), 2.25 (t, *J* = 7.3 Hz, 2H), 1.92 (quin, J = 7.1 Hz, 2H). ¹³C NMR (101 MHz, DMSO) δ 174.0, 155.2, 141.1, 141.1, 139.3, 137.4, 135.9, 129.9, 125.7, 117.1, 48.2, 30.8, 23.8, 20.8, 19.4, 11.9. HRMS: calc. for [M+H]⁺ C₁₈H₂₁O₃N₄: 341.16082, found: 341.16096.

### Example A.16: General procedure IV for amide coupling

To a solution of acid derivative **14b*** (0.049 g, 0.15 mmol, 1.0 equiv) and DMAP (0.024 g, 0.195 mmol, 1.3 equiv) in THF/DCM (1:1, 0.6 mL and 0.6 mL) was added EDC·HCl (0.037 g, 0.195 mmol, 1.3 equiv), followed by the corresponding amine derivative (0.158 mmol, 1.05 equiv) and the resulting solution was stirred for 16 h at ambient temperature. Once completed, as observed by TLC, the reaction mixture was diluted with CH₂Cl₂ (5 mL) and quenched by adding saturated NaHCO₃ solution (5 mL). The aqueous layer was extracted with dichloromethane (2 x 5 mL) and combined organic layers were sequentially washed with saturated NH₄Cl solution (5 mL x 2), H₂O (5 ml x 2) and brine (5 mL); dried over Na₂SO₄, filtered and concentrated under vacuum. The crude residue was further purified by FCC on silica-gel using 3.5% MeOH/CH₂Cl₂ as an eluent to afford desired amide compounds.

### Example A.16.1: 4-(3,4-dimethyl-7-oxo-2-(p-tolyl)-2H-pyrazolo[3,4-d]pyridazin-6(7H)-yl)-N-(2-phenylpropyl)butanamide (50)

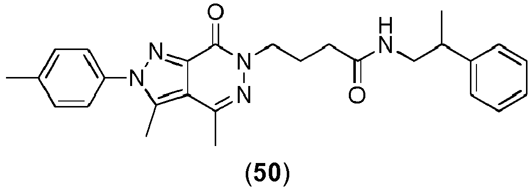

The title compound (1.20 g, 2.62 mmol, 94%) was prepared according to general procedure IV by treating acid derivative **14b*** (0.95 g, 2.79 mmol) with β-methylphenethylamine (0.426 mL, 2.93 mmol), EDC·HCl (0.696 g, 3.63 mmol) and DMAP (0.44 g, 3.628 mmol) in a DCM/THF (9/9 mL) solvent mixture. ¹H NMR (400 MHz, CDCl₃) δ 7.40 - 7.31 (m, 4H), 7.30 - 7.24 (m, 4H), 7.23 - 7.16 (m, 1 H), 4.11 - 3.97 (m, 2H), 3.58 - 3.49 (m, 1 H), 3.48 - 3.39 (m, 1 H), 3.09 (sex, J = 7.1 Hz, 1 H), 2.64 (s, 3H), 2.54 (s, 3H), 2.46 (s, 3H), 2.17 (t, *J* = 6.5 Hz, 2H), 2.12 - 2.02 (m, 2H), 1.31 (d, *J* = 7.0 Hz, 3H). ¹³C NMR (101 MHz, CDCl₃) δ 173.2, 157.3, 144.9, 142.2, 142.1, 140.3, 136.8, 136.3, 130.3, 128.9, 127.7, 126.8, 126.2, 118.1, 48.8, 46.7, 40.0, 33.6, 26.1, 21.7, 20.3, 20.1, 12.7. HRMS: calc. for [M+H]⁺ C₂₇H₃₂O₂N₅: 458.25505, found: 458.25514.

### B. Crystallography

### Example B.1. Crystallization of compound 22 (see Figure 1)

Compound **22** was co-crystallized with PDEδ by mixing a solution of 500 µM of compound **22** with an equimolar solution of PDEδ resulting in 1 % DMSO as a final concentration.

Crystals were obtained from a Qiagen PEGs suite (0.2 M Calcium chloride, 20% (w/v) PEG 3350). For flash freezing the crystals in liquid nitrogen, we used cryoprotectant solution containing the mother liquor components in addition to glycerol. X-rays data were collected at the X10SA beamline of the Suisse Light Source, Villigen. XDS program was used for processing the data. For solving the structures, molecular replacement was applied using the program MoIRep from the CCP4 software and PDEδ without bound farnesyl group, from the complex PDEδ-farnesylated Rheb complex (PDB 3T5G) as a search model. Further refinement of the model was performed using a combination of manual refinement (COOT program) and the maximum likelihood restrained refinement (REFMAC5 program). Final validation of the model using Ramachandran plot statistics showed none of the residues to be outliers (see data collection and refinement statistics in **Table 1).**

**Table 1. Data collection and refinement statistics.**

| | **22** |
|---|---|
| **Data collection** | |
| Space group | P 3₂ 21 |
| Cell dimensions | |
| *a, b, c* (Å) | 55.71,55.71, 114.67 |
| A, β, γ (°) | 90, 90, 120 |
| Resolution (Å) | 2.10 |
| Rsym or *R*_{merge} | 6.4 (46.4) |
| / / σ/ | 20.17 (4.39) |
| Completeness (%) | 99.9(100) |
| Redundancy | 7.91 (8.23) |
| | |
| **Refinement** | |
| Resolution (Å) | 2.10 |
| No. reflections | 11932 |
| *R*_{work} / *R*_{free} | 19.53 / 24.80 |
| No. atoms | |
| Protein | 1237 |
| Ligand/ion | 32 |
| Water | 23 |
| *B*-factors | |
| Protein | 39.3 |
| Ligand/ion | 40.7 |
| Water | 39.8 |
| | |
| R.m.s. deviations | |
| Bond lengths (Å) | 0.020 |
| Bond angles (°) | 1.945 |

| | |
|---|---|
| Values in parentheses correspond to the high resolution shell | |

### Example B.2: Crystallization of compound 29 (see Figure 3)

Compound **29** was co-crystallized with PDEδ by mixing a solution of 500 µM of compound **29** with an equimolar solution of PDEδ resulting in 1 % DMSO as a final concentration.

Crystals were obtained from a Qiagen PEGs suite (0.2 M Calcium acetate, 20% (w/v) PEG 3350). For flash freezing the crystals in liquid nitrogen, we used cryoprotectant solution containing the mother liquor components in addition to glycerol. X-rays data were collected at the X10SA beamline of the Suisse Light Source, Villigen. XDS program was used for processing the data. For solving the structures, molecular replacement was applied using the program MoIRep from the CCP4 software and PDEδ without bound farnesyl group, from the complex PDEδ-farnesylated Rheb complex (PDB 3T5G) as a search model. Further refinement of the model was performed using a combination of manual refinement (COOT program) and the maximum likelihood restrained refinement (REFMAC5 program). Final validation of the model using Ramachandran plot statistics showed none of the residues to be outliers (see data collection and refinement statistic in **Table 2).**

**Table 2. Data collection and refinement statistics.**

| | **29** |
|---|---|
| **Data collection** | |
| Space group | P 1 |
| Cell dimensions | |
| *a, b, c* (Å) | 31.76, 40.90, 68.80 |
| A, β, γ (°) | 97.70, 102.38, 89.31 |
| Resolution (Å) | 2.60 |
| Rsym or Rmerge | 11.3 (46.6) |
| // σ/ | 9.45 (3.26) |
| Completeness (%) | 97.7 (96.7) |
| Redundancy | 3.45 (3.40) |
| | |
| **Refinement** | |
| Resolution (Å) | 2.60 |
| No. reflections | 9570 |
| *R*_{work} / *R*_{free} | 18.13 / 25.57 |
| No. atoms | |
| Protein | 2432 |
| Ligand/ion | 64 |
| Water | 16 |
| *B*-factors | |
| Protein | 37.7 |
| Ligand/ion | 40.2 |
| Water | 37.3 |
| R.m.s. deviations | |
| Bond lengths (Å) | 0.013 |
| Bond angles (°) | 1.732 |

| | |
|---|---|
| Values in parentheses correspond to the high resolution shell | |

### C. Biochemical experiments

### Example C.1: Alpha-Screen (Nature 2013, 497, 638)

Screening based on Alpha-technology was conducted in white, non-binding 1536-well plates (Corning) in a final volume of 6 µL. For the screen a mixture of His₆-PDEδ, and biotinylated K-Ras-peptide (final concentrations 100 nM and 250 nM in HEPES 20 mM, 100 mM NaCl, 0,005% Chaps, pH 7.5) were added to the 1536-well plates. Compound solutions were directly added from 10 mM DMSO stock solutions to a final concentration of 10 µM and the resulting mixture was incubated for 30 min (For dose-response curves, compounds were tested at concentrations betwen 10 µM and 5 nM). Premixed Nickel Chelate Acceptor Beads and Streptavidin Donor Beads were added to a final concentration of 10 µg/mL. The resulting mixture was incubated at 4 °C overnight. Plates were read on a Paradigm reader (Molecular devices, Alphascreen 1536 HTS detection cartridge, temperature 29°C-33°C).

### Example C.2: Displacement titrations of labeled Atorvastatin-probe for the determination of K_{D} values:

Binding to PDEδ was validated and quantified by means of a displacement assay employing a fluorescent-tagged analog of the HMG-CoA reductase inhibitor Atorvastatin (Lipitor®) which has previously been shown to also bind to PDEδ. *(*Nat. Chem. Biol. 2011, 7, 375-383) The *K*_{d} values were determined by the Fluorescence polarization competition binding assay previously developed by the inventors *(*Nature 2013, 497, 638).

**Table 3. K_{D} values for compounds of general formula (I) as determined by means of fluorescence polarization assay**

| **Compound** | *K*_{D} **[nM]** | **Compound** | **K_{D} [nM]** |
|---|---|---|---|
| **1** | +++ | **29** | ++++ |
| **2** | +++ | **30** | +++ |
| **3** | +++ | **31** | +++ |
| **4** | ++++ | **32** | +++ |
| **5** | +++ | **33** | +++ |
| **6** | ++ | **34** | +++ |
| **7** | ++ | **35** | +++ |
| **8** | ++ | **36** | ++++ |
| **9** | ++ | **37** | ++++ |
| **10** | +++ | **38** | +++ |
| **11** | ++++ | **39** | +++ |
| **12** | ++++ | **40** | ++ |
| **13** | ++++ | **41** | ++ |
| **14** | ++++ | **42** | ++++ |
| **15** | ++++ | **43** | ++++ |
| **16** | ++ | **44** | ++++ |
| **17** | + | **45** | ++++ |
| **18** | + | **46** | ++++ |
| **19** | + | **47** | ++++ |
| **20** | + | **48** | ++++ |
| **21** | ++ | **49** | ++++ |
| **22** | ++ | **50** | ++++ |
| **23** | + | **51** | ++ |
| **24** | + | **52** | ++++ |
| **25** | ++ | **53** | ++++ |
| **26** | ++ | | |
| **27** | ++ | | |
| **28** | + | | |
| | K_{D} ≤ 30 nM | | ++++ |
| | 30 nM < K_{D} ≤ 100 nM | | +++ |
| | 100 nM < K_{D} ≤ 1000 nM | | ++ |
| | K_{D} > 1000 nM | | + |

### Example C.3: Inhibitory effect of compound 50 at different concentrations on interaction of Rheb (Ras homolog enriched in brain) with PDEδ and localization in live MDCK cells (See Figure 4).

To address whether compound **50** inhibits the interaction between PDEδ and Ras family proteins in live cells, fluorescence lifetime imaging microscopy (FLIM)-based quantitative fluorescence resonance energy transfer (FRET) measurements were carried out. These experiments allow the quantitative determination of the fraction (α) of interacting mCitrine (a yellow fluorescent protein) tagged Ras proteins with mCherry (a red fluorescent protein) tagged PDEδ in living cells by the change in the fluorescence lifetime (τₐᵥ) of the mCitrine fused to Ras. mCitrine-Rheb was selected as the farnesylated Ras family protein due to its more clearly measurable interaction with mCherry-PDEδ in live cells.

Hence, MDCK cells were co-transfected with mCherry-PDEδ and mCitrine-Rheb. The fluorescence patterns of both mCitrine-Rheb (first row) and mCherry-PDEδ (second row) were homogeneous in untreated cells (Pre) showing a clear solubilization of mCitrine-Rheb by mCherry-PDEδ. Under these conditions a substantial drop in the mCitrine fluorescence lifetime (third row) corresponds to a substantial molar fraction (α) of mCitrine-Rheb (fourth row) that was in complex with mCherry-PDEδ. Compound 50 was administered to the final indicated concentrations and 5 min after the administration, fluorescence lifetime images were acquired by using a confocal laser-scanning microscope (FV1000, Olympus) equipped with a time-correlated single-photon counting module (LSM Upgrade Kit, Picoquant). Intensity thresholds were applied to segment the cells from the background fluorescence. Data were further analyzed to obtain images of the molar fraction (α) of interacting mCherry-PDEδ/mCitrine-Rheb. Thus, it can be observed that the interaction mCherry-PDEδ/mCitrine-Rheb is lost upon incubation with different concentrations of compound 50 leading to an increased average fluorescence lifetime of mCitrine and reduced computed fraction (α) of interacting PDEδ-Rheb with the increase in the concentration of compound 50.

### Example C.4: Study of the interaction K-Ras-PDEδ and inhibition of said interaction by the compounds of the present invention (see Figure 5)

MDCK cells were co-transfected with mCherry-PDEδ and mCitrine-K-Ras. Fluorescence lifetime images were acquired using a confocal laser-scanning microscope (FV1000, Olympus) equipped with a time-correlated single-photon counting module (LSM Upgrade Kit, Picoquant). 5 min after the administration of Bryostatin, fluorescence lifetime images were acquired and 5 µM of Compound 50 was administered. After 10 minutes in total, another fluorescence lifetime image was taken. Intensity thresholds were applied to segment the cells from the background fluorescence. Data were further analyzed to obtain images of the molar fraction (α) of interacting mCherry-PDEδ/mCitrine-Rheb.

### Example C.5: Inhibition of the K-Ras dependent cell proliferation by the inventive compounds (See Figure 6)

Real time cell analysis (RTCA) measurements were performed in order to determine the dose-dependent effect of the compounds of the present invention on the proliferation of K-Ras independent cells (PANC-1 (see **Figures 6a****, 6d****),** BxPC-3 (see **Figures 6e****))** and K-Ras dependent cells (Panc-Tu-I (see **Figures 6b****, 6f****),** Capan-1 (see **Figures 6c****, 6g****)).** RTCA measurements were performed using 16-well E-plates on the Dual Plate xCELLigence instrument (Roche Applied Science, Indianapolis IN). E-Plates were blanked with 100 µl of DMEM medium containing 10% FCS. After blanking, trypsinized cells were counted with a Beckman Coulter Vi-cell™ counter and 7.500 to 15.000 cells (cell line dependent) were plated in each well of the 16-well E-plates (ACEA) in a final volume of 200 µl. After seeding, cells were allowed to settle for 5 to 10 minutes at room temperature before being reinserted into the xCELLigence instrument. The RTCA is localized in a humidified incubator at 37°C with 10% CO₂. Continuous impedance measurements were monitored every 15 min up to 100 hours. 24 hours after seeding, compound **50** was administered. The derivatives were calculated for the time frame indicated by the black bar.

As shown by Figures **6b****,** **6f****,** **6c** and **6g****,** addition of compound **50** inhibits the proliferation of K-Ras dependent cells Panc-Tu-I and BxPC-3, leading after a certain time to cell death (see **Figure 6f****).**

## Claims

1. A compound of the general (I) 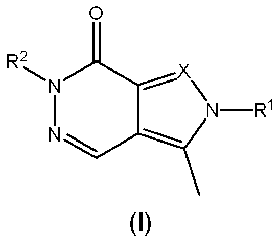 wherein
X represents N or C(CH₃);
R¹ represents R³ and R² represents -CH₂CH₂CH₂R⁴, or R¹ represents -CH₂CH₂CH₂R⁴ and R² represents R³;
R³ represents 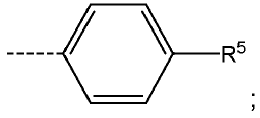 R⁵ is selected from -H, -CH₃, -C₂H₅, -CH₂CH₂CH₃, and -CH(CH₃)₂;
R⁴ represents: -C(O)-NH-R⁶, 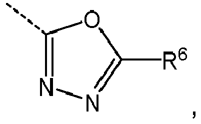 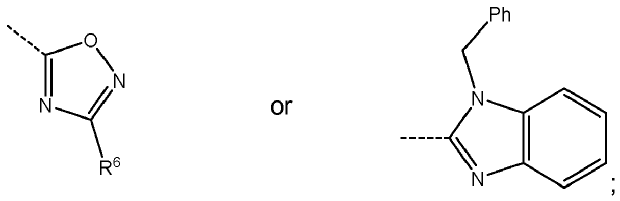 R⁶ represents -(CR⁸R⁹)ₙ₁-(CH₂)ₙ₂-R⁷; R⁷ represents: 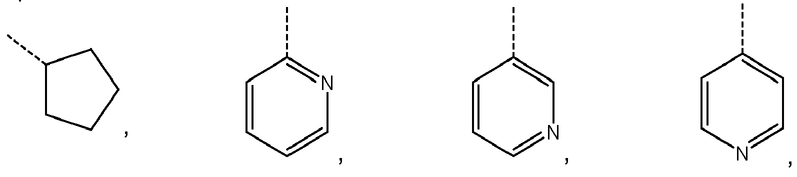 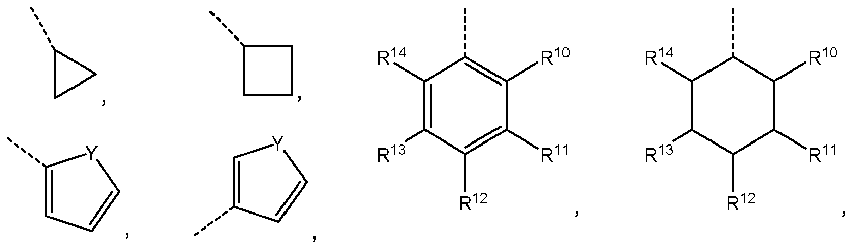 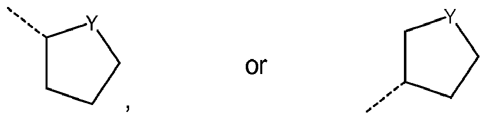 -Y- is selected from -O- and -S-;
R⁸, R⁹ are independently of each other selected from -H, -CH₃ and -C₂H₅; R¹⁰, R¹¹, R¹², R¹³ and R¹⁴ are independently of each other selected from: -H, -Cl, -F, -CH₃, -CH₂CH₃, -CH₂CH₂CH₃, -CH(CH₃)₂, -OCH₃, -OCH₂CH₃, -OCH₂CH₂CH₃, -OCH(CH₃)₂ and -CF₃;
n1 and n2 are independently of each other selected from 0 or 1;
and enantiomers, mixtures of enantiomers, diastereomers, mixtures of diastereomers, hydrates, solvates, tautomers, racemates and pharmaceutically acceptable salts thereof.

2. The compound according to claim 1 of general formula (**II**) 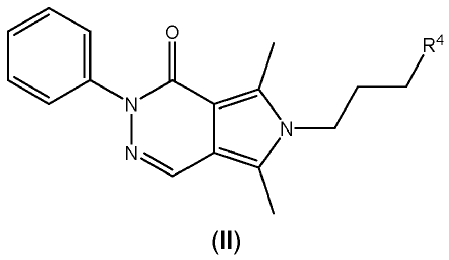 wherein
R⁴ represents: -C(O)-NH-R⁶, 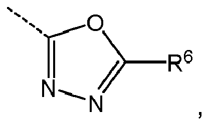 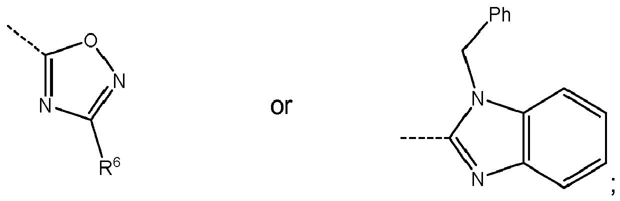 R⁶ represents -R⁷ or -CH₂-R⁷; R⁷ represents: 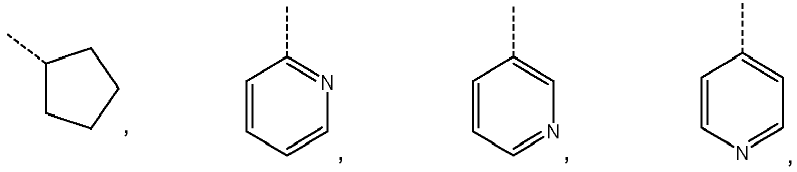 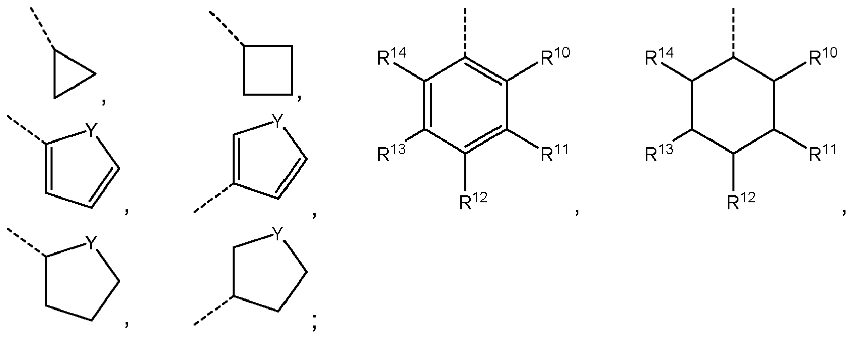 -Y- is selected from -O- and -S-, and
R¹⁰, R¹¹, R¹² , R¹³ and R¹⁴ are independently of each other selected from: -H, -Cl, -F, -CH₃, -CH₂CH₃, -CH₂CH₂CH₃, -CH(CH₃)₂, -OCH₃, -OCH₂CH₃, -OCH₂CH₂CH₃, -OCH(CH₃)₂ and -CF₃.

3. The compound according to claim 1 or 2 of general formula (**III**) 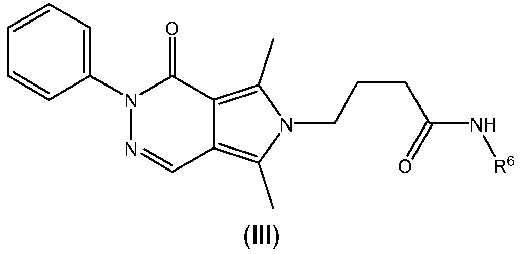 wherein R⁶ represents -R⁷ or -CH₂-R⁷; R⁷ is selected from: 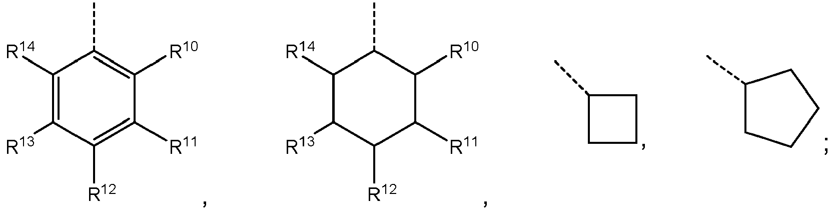 and
R¹⁰, R¹¹, R¹² , R¹³ and R¹⁴ are independently of each other selected from: -H, -Cl, -F, -CH₃, -CH₂CH₃, -CH₂CH₂CH₃, -CH(CH₃)₂, , -OCH₃, -OCH₂CH₃, -OCH₂CH₂CH₃, -OCH(CH₃)₂ and -CF₃.

4. The compound according to claim 2 or 3, wherein R⁶ is selected from: 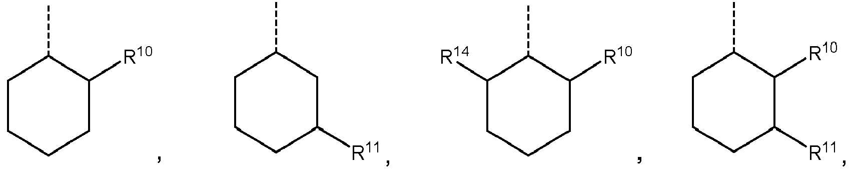 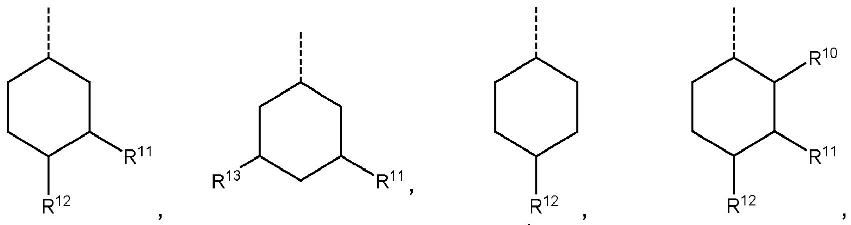 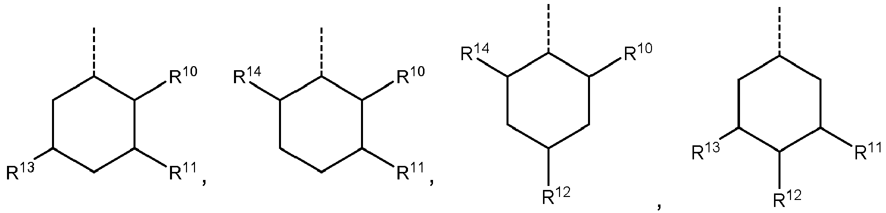 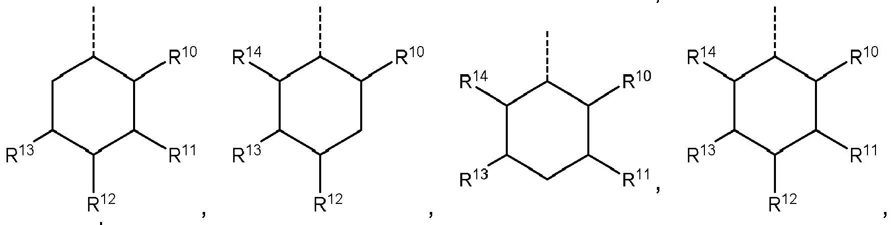 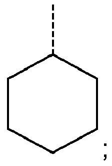 and R¹⁰, R¹¹, R¹², R¹³ and R¹⁴ are independently of each other selected from: -CH₃, -CH₂CH₃, -CH₂CH₂CH₃, -CH(CH₃)₂ and -CF₃.

5. The compound according to claim 2 or 3, wherein R⁶ represents: 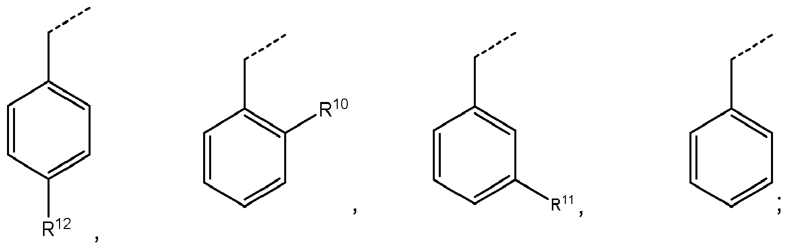 and R¹⁰, R¹¹ and R¹² represent independently of each other -Cl or -F.

6. The compound according to claim 2, wherein
R⁴ represents: 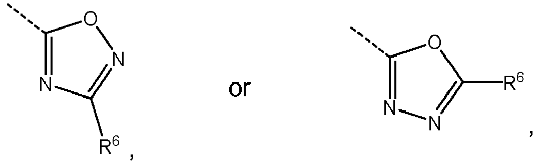 R⁶ is selected from: 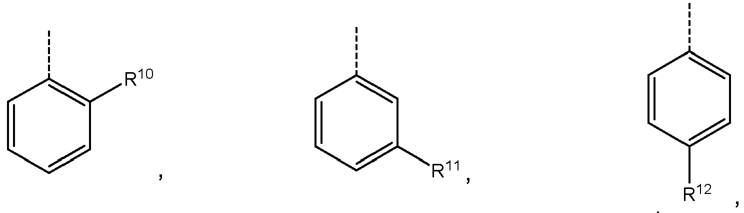 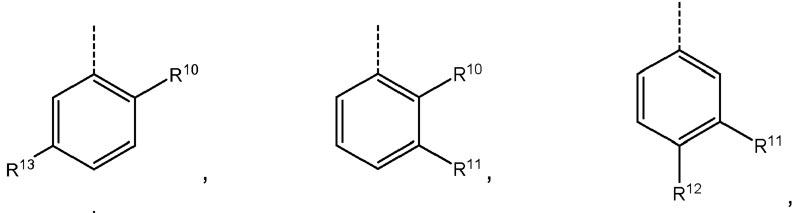 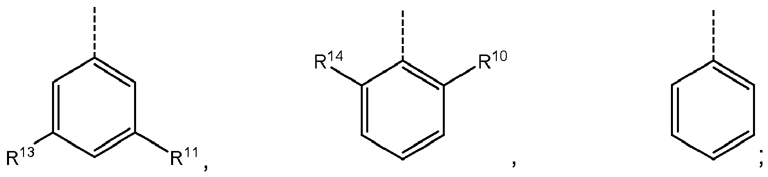 and R¹⁰, R¹¹, R¹², R¹³ and R¹⁴ are independently of each other selected from: -CH₃, -OCH₃, -CF₃, and -F.

7. The compound according to claim 1 of general formula (IV) 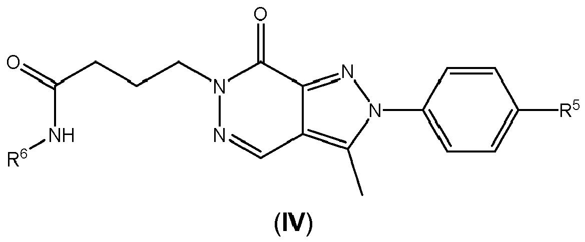 wherein
R⁵ is selected from -H and -CH₃;
R⁶ represents -(CR⁸R⁹)ₙ₁-(CH₂)ₙ₂-R⁷;
R⁷ represents: 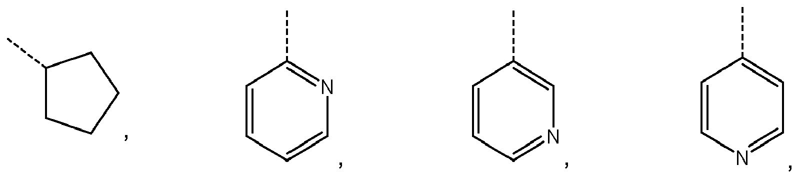 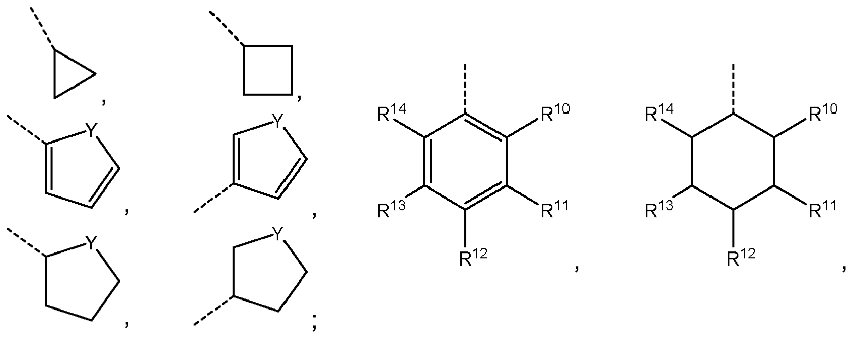 -Y- is selected from -O- and -S-;
R⁸, R⁹ are independently of each other selected from -H, -CH₃ and -C₂H₅; R¹⁰, R¹¹, R¹² , R¹³ and R¹⁴ are independently of each other selected from: -H, -Cl, -F, -CH₃, -CH₂CH₃, -CH₂CH₂CH₃, -CH(CH₃)₂, , -OCH₃, -OCH₂CH₃, -OCH₂CH₂CH₃, -OCH(CH₃)₂, -CF₃;
and n1 and n2 are independently of each other selected from 0 or 1.

8. The compound according to claim 7, wherein R⁵ represents -CH₃ and
R⁶ is selected from

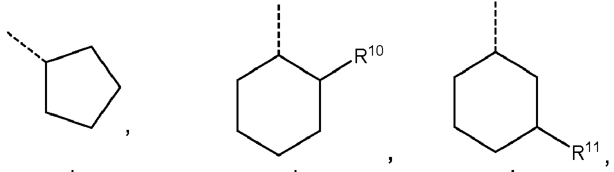

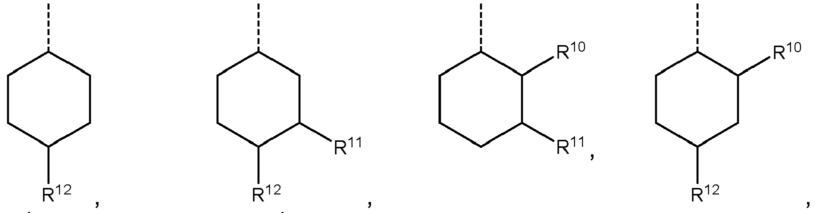

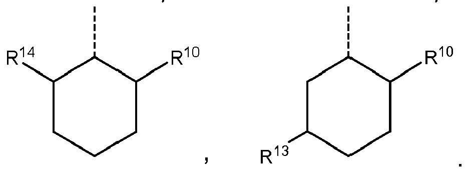

9. The compound according to claim 7, wherein R⁶ is selected from: 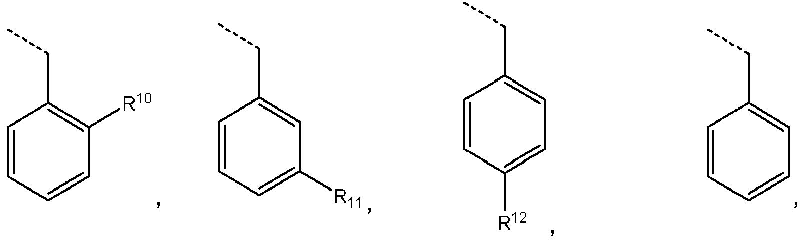 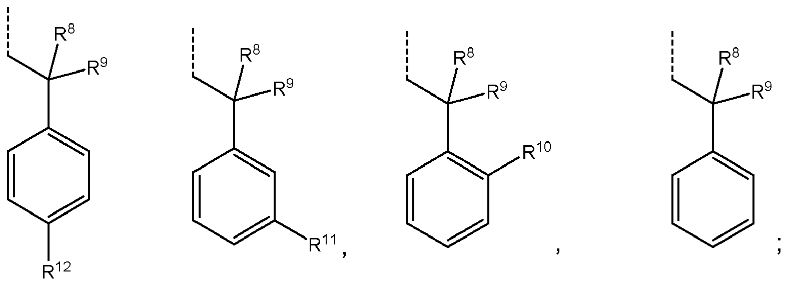 R⁸, R⁹ are independently of each other selected from -H, -CH₃ and -C₂H₅; R¹⁰, R¹¹, R¹², R¹³ and R¹⁴ are independently of each other selected from: -Cl, -F, -CH₃, -OCH₃, -OCH(CH₃)₂ and -CF₃.

10. The compound according to claim 1 selected from:
*N*-benzyl-4-(5,7-dimethyl-1-oxo-2-phenyl-1*H*-pyrrolo[3,4-*d*]pyridazin-6(2*H*)-yl)butanamide,
4-((5,7-dimethyl-1-oxo-2-phenyl-1*H*-pyrrolo[3,4-*d*]pyridazin-6(2*H*)-yl)-*N*-(3-methoxybenzyl)butanamide,
*N*-(3-chlorobenzyl)-4-(5,7-dimethyl-1-oxo-2-phenyl-1*H*-pyrrolo[3,4-*d*]pyridazin-6(2H)-yl)butanamide,
*N*-(2-chlorobenzyl)-4-(5,7-dimethyl-1-oxo-2-phenyl-1*H*-pyrrolo[3,4-*d*]pyridazin-6(2H)-yl)butanamide,
*N*-(4-chlorobenzyl)-4-(5,7-dimethyl-1-oxo-2-phenyl-1*H*-pyrrolo[3,4-*d*]pyridazin-6(2H)-yl)butanamide,
4-((5,7-dimethyl-1-oxo-2-phenyl-1*H*-pyrrolo[3,4-*d*]pyridazin-6(2*H*)-yl)-*N*-(thiophen-2-ylmethyl)butanamide,
4-((5,7-dimethyl-1-oxo-2-phenyl-1*H*-pyrrolo[3,4-*d*]pyridazin-6(2*H*)-yl)-*N*-(furan-2-ylmethyl)butanamide,
4-((5,7-dimethyl-1-oxo-2-phenyl-1*H*-pyrrolo[3,4-*d*]pyridazin-6(2*H*)-yl)-*N*-(pyridin-2-ylmethyl)butanamide,
*N*-cyclopropyl-4-(5,7-dimethyl-1-oxo-2-phenyl-1*H*-pyrrolo[3,4-*d*]pyridazin-6(2*H*)-yl)butanamide,
*N*-cyclopentyl-4-(5,7-dimethyl-1-oxo-2-phenyl-1*H*-pyrrolo[3,4-*d*]pyridazin-6(2*H*)-yl)butanamide,
*N*-cyclohexyl-4-(5,7-dimethyl-1-oxo-2-phenyl-1*H*-pyrrolo[3,4-*d*]pyridazin-6(2*H*)-yl)butanamide,
4-((5,7-dimethyl-1-oxo-2-phenyl-1*H*-pyrrolo[3,4-*d*]pyridazin-6(2*H*)-yl)-*N*-(4-methylcyclohexyl)butanamide,
4-((5,7-dimethyl-1-oxo-2-phenyl-1*H*-pyrrolo[3,4-*d*]pyridazin-6(2*H*)-yl)-*N*-(2-methylcyclohexyl)butanamide,
4-((5,7-dimethyl-1-oxo-2-phenyl-1*H*-pyrrolo[3,4-*d*]pyridazin-6(2*H*)-yl)-*N*-(2,3-d imethylcyclohexyl)butanamide,
6-((3-(1-benzyl-1*H*-benzo[d]imidazol-2-yl)propyl)-5,7-dimethyl-2-phenyl-2,6-dihydro-1*H*-pyrrolo[3,4-*d*]pyridazin-1-one,
5,7-Dimethyl-2-phenyl-6-(3-(3-phenyl-1,2,4-oxadiazol-5-yl)propyl)-2,6-dihydro-1*H-*pyrrolo[3,4-*d*]pyridazin-1-one,
5,7-Dimethyl-2-phenyl-6-(3-(3-(p-tolyl)-1,2,4-oxadiazol-5-yl)propyl)-2,6-dihydro-1 *H*-pyrrolo[3,4-*d*]pyridazin-1-one,
6-((3-(3-(4-methoxyphenyl)-1,2,4-oxadiazol-5-yl)propyl)-5,7-dimethyl-2-phenyl-2,6-dihydro-1*H*-pyrrolo[3,4-*d*]pyridazin-1-one,
6-((3-(3-(2,5-dimethoxyphenyl)-1,2,4-oxadiazol-5-yl)propyl)-5,7-dimethyl-2-phenyl-2,6-dihydro-1*H*-pyrrolo[3,4-*d*]pyridazin-1-one,
5,7-dimethyl-2-phenyl-6-(3-(3-(4-(trifluoromethyl)phenyl)-1,2,4-oxadiazol-5-yl)propyl)-2,6-dihydro-1*H*-pyrrolo[3,4-*d*]pyridazin-1-one,
6-((3-(3-(4-fluorophenyl)-1,2,4-oxadiazol-5-yl)propyl)-5,7-dimethyl-2-phenyl-2,6-dihydro-1*H*-pyrrolo[3,4-*d*]pyridazin-1-one,
5,7-Dimethyl-2-phenyl-6-(3-(5-phenyl-1,3,4-oxadiazol-2-yl)propyl)-2,6-dihydro-1*H-*pyrrolo[3,4-*d*]pyridazin-1-one,
5,7-Dimethyl-2-phenyl-6-(3-(5-(p-tolyl)-1,3,4-oxadiazol-2-yl)propyl)-2,6-dihydro-1 *H*-pyrrolo[3,4-*d*]pyridazin-1-one,
6-((3-(5-(4-Methoxyphenyl)-1,3,4-oxadiazol-2-yl)propyl)-5,7-dimethyl-2-phenyl-2,6-dihydro-1*H*-pyrrolo[3,4-*d*]pyridazin-1-one,
6-((3-(5-(2-methoxyphenyl)-1,3,4-oxadiazol-2-yl)propyl)-5,7-dimethyl-2-phenyl-2,6-dihydro-1*H*-pyrrolo[3,4-*d*]pyridazin-1-one,
5,7-Dimethyl-2-phenyl-6-(3-(5-(4-(trifluoromethyl)phenyl)-1,3,4-oxadiazol-2-yl)propyl)-2,6-dihydro-1*H*-pyrrolo[3,4-*d*]pyridazin-1-one,
6-((3-(5-(4-Fluorophenyl)-1,3,4-oxadiazol-2-yl)propyl)-5,7-dimethyl-2-phenyl-2,6-dihydro-1*H*-pyrrolo[3,4-*d*]pyridazin-1-one,
5,7-Dimethyl-2-phenyl-6-(3-(5-(pyridin-4-yl)-1,3,4-oxadiazol-2-yl)propyl)-2,6-dihydro-1*H*-pyrrolo[3,4-*d*]pyridazin-1-one,
*N-*(3-chloro-2-methylphenyl)-4-(3,4-dimethyl-7-oxo-2-phenyl-2*H*-pyrazolo[3,4-*d*]pyridazin-6(7*H*)-yl)butanamide,
4-((3,4-dimethyl-7-oxo-2-phenyl-2*H*-pyrazolo[3,4-*d*]pyridazin-6(7*H*)-yl)-*N*-(furan-2-ylmethyl)butanamide,
*N*-benzyl-4-(3,4-dimethyl-7-oxo-2-phenyl-2*H*-pyrazolo[3,4-*d*]pyridazin-6(7*H*)-yl)butanamide,
4-((3,4-dimethyl-7-oxo-2-phenyl-2*H*-pyrazolo[3,4-*d*]pyridazin-6(7*H*)-yl)-*N*-(4-methoxybenzyl)butanamide,
4-((3,4-dimethyl-7-oxo-2-phenyl-2*H*-pyrazolo[3,4-*d*]pyridazin-6(7*H*)-yl)-*N*-(3-methoxybenzyl)butanamide,
4-((3,4-dimethyl-7-oxo-2-phenyl-2*H*-pyrazolo[3,4-*d*]pyridazin-6(7*H*)-yl)-*N*-(2-methoxybenzyl)butanamide,
4-((3,4-dimethyl-7-oxo-2-phenyl-2*H*-pyrazolo[3,4-*d*]pyridazin-6(7*H*)-yl)-*N*-(4-methylbenzyl)butanamide,
4-((3,4-dimethyl-7-oxo-2-phenyl-2*H*-pyrazolo[3,4-*d*]pyridazin-6(7*H*)-yl)-*N-*phenethylbutanamide,
4-((3,4-dimethyl-7-oxo-2-phenyl-2*H*-pyrazolo[3,4-*d*]pyridazin-6(7*H*)-yl)-*N*-(4-methylphenethyl)butanamide,
4-((3,4-dimethyl-7-oxo-2-(p-tolyl)-2*H*-pyrazolo[3,4-*d*]pyridazin-6(7*H*)-yl)-*N*-(furan-2-ylmethyl)butanamide,
4-((3,4-dimethyl-7-oxo-2-(*p*-tolyl)-2*H*-pyrazolo[3,4-*d*]pyridazin-6(7*H*)-yl)-*N-*((tetrahydrofuran-2-yl)methyl)butanamide,
4-((3,4-dimethyl-7-oxo-2-(p-tolyl)-2*H*-pyrazolo[3,4-*d*]pyridazin-6(7*H*)-yl)-*N*-(pyridin-2-ylmethyl)butanamide,
4-((3,4-dimethyl-7-oxo-2-(p-tolyl)-2*H*-pyrazolo[3,4-*d*]pyridazin-6(7*H*)-yl)-*N*-(pyridin-3-ylmethyl)butanamide,
*N*-benzyl-4-(3,4-dimethyl-7-oxo-2-(*p*-tolyl)-2*H*-pyrazolo[3,4-*d*]pyridazin-6(7*H*)-yl)butanamide,
4-((3,4-dimethyl-7-oxo-2-(*p*-tolyl)-2*H*-pyrazolo[3,4-*d*]pyridazin-6(7*H*)-yl)-*N*-(4-methoxybenzyl)butanamide,
4-((3,4-dimethyl-7-oxo-2-(p-tolyl)-2*H*-pyrazolo[3,4-*d*]pyridazin-6(7*H*)-yl)-*N*-(4-methylbenzyl)butanamide,
4-((3,4-dimethyl-7-oxo-2-(*p*-tolyl)-2*H*-pyrazolo[3,4-*d*]pyridazin-6(7*H*)-yl)-*N*-(4-methylbenzyl)butanamide,
4-((3,4-dimethyl-7-oxo-2-(p-tolyl)-2*H*-pyrazolo[3,4-*d*]pyridazin-6(7*H*)-yl)-*N-*phenethylbutanamide,
4-((3,4-dimethyl-7-oxo-2-(p-tolyl)-2*H*-pyrazolo[3,4-*d*]pyridazin-6(7*H*)-yl)-*N*-(4-methylphenethyl)butanamide,
*N*-(4-chlorophenethyl)-4-(3,4-dimethyl-7-oxo-2-(p-tolyl)-2*H*-pyrazolo[3,4-*d*]pyridazin-6(7*H*)-yl)butanamide,
*N*-(3-chlorophenethyl)-4-(3,4-dimethyl-7-oxo-2-(p-tolyl)-2*H*-pyrazolo[3,4-*d*]pyridazin-6(7*H*)-yl)butanamide,
4-((3,4-dimethyl-7-oxo-2-(p-tolyl)-2*H*-pyrazolo[3,4-*d*]pyridazin-6(7*H*)-yl)-*N*-(2-phenylpropyl)butanamide,
*N*-cyclopropyl-4-(3,4-dimethyl-7-oxo-2-(p-tolyl)-2*H*-pyrazolo[3,4-*d*]pyridazin-6(7*H*)-yl)butanamide,
*N*-cyclopentyl-4-(3,4-dimethyl-7-oxo-2-(p-tolyl)-2*H*-pyrazolo[3,4-*d*]pyridazin-6(7*H*)-yl)butanamide, and
4-((3,4-dimethyl-7-oxo-2-(p-tolyl)-2*H*-pyrazolo[3,4-*d*]pyridazin-6(7*H*)-yl)-*N*-(4-methylcyclohexyl)butanamide.

11. Compound according to any one of claims 1 - 10 for use as pharmaceutically active agent in medicine.

12. Compound according to any one of claims 1 - 10 for use in the treatment or prophylaxis of proliferative diseases, tumors or cancers.

13. Compound according to claim 12, wherein the proliferative diseases, tumors and cancers are selected from the group comprising: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

14. Pharmaceutical composition comprising at least one compound according to any of the claims 1 - 10 as an active ingredient, together with at least one pharmaceutically acceptable carrier, excipient and/or diluent.

15. Pharmaceutical composition according to claim 14 for use in the treatment or prophylaxis of proliferative diseases, tumors or cancer.
